(19) Europäisches Patentamt
European Patent Office
Office européen des brevets

(11) EP 4 194 434 A1

(12) # EUROPEAN PATENT APPLICATION
published in accordance with Art. 153(4) EPC

(43) Date of publication:
**14.06.2023 Bulletin 2023/24**

(21) Application number: **22862354.2**

(22) Date of filing: **08.08.2022**

(51) International Patent Classification (IPC):
***C07C 43/23*** *(2006.01)*

(52) Cooperative Patent Classification (CPC):
**C07C 43/23; C07C 49/82; C07C 69/675; C07C 255/54; C07C 311/09; C07D 307/20; C08G 18/28; C08G 18/32; C08G 18/42; C08G 18/44; C08G 18/66; C08G 18/75; C08G 18/76; C09D 5/24; C09D 7/63;** (Cont.)

(86) International application number:
**PCT/JP2022/030244**

(87) International publication number:
**WO 2023/062922 (20.04.2023 Gazette 2023/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.10.2021 JP 2021169777**

(71) Applicant: **SHIN-ETSU CHEMICAL CO., LTD.**
**Tokyo 100-0005 (JP)**

(72) Inventors:
- **NONAKA, Shiori**
  **Joetsu-shi, Niigata 942-8601 (JP)**
- **HASEGAWA, Koji**
  **Tokyo 100-0005 (JP)**
- **WATANABE, Osamu**
  **Joetsu-shi, Niigata 942-8601 (JP)**

(74) Representative: **Sonnenhauser, Thomas Martin**
**Wuesthoff & Wuesthoff**
**Patentanwälte PartG mbB**
**Schweigerstraße 2**
**81541 München (DE)**

(54) **POLYURETHANE, METHOD FOR PRODUCING POLYURETHANE, ELECTROCONDUCTIVE PASTE COMPOSITION, ELECTROCONDUCTIVE WIRE, AND METHOD FOR PRODUCING ELECTROCONDUCTIVE WIRE**

(57) A polyurethane contains a phenolic hydroxyl group represented by the following general formula (1A). Thus, the present invention provides: a conductive paste composition for forming a stretchable conductive wire which varies slightly in electric conductivity at the time of elongation and shrinkage; and a polyurethane providing the composition.

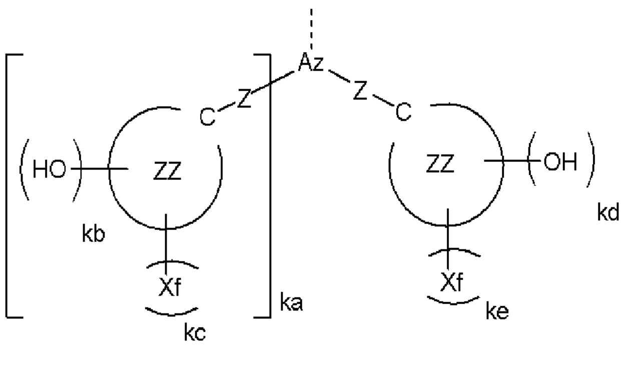

(1A)

EP 4 194 434 A1

(52) Cooperative Patent Classification (CPC): (Cont.)
**C09D 175/06; H01B 1/22; H01B 1/24; H01B 7/06; H01B 13/00; H05K 1/09; H05K 3/10**

## Description

TECHNICAL FIELD

The present invention relates to a polyurethane, a method for producing a polyurethane, a conductive paste composition, a conductive wire, and a method for producing a conductive wire.

BACKGROUND ART

A recent growing popularity of Internet of Things (IoT) has accelerated the development of wearable devices that can be attached to a human body. Particularly, wearable devices are expected to be utilized in the fields of medicine, health care, longterm care, and sports, and examined for early detection of diseases and health management by real-time monitoring of biological information and exercise information or body movements sensing.

The forms of wearable devices for continuously measuring biological information include: an accessorytype such as watches, eye-glasses, and ear phones; a cloth-type; a patch-type for direct attachment to a body; and the like. For stable measurement of accurate biological information, it is required to fit a wearable device to a body surface in use. Particularly, cloth-type and patch-type devices are required to have high flexibility and stretchability to follow body movements, as well as durability against repetitive elongations and shrinkages. Thus, it is important to develop techniques and materials for imparting stretchability to wires and sensors.

For example, a wearable device disclosed in Patent Document 1 uses a bellows-shaped stretchable silver wire covered with a stretchable urethane film. Even if a metal wire itself does not have stretchability, the wiring design enables pseudo-stretchability and ensures electric conductivity.

Moreover, there have been reported: a method in which a woven fabric is formed by weaving a conductive thread to impart stretchability (Patent Document 2); and a method using a stretchable conductive composite thread formed by winding a conductive fiber around a stretchable elastic fiber (Patent Document 3).

However, the method of weaving a conductive thread has problems including a limited pattern shape to be formed and low throughput in comparison with pattern formation by printing. Further, in the method of Patent Document 1, a pattern shape is also limited and compact wiring design is difficult. Hence, stretchable conductive paste and ink have been actively developed to form, by printing, a wire enabling electric conduction even during elongation.

There are many proposals or applications such as, for example, stretchable wires using Galinstan including gallium-indium-tin or a liquid metal including gallium-indium; a stretchable wire in which silver nanowires are mixed as a metal additive (Patent Document 4); a stretchable wire which contains a combination of a fluorine rubber, a surfactant, and a silver filler and generates silver nanoparticles during annealing (Patent Document 5); a stretchable wire using silver flakes having suitable tap density and average particle diameter (Patent Document 6); a stretchable wire using a silver powder having suitable particle diameter, particle size distribution, and porosity (Patent Document 7); or the like.

CITATION LIST

PATENT LITERATURE

Patent Document 1: JP 3923861 B
Patent Document 2: JP 6657525 B
Patent Document 3: JP 2019-076214 A
Patent Document 4: WO 2017/217509 A1
Patent Document 5: WO 2018/110632 A1
Patent Document 6: JP 2019-110093 A
Patent Document 7: WO 2018/235734 A1


SUMMARY OF INVENTION

TECHNICAL PROBLEM

The present invention has been made to solve the above-mentioned problems, and the present invention aims to provide: a conductive paste composition for forming a stretchable conductive wire which varies slightly in electric conductivity at the time of elongation and shrinkage; and a polyurethane for providing the composition.

SOLUTION TO PROBLEM

To achieve the object, the present invention provides a polyurethane containing a phenolic hydroxyl group represented by the following general formula (1A),

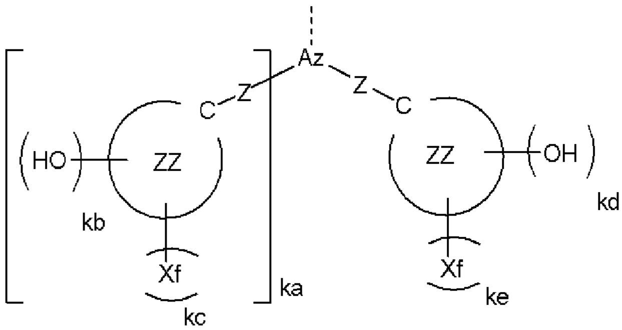

(1A)

wherein Az represents a linear, branched, or cyclic (ka+2)-valent hydrocarbon group or a fluorinated hydrocarbon group having 1 to 20 carbon atoms, and $-CH_2-$constituting the (ka+2)-valent hydrocarbon group is optionally substituted with -O-, $-NR^4-$, -C(=O)-, or - $Si(R^2R^3)-$; each of $R^2$ and $R^3$ is a linear, branched, or cyclic alkyl group having 1 to 6 carbon atoms, or a phenyl group; $R^4$ is a hydrogen atom, or a linear or branched alkyl group having 1 to 4 carbon atoms; Z represents a single bond or an oxygen atom; each Xf independently represents a hydrogen atom, a halogen atom, a linear, branched, or cyclic monovalent hydrocarbon group having 1 to 10 carbon atoms optionally substituted with a fluorine atom, an alkoxy group having 1 to 10 carbon atoms optionally substituted with a fluorine atom, or an electron-withdrawing group; each of ring ZZ independently represents an aromatic monocyclic or polycyclic ring having 5 to 20 carbon atoms; each carbon atom of the ring ZZ is optionally substituted with a nitrogen atom, an oxygen atom, or a sulfur atom; "ka" represents an integer of 0 to 2; "kb" and "kd" each represent 1 or 2; "kc" and "ke" each represent an integer of 0 to 2; and a dashed line represents a bonding arm.

Such a polyurethane provides a conductive paste composition for forming a stretchable conductive wire which varies slightly in electric conductivity at the time of elongation and shrinkage.

Further, in the present invention, the polyurethane preferably contains a phenolic hydroxyl group represented by the following general formula (1B),

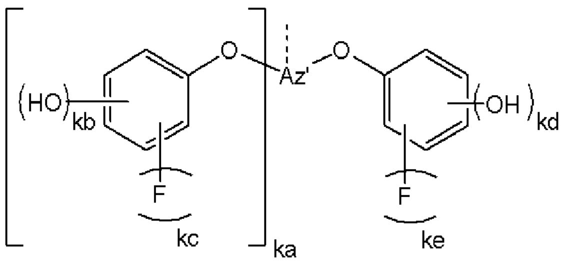

(1B)

wherein Az' represents a linear, branched, or cyclic (ka+2)-valent hydrocarbon group or a fluorinated hydrocarbon group having 1 to 19 carbon atoms, and $-CH_2-$constituting the (ka+2)-valent hydrocarbon group is optionally substituted with -O-, $-NR^4-$, -C(=O)-, or - $Si(R^2R^3)-$; "ka" represents an integer of 0 to 2; "kb", "kc", "kd", and "ke" each represent 1 or 2; $R^2$, $R^3$, and $R^4$ are as defined above; and a dashed line represents a bonding arm.

Such a polyurethane can further decrease change in electric conductivity at the time of elongation and shrinkage when used as a conductive paste composition for forming a stretchable conductive wire.

Further, in the present invention, the polyurethane preferably further contains one or more weakly acidic functional groups represented by the following general formulae (1a) to (1c),

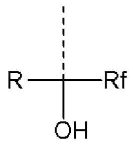

(1a)

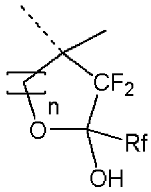

(1b)

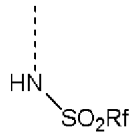

(1c)

wherein R represents a hydrogen atom, a fluorine atom, or a linear, branched, or cyclic hydrocarbon group having 1 to 10 carbon atoms optionally substituted with a fluorine atom; Rf represents a fluorine atom, or a linear, branched, or cyclic fluorinated hydrocarbon group having 1 to 10 carbon atoms; "n" is an integer of 1 or 2; and a dashed line represents a bonding arm.

When such a polyurethane is used for a conductive paste composition for forming a stretchable conductive wire, it can further decrease change in electric conductivity at the time of elongation and shrinkage.

Further, in the present invention, the polyurethane preferably further contains one or more structures represented by the following general formulae (2a) to (2c),

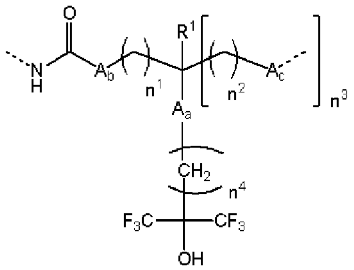

(2a)

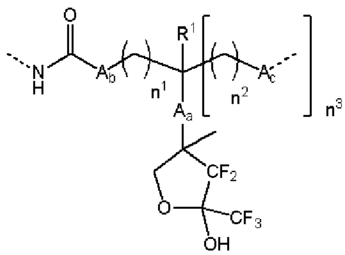

(2b)

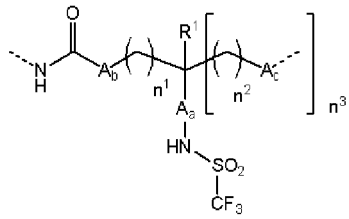

(2c)

wherein $R^1$ is a hydrogen atom or a monovalent hydrocarbon group having 1 to 3 carbon atoms; $A_a$ represents a single bond, or a linear, branched, or cyclic divalent hydrocarbon group having 1 to 20 carbon atoms, and $-CH_2-$ constituting $A_a$ is optionally substituted with -O-, -C(=O)-, -C(=O)O-, or $-C_6H_4-$, or is optionally $-NR^4-C(=O)-$; each of $A_b$ and $A_c$ independently represents any group selected from the group consisting of -O-, $-O-C(=O)-NR^4-$, $-NR^4-$, and - C(=O)O-; each of $n^1$, $n^2$, and $n^4$ is an integer of 0 to 10; $n^3$ is an integer of 0 or 1; $R^4$ is as defined above; and a dashed line represents a bonding arm.

When such a polyurethane is contained in a conductive paste composition for forming a stretchable conductive wire, it can further decrease change in electric conductivity at the time of elongation and shrinkage.

Further, the present invention provides a method for producing the polyurethane comprising introducing the phenolic hydroxyl group to a polyurethane by using an alcohol or amine represented by the following general formula (1C) after a chain extension reaction,

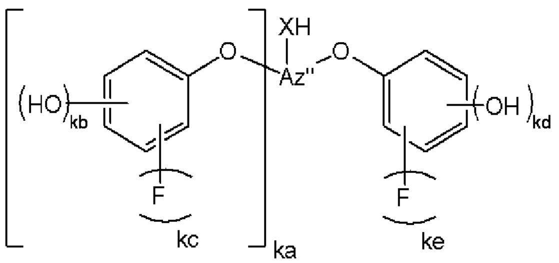

(1C)

wherein Az" represents a linear, branched, or cyclic (ka+2)-valent hydrocarbon group or a fluorinated hydrocarbon group having 1 to 19 carbon atoms, and $-CH_2-$constituting the (ka+2)-valent hydrocarbon group is optionally substituted with -O-, -C(=O)-, or $-Si(R^2R^3)-$; X represents an oxygen atom or $NR^4$; "ka" represents an integer of 0 to 2; "kb", "kc", "kd", and "ke" each represent 1 or 2; and $R^2$, $R^3$, and $R^4$ are as defined above.

Such a method for producing a polyurethane enables easy synthesis of the aforementioned polyurethane.

In this case, it is preferable to use one or more alcohols represented by the following general formulae (3a) to (3c) as a chain extender to introduce the weakly acidic functional group to the polyurethane,

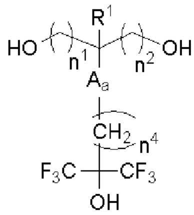 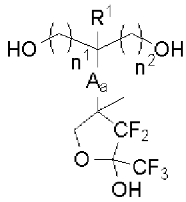 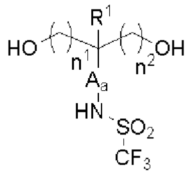

(3a) (3b) (3c)

wherein $R^1$ is a hydrogen atom or a monovalent hydrocarbon group having 1 to 3 carbon atoms; $A_a$ is a single bond, or a linear, branched, or cyclic divalent hydrocarbon group having 1 to 20 carbon atoms, and $-CH_2-$constituting $A_a$ is optionally substituted with -O-, - C(=O)-, -C(=O)O-, or $-C_6H_4-$, or is optionally $-NR^4-C(=O)-$; $R^4$ is a hydrogen atom, or a linear or branched alkyl group having 1 to 4 carbon atoms; and each of $n^1$, $n^2$, and $n^4$ is an integer of 0 to 10.

Such a method for producing a polyurethane makes it possible to readily introduce the weakly acidic functional group to the polyurethane.

Further, the present invention provides a conductive paste composition containing (A) a conductive filler, (B) the polyurethane, and (C) a solvent.

Such a conductive paste composition allows formation of a stretchable conductive wire which varies slightly in electric conductivity at the time of elongation and shrinkage.

Further, in the present invention, the conductive paste composition preferably further contains (D) a phenol compound.

Such a conductive paste composition allows formation of a stretchable conductive wire which shows less change in electric conductivity at the time of elongation and shrinkage.

Further, in the present invention, the phenol compound as the component (D) preferably contains a structure represented by the following general formula (2A),

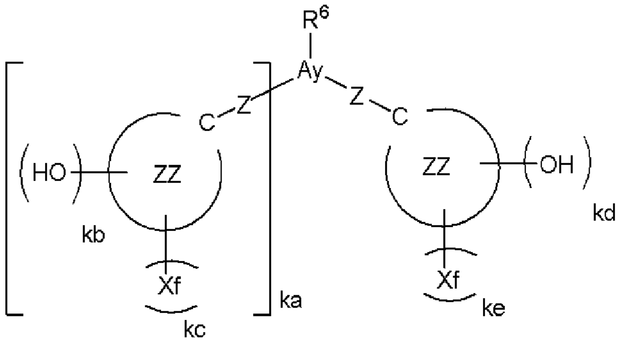

(2A)

wherein $R^6$ represents a hydrogen atom, a halogen atom, a cyano group, or a hydroxyl group; Ay represents a linear, branched, or cyclic (ka+2)-valent hydrocarbon group or a fluorinated hydrocarbon group having 1 to 20 carbon atoms, and $-CH_2-$ constituting the (ka+2)-valent hydrocarbon group is optionally substituted with -O-, - C(=O)-, or $-Si(R^2R^3)-$; "ka" represents an integer of 0 to 2; "kb" and "kd" each represent 1 or 2; "kc" and "ke" each represent an integer of 0 to 2; and Z, Xf, ZZ, $R^2$, and $R^3$ are as defined above.

Such a conductive paste composition allows formation of a stretchable conductive wire which shows less change in electric conductivity at the time of elongation and shrinkage.

Further, in the present invention, the phenol compound as the component (D) preferably contains a structure represented by the following general formula (2B),

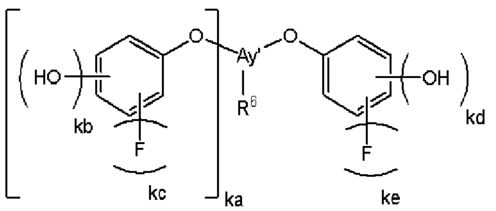

(2B)

wherein Ay' represents a linear, branched, or cyclic (ka+2)-valent hydrocarbon group or a fluorinated hydrocarbon group having 1 to 19 carbon atoms, and $-CH_2-$constituting the (ka+2)-valent hydrocarbon group is optionally substituted with

-O-, -C(=O)-, or -Si($R^2R^3$)-; "ka" represents an integer of 0 to 2; "kb", "kc", "kd", and "ke" each represent 1 or 2; and $R^2$, $R^3$, and $R^6$ are as defined above.

Such a conductive paste composition allows formation of a stretchable conductive wire which shows even smaller change in electric conductivity at the time of elongation and shrinkage.

In this case, the conductive filler as the component (A) is preferably contained in a proportion exceeding 70 parts by mass relative to 100 parts by mass of a total of the components (A) and (B).

Such a conductive paste composition allows formation of a stretchable conductive wire which has sufficient electric conductivity and shows less change in the electric conductivity at the time of elongation and shrinkage.

Further, in the present invention, the conductive filler as the component (A) is preferably a powder selected from the group consisting of gold, silver, silver chloride, platinum, copper, tin, iron, magnesium, titanium, nickel, palladium, aluminum, tungsten, molybdenum, ruthenium, chromium, indium, solder, carbon, and composites thereof.

Such a conductive paste composition allows formation of a stretchable conductive wire with enhanced electric conductivity.

In this case, the conductive filler as the component (A) is particularly preferably a silver powder.

Such a conductive filler is suitable from the comprehensive perspective of electric conductivity and price.

Further, in the present invention, the conductive filler as the component (A) preferably has an average particle size of 5 nm to 10 $\mu$m.

It is suitable to blend such a conductive filler in the conductive paste composition.

Further, the present invention provides a conductive wire formed on a substrate, the conductive wire containing a baked product of the aforementioned conductive paste composition.

Such a conductive wire varies slightly in electric conductivity at the time of elongation and shrinkage.

In this case, the substrate is preferably stretchable.

Such a substrate is suitable for the conductive wire of the present invention.

In this case, the substrate is preferably a thermoplastic polyurethane.

Such a substrate is more suitable for the conductive wire of the present invention.

Further, in the conductive wire of the present invention, electric resistance upon 20% elongation is preferably 500% or less of electric resistance before the elongation.

Such a conductive wire varies slightly in electric conductivity at the time of elongation and shrinkage, and can fit to a body surface in use.

Further, a maximum electric resistance when the conductive wire of the present invention is elongated and shrunk repeatedly 1000 times with an elongation ratio of 20% is preferably 5000% or less of electric resistance before the elongations and shrinkages.

Such a conductive wire has appropriate conduction stability in repetitive elongations and shrinkages.

Further, the present invention provides a method for producing a conductive wire by using the aforementioned conductive paste composition to form a conductive wire on a substrate, wherein the conductive wire is formed with a baking temperature of 60 to 160°C.

Such a method for producing a conductive wire makes it possible to form a conductive wire on a substrate with low heat resistance, and ensures to provide a conductive wire which varies slightly in electric conductivity at the time of elongation and shrinkage.

Further, in the present invention, a conductive wire can also be formed on a substrate by printing the conductive paste composition.

By forming a wire pattern by printing, designability and productivity of the wire can be improved.

## ADVANTAGEOUS EFFECTS OF INVENTION

As described above, a conductive paste composition containing the polyurethane of the present invention makes it possible to form a conductive wire which varies slightly in electric conductivity at the time of elongation and shrinkage, and is capable of efficiently transmitting electric signals to a device (i.e., excellent in electric conductivity), and which is lightweight and can be manufactured at low cost.

Moreover, because of the excellent conduction stability in repetitive elongations and shrinkages, it is possible to form a conductive wire suitable for wearable devices in which strain is generated by human body movement.

## DESCRIPTION OF EMBODIMENTS

In conventional techniques, a conductive wire using a conductive paste obtained by mixing a metal filler into a resin contains an insulating component. Thus, the electric resistance is higher than those of a metal wire, increase in the resistance due to elongation and degradation due to repetitive elongations and shrinkages are inevitable, and sometimes the wire is broken. Moreover, it is concerned that change in electric conductivity due to elongation and

shrinkage may affect the device operation. For these reasons, there have been demands for the development of a conductive material composition having higher electric conductivity and less change in the electric conductivity at the time of elongation and shrinkage.

As a result of the intensive studies for solving the aforementioned problems, the inventors found that by using a conductive paste composition containing (A) a conductive filler, (B) a polyurethane containing a phenolic hydroxyl group represented by the following general formula (1A), and (C) a solvent, it is possible to obtain a conductive wire that has low resistance, less decrease in electric conductivity upon elongation, and excellent conduction stability in repetitive elongations and shrinkages.

Particularly, the polyurethane containing the phenolic hydroxyl group represented by the following general formula (1A) has not been known conventionally.

In other words, the present invention is the polyurethane containing the phenolic hydroxyl group represented by the following general formula (1A),

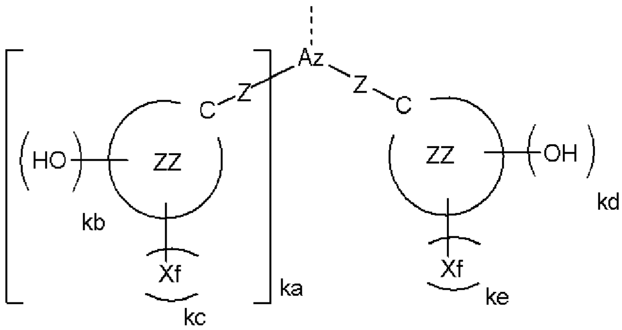

(1A)

wherein Az represents a linear, branched, or cyclic (ka+2)-valent hydrocarbon group or a fluorinated hydrocarbon group having 1 to 20 carbon atoms, and $-CH_2-$constituting the (ka+2)-valent hydrocarbon group is optionally substituted with -O-, $-NR^4-$, -C(=O)-, or - $Si(R^2R^3)-$; each of $R^2$ and $R^3$ is a linear, branched, or cyclic alkyl group having 1 to 6 carbon atoms, or a phenyl group; $R^4$ is a hydrogen atom, or a linear or branched alkyl group having 1 to 4 carbon atoms; Z represents a single bond or an oxygen atom; each Xf independently represents a hydrogen atom, a halogen atom, a linear, branched, or cyclic monovalent hydrocarbon group having 1 to 10 carbon atoms optionally substituted with a fluorine atom, an alkoxy group having 1 to 10 carbon atoms optionally substituted with a fluorine atom, or an electron-withdrawing group; each of ring ZZ independently represents an aromatic monocyclic or polycyclic ring having 5 to 20 carbon atoms; each carbon atom of the ring ZZ is optionally substituted with a nitrogen atom, an oxygen atom, or a sulfur atom; "ka" represents an integer of 0 to 2; "kb" and "kd" each represent 1 or 2; "kc" and "ke" each represent an integer of 0 to 2; and a dashed line represents a bonding arm.

Hereinafter, the present invention will be described in detail, but the present invention is not limited thereto. Note that, in the following description, a chiral carbon, and thus an enantiomer and a diastereomer may exist in some of the structures represented by chemical formulae. In such cases, these isomers are represented by one representative formula. One of these isomers may be used, or mixtures thereof may be used.

Polyurethane

The polyurethane of the present invention contains a phenolic hydroxyl group represented by the following general formula (1A),

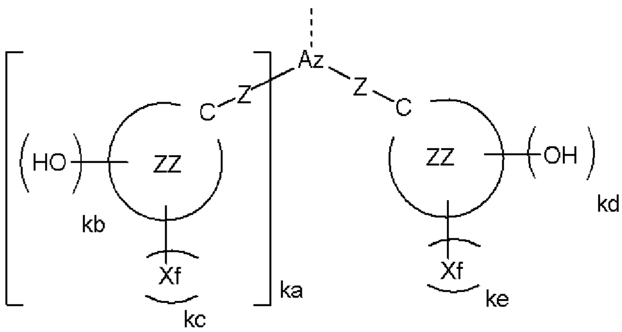

(1A)

wherein Az represents a linear, branched, or cyclic (ka+2)-valent hydrocarbon group or a fluorinated hydrocarbon group having 1 to 20 carbon atoms, and $-CH_2-$constituting the (ka+2)-valent hydrocarbon group is optionally substituted with -O-, $-NR^4-$, -C(=O)-, or - $Si(R^2R^3)$-; each of $R^2$ and $R^3$ is a linear, branched, or cyclic alkyl group having 1 to 6 carbon atoms, or a phenyl group; $R^4$ is a hydrogen atom, or a linear or branched alkyl group having 1 to 4 carbon atoms; Z represents a single bond or an oxygen atom; each Xf independently represents a hydrogen atom, a halogen atom, a linear, branched, or cyclic monovalent hydrocarbon group having 1 to 10 carbon atoms optionally substituted with a fluorine atom, an alkoxy group having 1 to 10 carbon atoms optionally substituted with a fluorine atom, or an electron-withdrawing group; each of ring ZZ independently represents an aromatic monocyclic or polycyclic ring having 5 to 20 carbon atoms; each carbon atom of the ring ZZ is optionally substituted with a nitrogen atom, an oxygen atom, or a sulfur atom; "ka" represents an integer of 0 to 2; "kb" and "kd" each represent 1 or 2; "kc" and "ke" each represent an integer of 0 to 2; and a dashed line represents a bonding arm.

Specific examples of the linear, branched, or cyclic (ka+2)-valent hydrocarbon group having 1 to 20 carbon atoms as Az include the following.

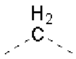
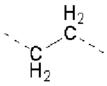
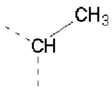
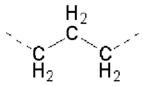
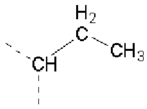
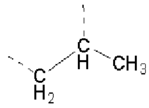
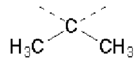

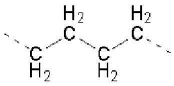
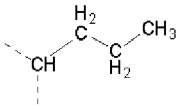
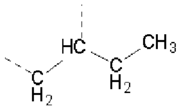
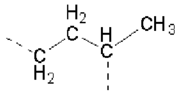
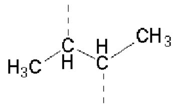

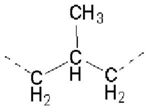
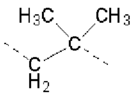
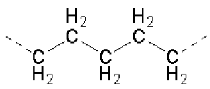
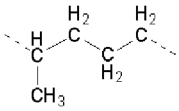
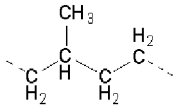

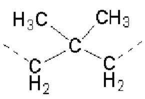
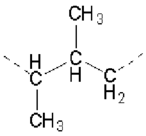
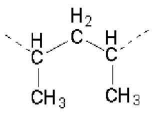
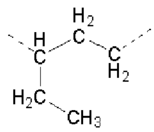
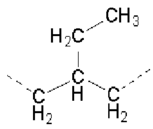
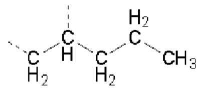

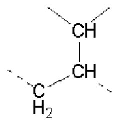
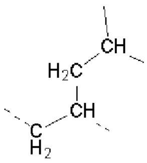
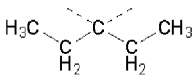
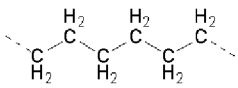
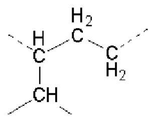

(wherein a dashed line represents a bonding arm.)

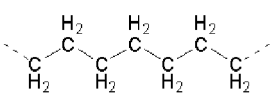
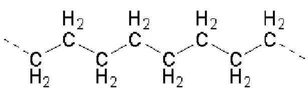
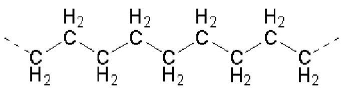

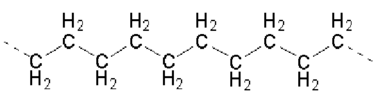
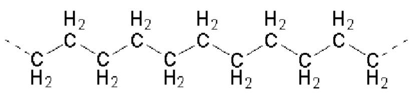

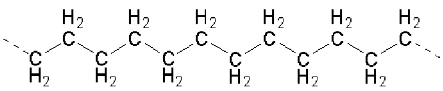
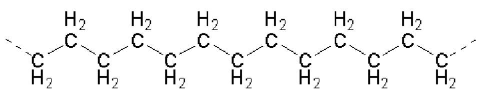

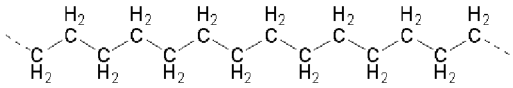 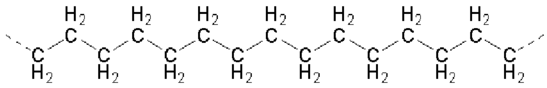

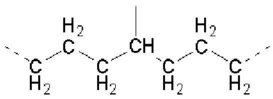 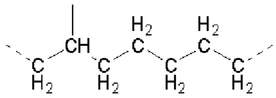 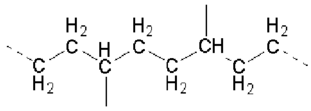

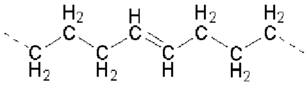 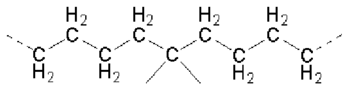

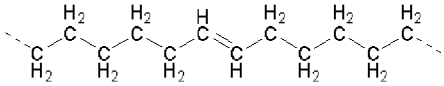 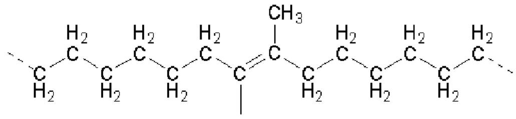

(wherein a dashed line represents a bonding arm.)

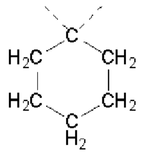 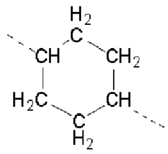 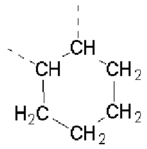 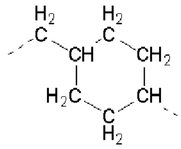 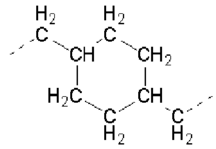

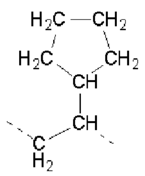 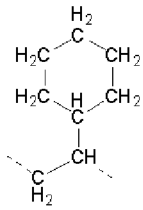 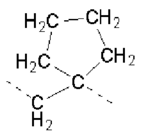 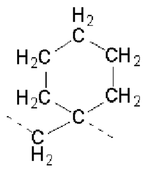 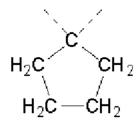 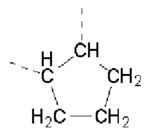

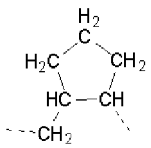 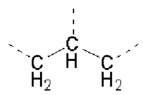 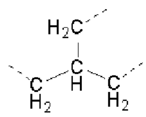 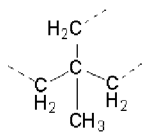 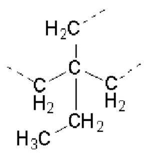 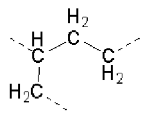

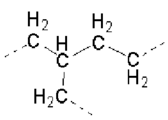 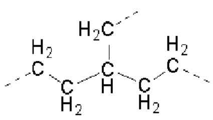 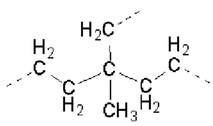 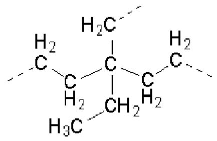 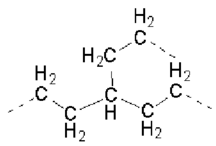

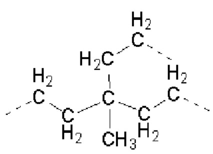 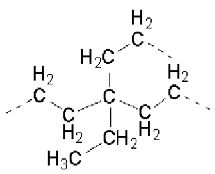 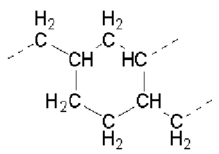 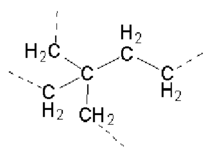 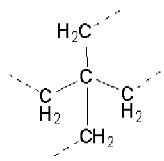

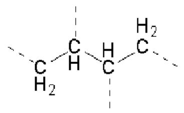 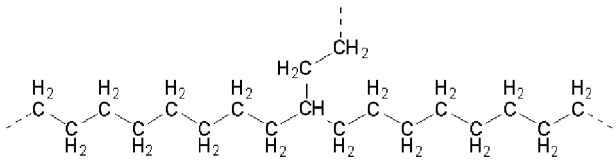

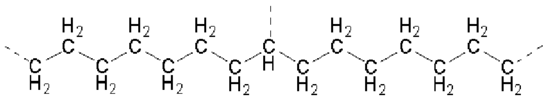 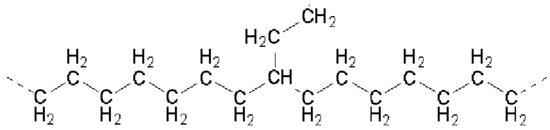

(wherein a dashed line represents a bonding arm.)

Specific examples of the linear, branched, or cyclic (ka+2)-valent fluorinated hydrocarbon group having 1 to 20 carbon atoms as Az include the one in which a part or all of the hydrogen atoms in the hydrocarbon group are substituted with a fluorine atom.

$-CH_2-$ constituting the (ka+2)-valent hydrocarbon group is optionally substituted with -O-, $-NR^4-$, -C(=O)-, or $-Si(R^2R^3)-$.

Specific examples of the linear, branched, or cyclic alkyl group having 1 to 6 carbon atoms of $R^2$ and $R^3$ include a methyl group, ethyl group, propyl group, isopropyl group, n-butyl group, sec-butyl group, tert-butyl group, n-pentyl group, n-hexyl group, cyclopropyl group, cychlobutyl group, cyclopentyl group, cyclohexyl group, and the like.

Specific examples of the linear or branched alkyl group having 1 to 4 carbon atoms of $R^4$ include a methyl group, ethyl group, propyl group, isopropyl group, and the like.

Z represents a single bond or an oxygen atom, and is preferably an oxygen atom.

Specific examples of the halogen atom of Xf include a fluorine atom, a chlorine atom, a bromine atom, and the like.

Specific examples of the linear, branched, or cyclic monovalent hydrocarbon group having 1 to 10 carbon atoms optionally substituted with a fluorine atom of Xf include: an alkyl group such as a methyl group, ethyl group, propyl group, isopropyl group, n-butyl group, sec-butyl group, tert-butyl group, cyclopropyl group, and cyclobutyl group; a trifluoromethyl group; a 2,2,2-trifluoroethyl group; and the like.

Specific examples of the alkoxy group having 1 to 10 carbon atoms optionally substituted with a fluorine atom of Xf include a methoxy group, ethoxy group, propoxy group, isopropoxy group, n-butoxy group, sec-butoxy group, tert-butoxy group, cyclopropoxy group, trifluoromethoxy group, 2,2,2-trifluoroethoxy group, and the like.

Specific examples of the electron-withdrawing group of Xf include a carbonyl group, alkoxycarbonyl group, cyano group, nitro group, sulfo group, formyl group, sulfonic acid ester group, amide group, -O-C(=O)-G- (G is a sulfur atom or NH), and the like.

Specific examples of the aromatic monocyclic or polycyclic ring having 5 to 20 carbon atoms of the ring ZZ include the following. As described below, the ring ZZ may further have a substituent.

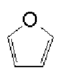 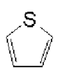 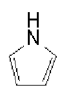 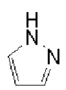 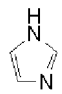 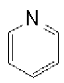 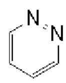 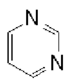 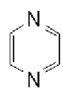 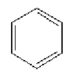

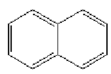 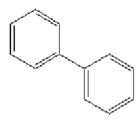 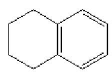 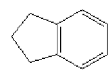 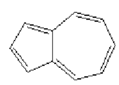 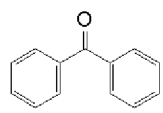

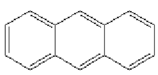 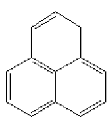 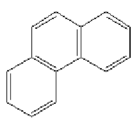 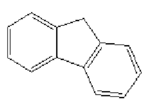 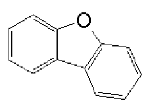 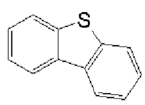

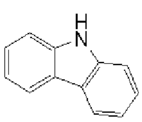 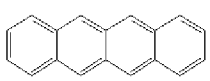 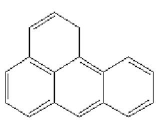 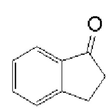 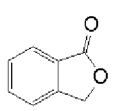

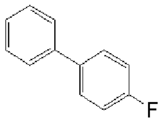 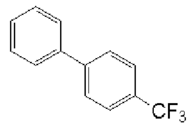 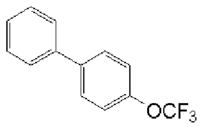 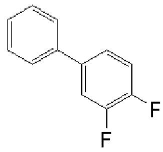 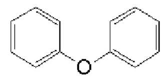

"ka" represents an integer of 0 to 2, "kb" and "kd" each represent 1 or 2, and "kc" and "ke" each represent an integer of 0 to 2.

Specific examples of the polyurethane containing the phenolic hydroxyl group represented by (1A) include a polyurethane obtained by reacting the following polyisocyanate, a high-molecular-weight polyol, and a chain extender. The polyisocyanate, the high-molecular-weight polyol, and the chain extender may be used individually or in combination.

Examples of the polyisocyanate include: aliphatic and alicyclic polyisocyanates, such as ethylene diisocyanate, trimethylene diisocyanate, tetramethylene diisocyanate, pentamethylene diisocyanate, hexamethylene diisocyanate (HDI), octamethylene diisocyanate, nonamethylene diisocyanate, 2-methylpentane-1,5-diisocyanate, 3-methylpentane-1,5-diisocyanate, 2,2'-dimethylpentane diisocyanate, 2,2,4-trimethylhexane diisocyanate, decamethylene diisocyanate, butene diisocyanate, 1,3-butadiene-1,4-diisocyanate, 2,4,4-trimethylhexamethylene diisocyanate, 1,6,11-undecamethylene triisocyanate, 1,3,6-hexamethylene triisocyanate, 1,8-diisocyanate-4-isocyanatomethyloctane, 2,5,7-trimethyl-1,8-diisocyanate-5-isocyanatomethyloctane, bis(isocyanatoethyl)carbonate, bis(isocyanatoethyl)ether, 1,4-butylene glycol dipropyl ether-ω, ω'-diisocyanate, lysine isocyanatomethyl ester, lysine triisocyanate, 2-isocyanatoethyl-2,6-diisocyanate hexanoate, 2-isocyanatopropyl-2,6-diisocyanate hexanoate, bis(4-isocyanate-n-butylidene)pentaerythritol, 2,6-diisocyanatomethyl caproate, isophorone diisocyanate (IPDI), 1,3-cyclohexyl diisocyanate, 1,4-cyclohexyl diisocyanate, 1,3-bis(isocyanatomethyl)cyclohexane, 1,4-bis(isocyanatomethyl)cyclohexane, 1,3-bis(isocyanatoethyl)cyclohexane, 1,4-bis(isocyanatoethyl)cyclohexane, methylcyclohexane diisocyanate, 2,2'-dimethyldicyclohexylmethane diisocyanate, 4,4'-dicyclohexylmethane diisocyanate, dimer acid diisocyanate, 2,5-diisocyanatomethylbicyclo[2,2,1]-heptane, 2,6-diisocyanatomethylbicyclo[2,2,1]-heptane, 2-isocyanatomethyl-2-(3-isocyanatopropyl)-5-isocyanatomethylbicyclo-[2,2,1]-heptane, 2-isocyanatomethyl-2-(3-isocyanatopropyl)-6-isocyanatomethylbicyclo-[2,2,1]-heptane, 2-isocyanatomethyl-3-(3-isocyanatopropyl)-5-(2-isocyanatoethyl)-bicyclo-[2,2,1]-heptane, 2-isocyanatomethyl-3-(3-isocyanatopropyl)-6-(2-isocyanatoethyl)-bicyclo-[2,2,1]-heptane, 2-isocyanatomethyl-2-(3-isocyanatopropyl)-5-(2-isocyanatoethyl)-bicyclo-[2,2,1]-heptane, and 2-isocyanatomethyl-2-(3-isocyanatopropyl)-6-(2-isocyanatoethyl)-bicyclo-[2,2,1]-heptane; aromatic polyisocyanates, such as 2,4-tolylene diisocyanate or 2,6-tolylene diisocyanate and isomer mixture thereof (TDI), 4,4'-diphenylmethane diisocyanate or 2,4'-diphenylmethane diisocyanate and isomer mixture thereof (MDI), toluidine diisocyanate (TODI), para-phenylene diisocyanate, naphthalene diisocyanate (NDI), and 4,4'-dibenzyl diisocyanate; aromatic-aliphatic polyisocyanates, such as ortho-xylylene diisocyanate, meta-xylylene diisocyanate, para-xylylene diisocyanate, 1,3-tetramethylxylylene diisocyanate, and 1,4-tetramethylxylylene diisocyanate; polymers thereof prepared as urethane-modified products, biuret-modified products, carbodiimide-modified products, uretoniminemodified products, uretdione-modified products, isocyanurate-modified products, or allophanate-modified products; and the like.

Examples of the high-molecular-weight polyol include polyoxypropylene glycols, polyoxyethylene glycols, polyoxytetramethylene glycols, and copolymers thereof such as polyether polyol, polyester polyol, polyester-amide polyol, polycarbonate polyol, acrylic polyol, polyolefin having a terminal hydroxyl group, silicone polyol, vegetable oil-based polyol, and the like.

Specific examples of the polyoxypropylene glycols and polyoxyethylene glycols include addition polymers of ethylene oxide, propylene oxide, butylene oxide, and the like using the following as an initiator: low-molecular-weight polyols such as dihydric alcohols (such as ethylene glycol, 1,2-propanediol, 1,3-propanediol, 1,2-butanediol, 1,3-butanediol, 1,4-butanediol, 1,5-pentanediol, 1,6-hexanediol, 1,7-heptanediol, 1,8-octanediol, 1,9-nonanediol, neopentyl glycol, 3-methyl-1,5-pentanediol, 3,3-dimethylolheptane, 2,2,2-trimethylpentanediol, 1,4-dihydroxy-2-butene, 2,6-dimethyl-1-octene-3,8-diol, diethylene glycol, triethylene glycol, dipropylene glycol, cyclohexane-1,3-diol, cyclohexane-1,4-diol, cyclohexane-1,3-dimethanol, cyclohexane-1,4-dimethanol, 1,3-adamantanedimethanol, dimer acid diol, 1,2-benzenediol, 1,3-benzenediol, 1,4-benzenediol, hydroquinone di(2-hydroxyethyl ether), bisphenol A, bis(β-hydroxyethyl)benzene, and xylylene glycol), trihydric alcohols (such as glycerin, trimethylolpropane, and triisopropanolamine), tetrahydric alcohols (such as pentaerythritol, α-methyl glucoside, and diglycerin), and polyhydric alcohols (such as sorbitol and sucrose); low-molecular-weight amino alcohols, such as monoethanolamine, dimethanolamine, and triethanolamine; and low-molecular-weight polyamines, such as ethylenediamine, propylenediamine, butanediamine, pentamethylenediamine, hexamethylenediamine, isophorone diamine, piperazine, toluenediamine, metaphenylenediamine, diphenylmethanediamine, xylylenediamine, dimethylthiotoluenediamine, and 4,4-methylenebis-o-chloroaniline. Note that the alkylene oxide, a constituent component of the polyester polyol, can be used individually, or two or more kinds thereof can be used in combination. Polyoxyalkylene polyol obtained from a combination of two or more kinds may have either a block or random structure.

Specific examples of the polyoxytetramethylene glycols include crystalline polyoxytetramethylene glycol, amorphous polyoxytetramethylene glycol obtained by copolymerizing THF with alkyl-substituted tetrahydrofuran, such as 3-methyltetrahydrofuran, or aforementioned dihydric alcohols, or the like.

Examples of the polyester polyol include: polymerization condensates between any of the aforementioned low-molecular-weight polyols and polyhydric carboxylic acid (such as oxalic acid, malonic acid, succinic acid, glutaric acid, adipic acid, pimelic acid, suberic acid, glutaconic acid, azelaic acid, sebacic acid, 1,1-dimethyl-1,3-dicarboxypropane, 3-methyl-3-ethylglutaric acid, 1,4-cyclohexyldicarboxylic acid, hexahydrophthalic acid, maleic acid, fumaric acid, itaconic acid, muconic acid, α-hydromuconic acid, β-hydromuconic acid, phthalic acid, ortho-phthalic acid, terephthalic acid, isophthalic acid, toluenedicarboxylic acid, naphthalenedicarboxylic acid, HET acid, dimer acid, and hydrogenated dimer acid) or a derivative, acid anhydride, or acid halide thereof; and ring-opening polymers of lactone (such as β-propiolactone, β-butyrolactone, γ-butyrolactone, γ-valerolactone, δ-valerolactone, and ε-caprolactone) or lactide (such as L-lactide and D-lactide) using the aforementioned low-molecular-weight polyol as an initiator. Note that the low-molecular-weight polyol, polyhydric carboxylic acid, oligomer acid, lactone, or lactide, which are constituent components of the polyester polyol, may be used individually, or two or more kinds thereof may be used in combination.

Examples of the polyester-amide polyol includes polymerization condensates obtained by substituting a part of the low-molecular-weight polyol of the polyester polyol with the aforementioned low-molecular-weight polyamine or amino alcohol.

Specific examples of the polycarbonate polyol include polymerization condensates between any of the aforementioned low-molecular-weight polyol and a carbonate compound, such as dimethyl carbonate, diethyl carbonate, ethylene carbonate, propylene carbonate, diphenyl carbonate, dinaphthyl carbonate, dianthryl carbonate, diphenanthryl carbonate, diindanyl carbonate, tetrahydronaphthyl carbonate, and the like. Note that the low-molecular-weight polyol or the carbonate compound, which are constituent components of the polycarbonate polyol, may be used individually, or two or more kinds thereof may be used in combination.

Specific examples of the acrylic polyol include copolymers obtained by copolymerizing hydroxy group-containing (meth)acrylate, such as 2-hydroxyethyl (meth)acrylate, hydroxypropyl (meth)acrylate, hydroxybutyl (meth)acrylate, 3-hydroxy-2,2-dimethylpropyl acrylate, 2,2-dihydroxymethylbutyl (meth)acrylate, pentaerythritol tri(meth)acrylate, polyhydroxyalkyl maleate, polyhydroxyalkyl fumarate, and the like, with vinyl monomer including: (meth)acrylic esters, such as methyl(meth)acrylate, ethyl(meth)acrylate, propyl(meth)acrylate, isopropyl(meth)acrylate, butyl(meth)acrylate, isobutyl(meth)acrylate, s-butyl(meth)acrylate, t-butyl(meth)acrylate, pentyl(meth)acrylate, isopentyl(meth)acrylate, hexyl(meth)acrylate, 2-ethylhexyl(meth)acrylate, octyl(meth)acrylate, nonyl(meth)acrylate, isononyl(meth)acrylate, decyl(meth)acrylate, dodecyl(meth)acrylate, cyclohexyl(meth)acrylate, phenyl(meth)acrylate, benzyl(meth)acrylate, allyl(meth)acrylate, ethylene glycol di(meth)acrylate, butylene glycol di(meth)acrylate, hexanediol di(meth)acrylate, and oligo ethylene glycol di(meth)acrylate; aromatic vinyls such as styrene, vinyltoluene, and α-methylstyrene; vinyl cyanides such as (meth)acrylonitrile; carboxyl group-containing vinyl monomers such as fumaric acid, maleic acid, and itaconic acid, or alkyl esters thereof; isocyanate group-containing monomers such as 3-(2-isocyanate-2-propyl)-α-methylstyrene; fluorinecontaining vinyl monomers such as tetrafluoroethylene, chlorotrifluoroethylene, trichlorofluoroethylene, hexafluoropropylene, vinylidene fluoride, vinyl fluoride, and trifluoromethyltrifluoroethylene; silicone monomers such as γ-(meth)acryloxypropyltrimethoxysilane; and the like. Note that the (meth)acrylate or vinyl monomer, which are constituent components of the acryl polyols, may be used individually, or two or more kinds thereof may be used in combination.

Specific examples of the polyolefin having a terminal hydroxyl group include compounds obtained by attaching a hydroxyl group to a terminal of a polymer, which is obtained by polymerizing one or two or more kinds of olefins including ethylene, propylene, butadiene, isoprene, styrene, acrylonitrile, vinyl ether, and vinyl acetate, as well as these olefins with a part of the structures thereof substituted with a halogen such as fluorine, chlorine, bromine, and the like.

Specific examples of the silicone polyol include: vinyl group-containing silicone compounds obtained by polymerizing γ-methacryloxypropyltrimethoxysilane or the like; and polysiloxanes having at least one terminal hydroxyl group per molecule, such as α,ω-dihydroxypolydimethylsiloxane and α,ω-dihydroxypolydiphenylsiloxane.

Specific examples of the vegetable oil-based polyol include: hydroxyl group-containing vegetable oils, such as castor oil and coconut oil; ester-modified castor oil polyol, dehydrated castor oil, partially dehydrated castor oil, and hydrogenated castor oil, which can be obtained from a reaction between castor oil fatty acid and polyol; and the like.

Examples of the chain extender to be used include: dihydric alcohols, such as ethylene glycol, 1,2-propanediol, 1,3-propanediol, 1,2-butanediol, 1,3-butanediol, 1,4-butanediol, 1,5-pentanediol, 1,6-hexanediol, 1,7-heptanediol, 1,8-octanediol, 1,9-nonanediol, neopentyl glycol, 3-methyl-1,5-pentanediol, 3,3-dimethylolheptane, 2,2,2-trimethylpentanediol, 1,4-dihydroxy-2-butene, 2,6-dimethyl-1-octene-3,8-diol, diethylene glycol, triethylene glycol, dipropylene glycol, cyclohexane-1,3-diol, cyclohexane-1,4-diol, cyclohexane-1,3-dimethanol, cyclohexane-1,4-dimethanol, 1,3-adamantanedimethanol, dimer acid diol, 1,2-benzenediol, 1,3-benzenediol, 1,4-benzenediol, hydroquinone di(2-hydroxyethyl ether), bisphenol A, bis(β-hydroxyethyl)benzene, and xylylene glycol; amines, such as ethanolamine, dimethanolamine, triethanolamine, ethylenediamine, propylenediamine, butanediamine, pentamethylenediamine, hexamethylenediamine, isophorone diamine, piperazine, toluenediamine, metaphenylenediamine, diphenylmethanediamine, xylylenediamine, dimethylthiotoluenediamine, and 4,4-methylenebis-o-chloroaniline; and the like.

Examples of the chain extender further containing a weakly acidic functional group include the following.

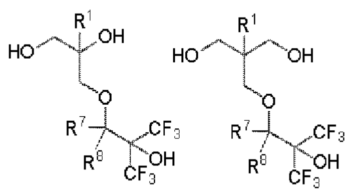
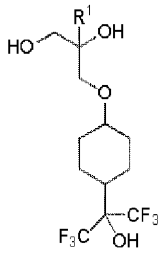
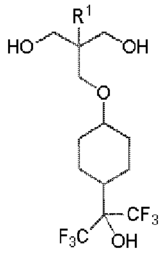
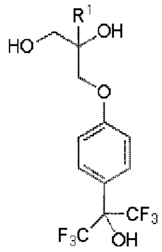
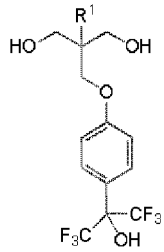
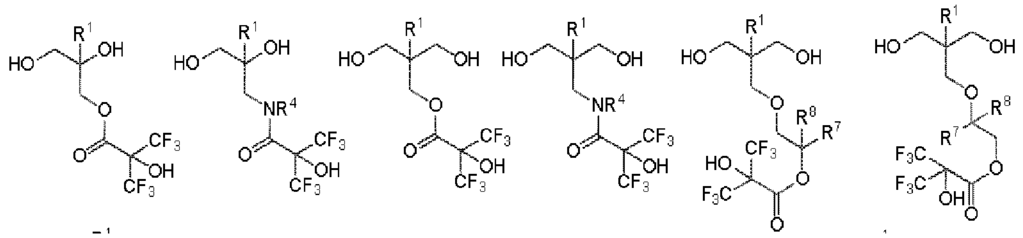
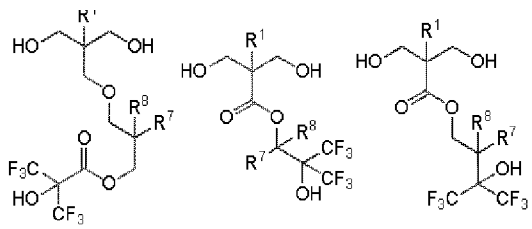
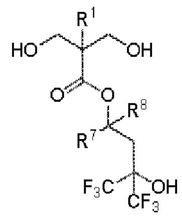
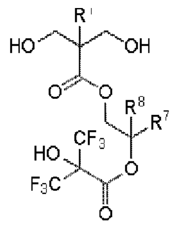
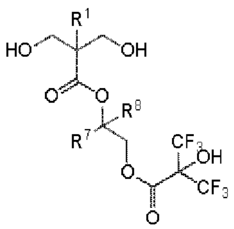
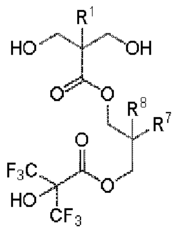
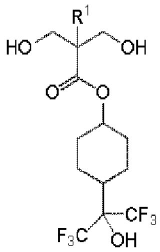
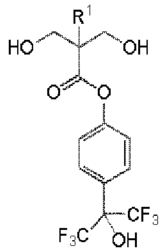
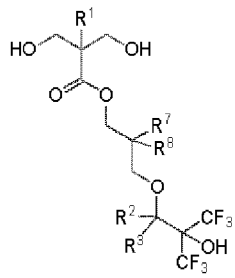
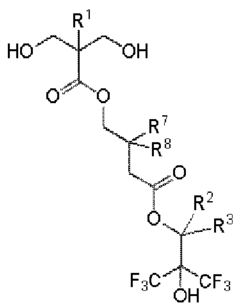
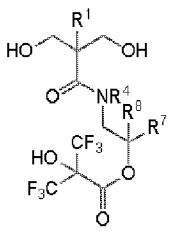
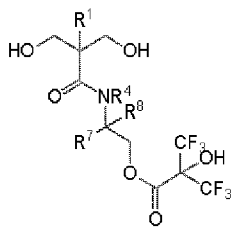
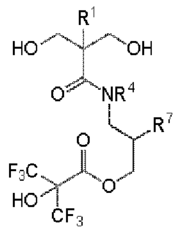
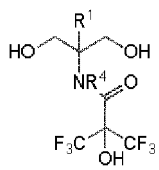

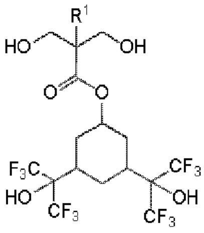 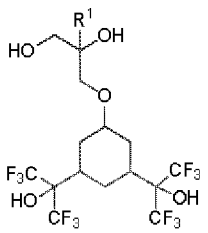 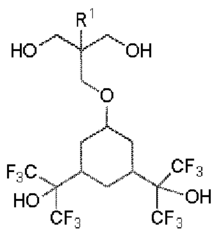

(wherein $R^1$, $R^4$, $R^7$, and $R^8$ are as defined above.)

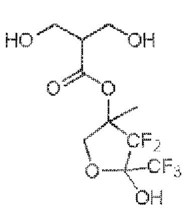 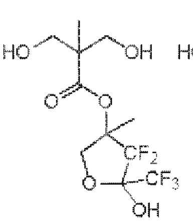 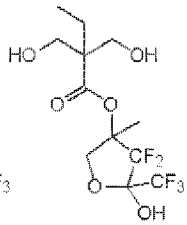 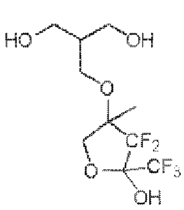 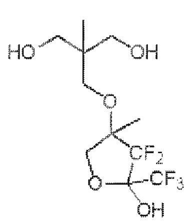 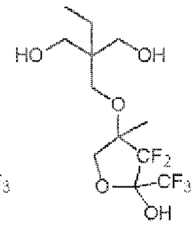

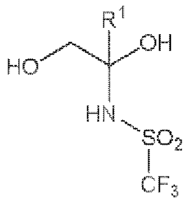 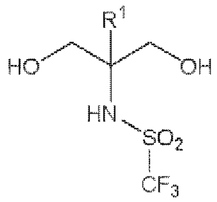 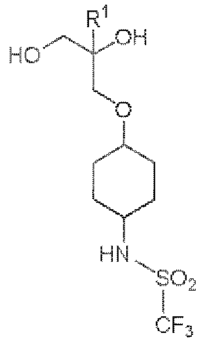 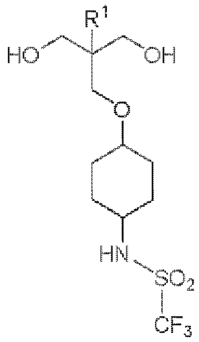 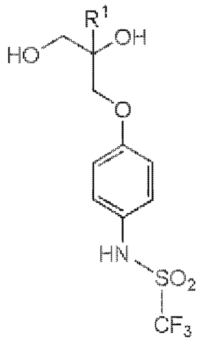

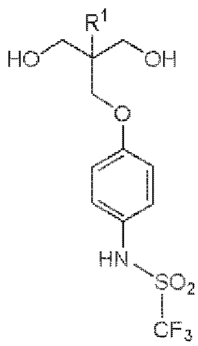 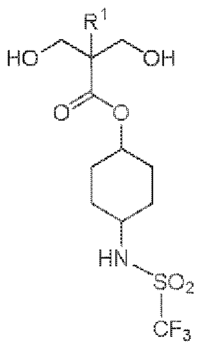 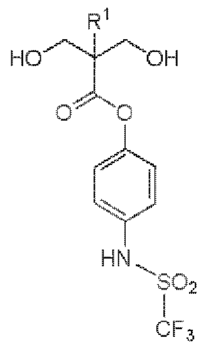

(wherein $R^1$ is as defined above.)

Examples of the chain extender further include the following substances which contain phenol.

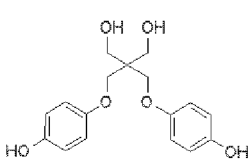 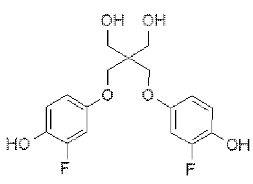 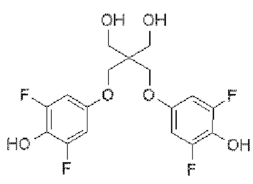 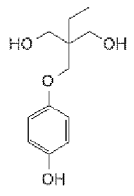 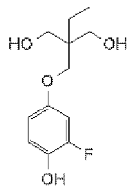

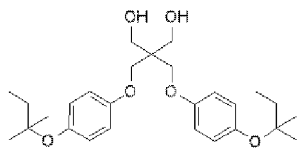
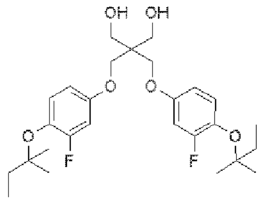
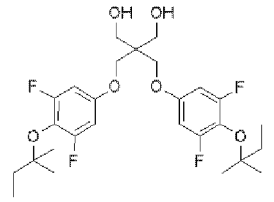
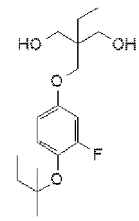

Specific examples of the polyurethane containing the phenolic hydroxyl group represented by (1A) include a polyurethane obtained from a reaction between the aforementioned polyisocyanate, high-molecular weight polyol, and chain extender.

Further examples of the polyurethane include a polyurethane obtained from a reaction between the aforementioned polyisocyanate, high-molecular weight polyol, and chain extender, as well as a capping agent below.

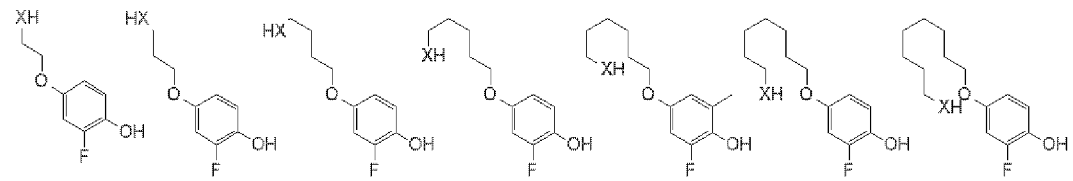

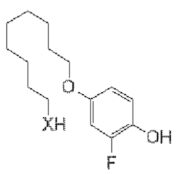
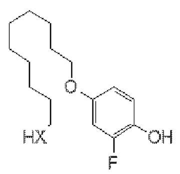
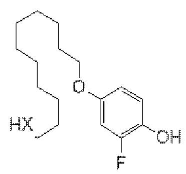
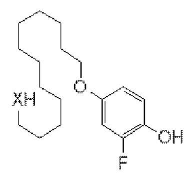
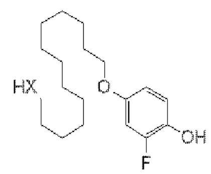

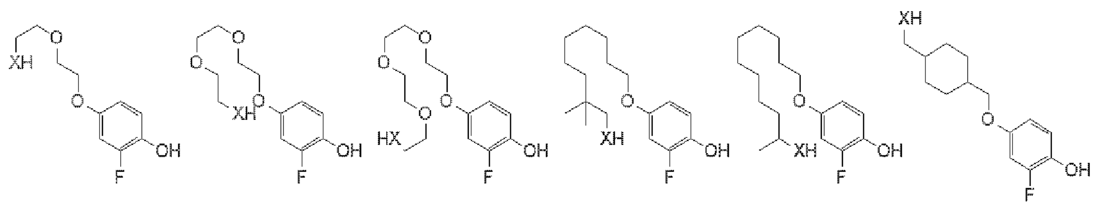

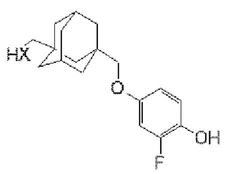
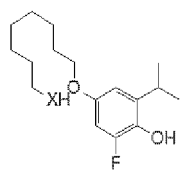
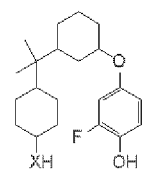
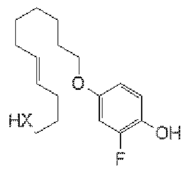

(wherein X represents an oxygen atom or $NR^4$, and $R^4$ is as defined above.)

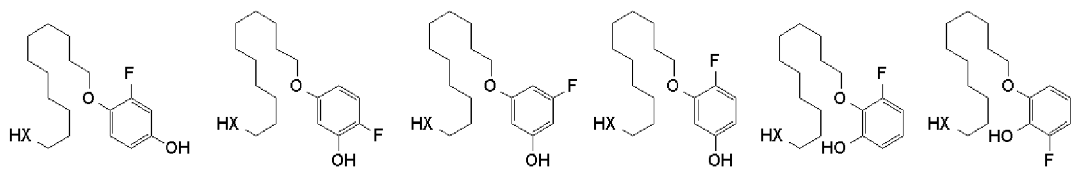

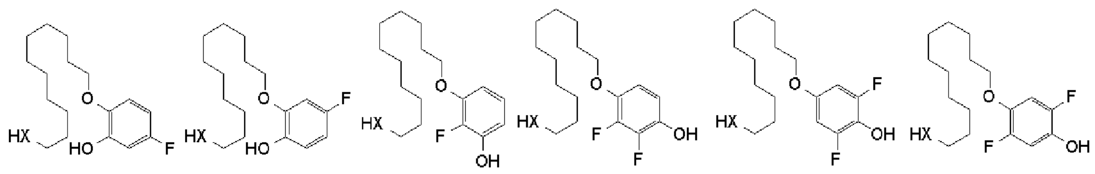

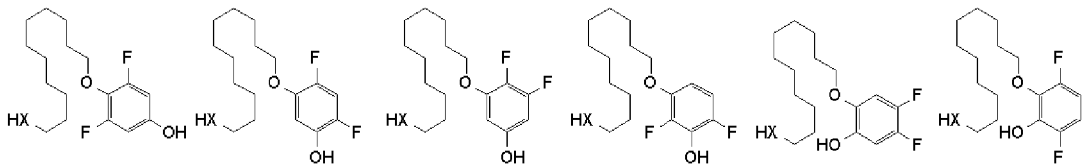

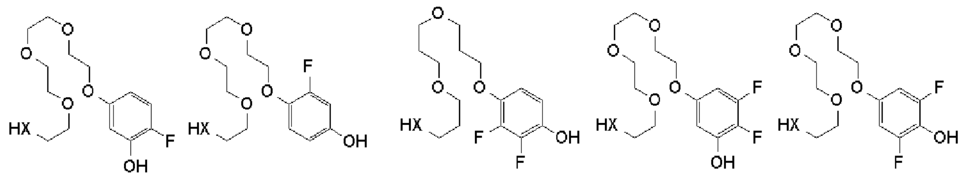

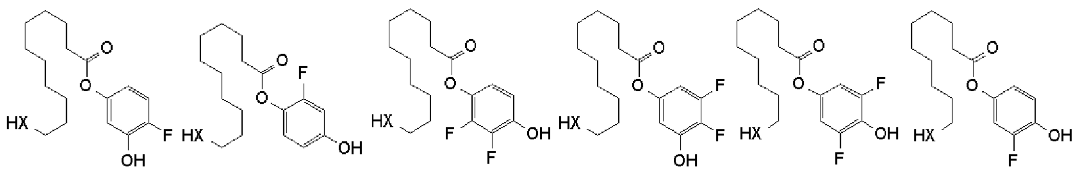

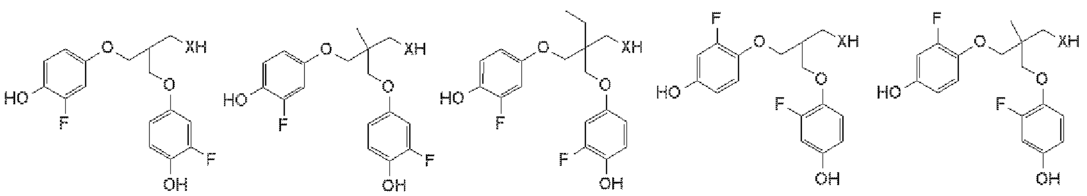

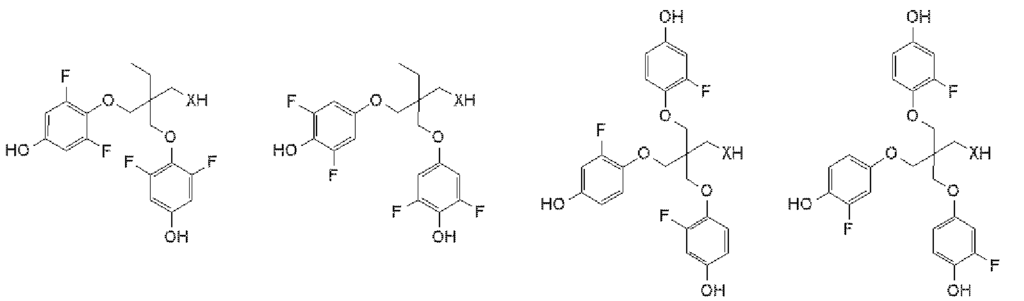

(wherein X represents an oxygen atom or $NR^4$, and $R^4$ is as defined above.)

The above examples include the following polyurethane,

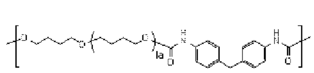

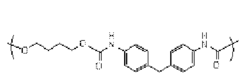

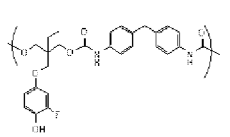

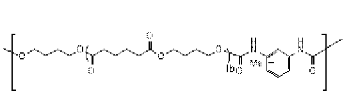
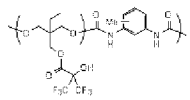
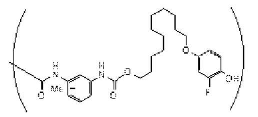

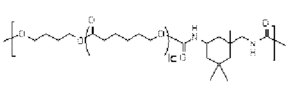
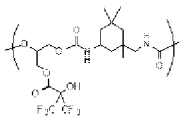
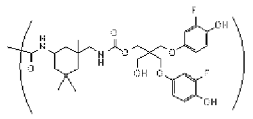

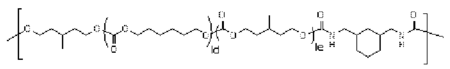
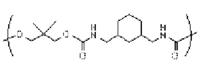
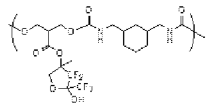
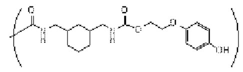

wherein "la" is an integer of 7 to 30; "lb" is an integer of 2 to 30; "lc" is an integer of 3 to 30; "ld" is an integer of 1 to 5; and "le" is an integer of 2 to 20.

The polyurethane containing the phenolic hydroxyl group represented by the above general formula (1A) is preferably particularly the one containing a phenolic hydroxyl group represented by the following general formula (1B),

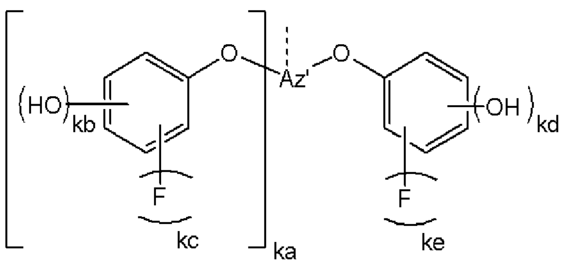 (1B)

wherein Az' represents a linear, branched, or cyclic (ka+2)-valent hydrocarbon group or a fluorinated hydrocarbon group having 1 to 19 carbon atoms, and $-CH_2$-constituting the (ka+2)-valent hydrocarbon group is optionally substituted with -O-, $-NR^4$-, -C(=O)-, or - $Si(R^2R^3)$-; "ka" represents an integer of 0 to 2; "kb", "kc", "kd", and "ke" each represent 1 or 2; $R^2$, $R^3$, and $R^4$ are as defined above; and a dashed line represents a bonding arm.

Az' represents a linear, branched, or cyclic (ka+2)-valent hydrocarbon group or a fluorinated hydrocarbon group having 1 to 19 carbon atoms, and $-CH_2$-constituting the (ka+2)-valent hydrocarbon group is optionally substituted with -O-, $-NR^4$-, -C(=O)-, or - Si $(R^2R^3)$-.

"ka" represents an integer of 0 to 2, and "kb", "kc", "kd", and "ke" each represent 1 or 2.

Specific examples of the substructures represented by the aforementioned general formulae (1A) and (1B) include the following.

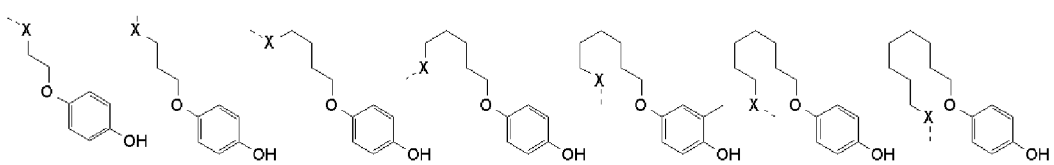

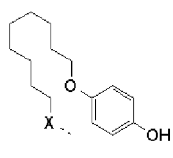
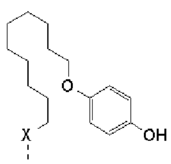
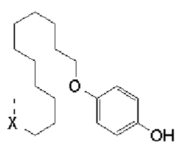
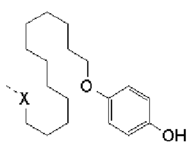
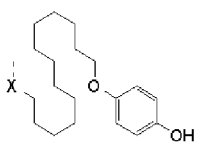

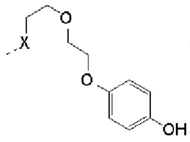 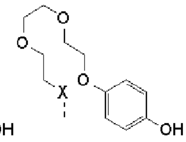 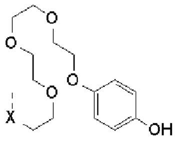 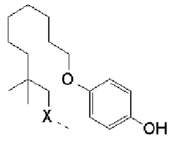 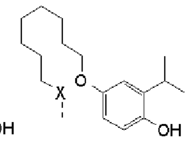 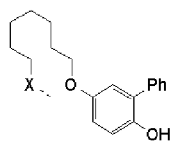

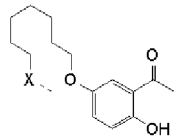 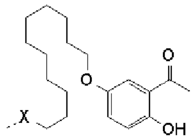 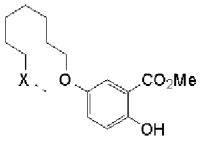 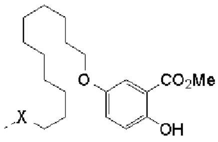 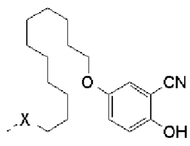

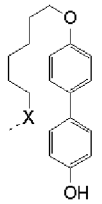 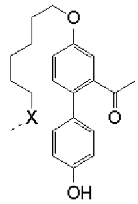 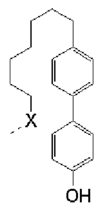 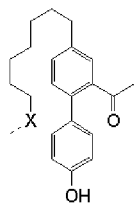 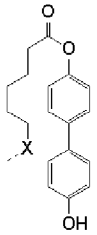 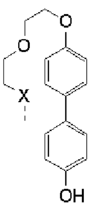 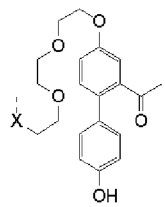

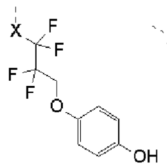 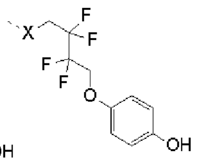 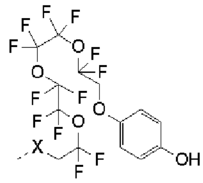 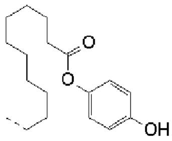 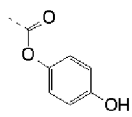 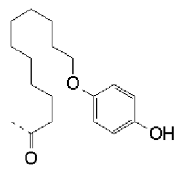

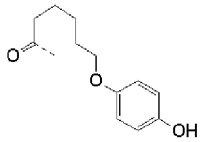 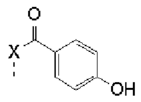 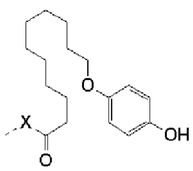 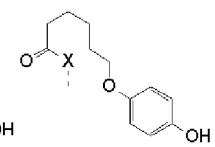

(wherein X represents an oxygen atom or $NR^4$; a dashed line represents a bonding arm; and $R^4$ is as defined above.)

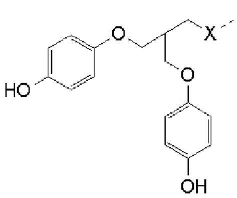 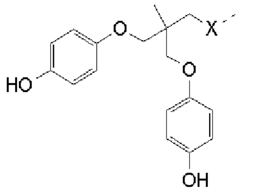 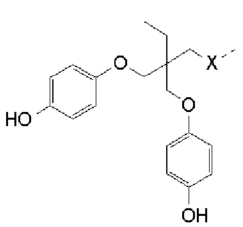 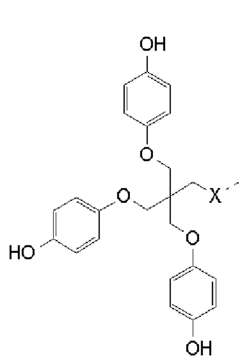

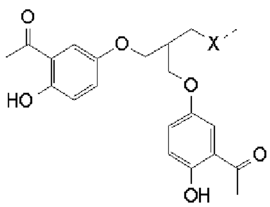 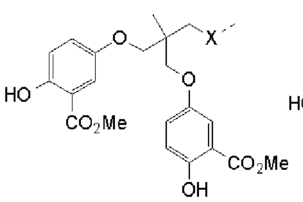 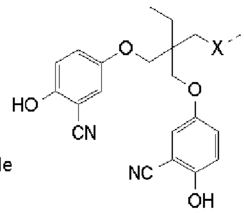 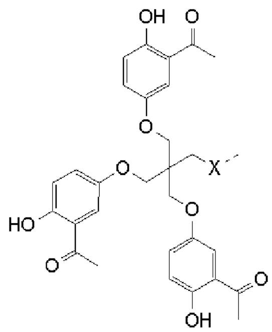

(wherein X represents an oxygen atom or $NR^4$; a dashed line represents a bonding arm; and $R^4$ is as defined above.)

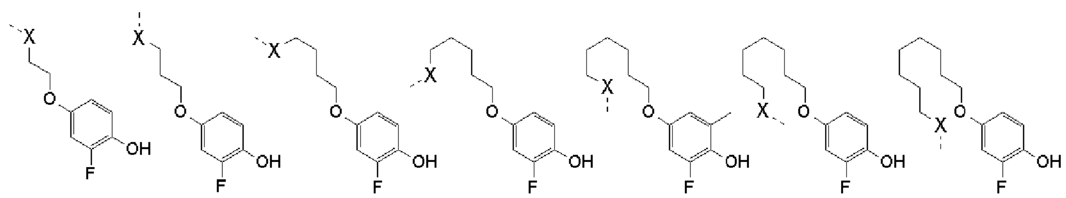

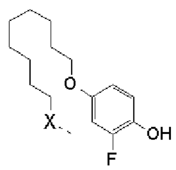 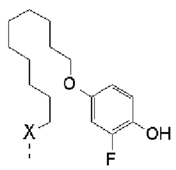 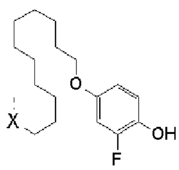 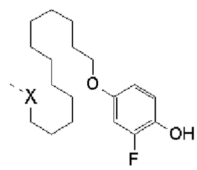 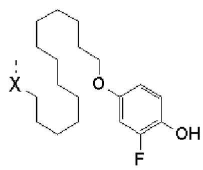

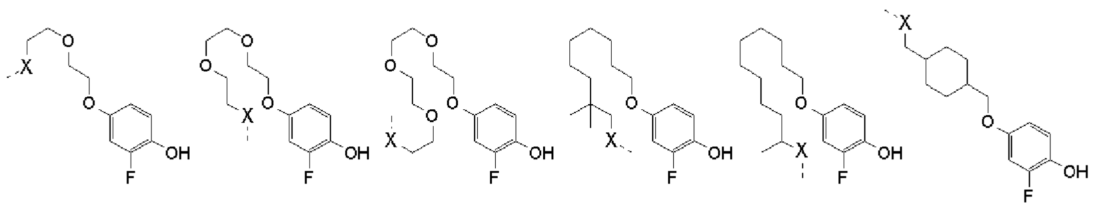

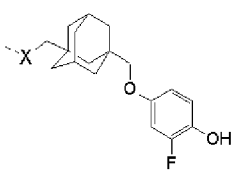 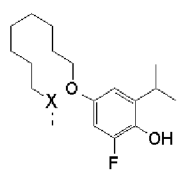 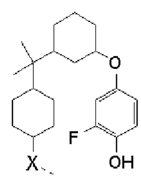 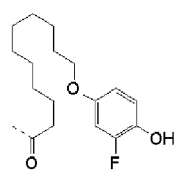 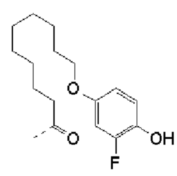

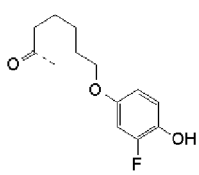 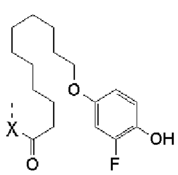 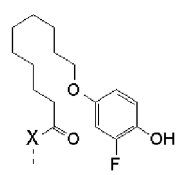 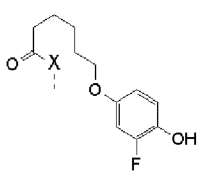 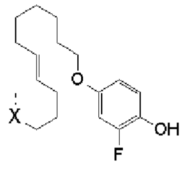

(wherein X represents an oxygen atom or $NR^4$; a dashed line represents a bonding arm; and $R^4$ is as defined above.)

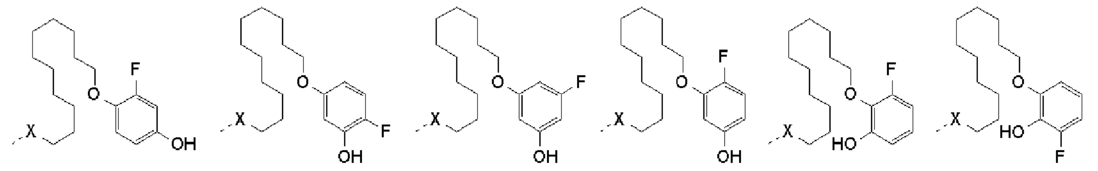

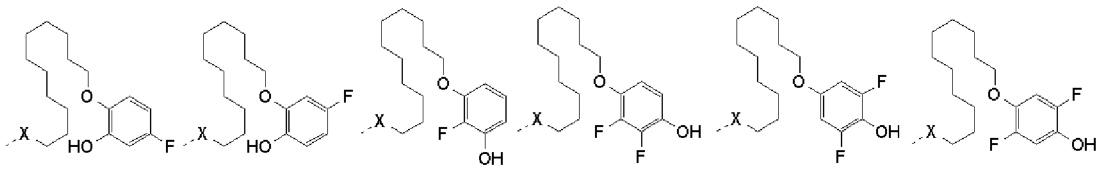

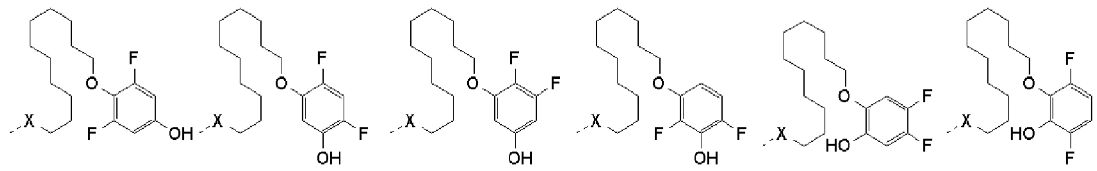

(wherein X represents an oxygen atom or $NR^4$; a dashed line represents a bonding arm; and $R^4$ is as defined above.)

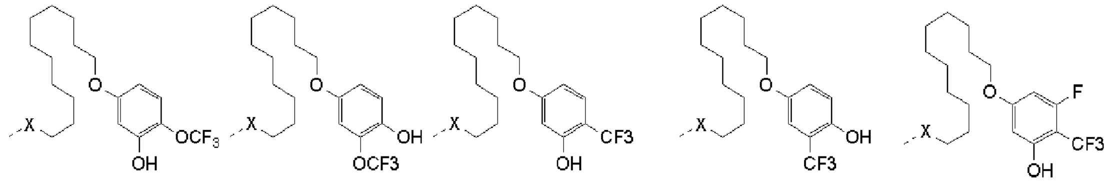

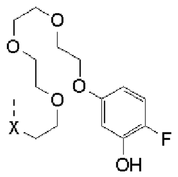
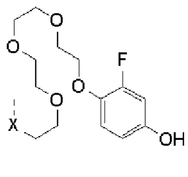
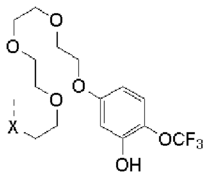
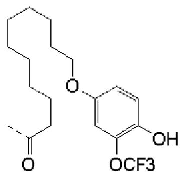
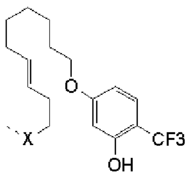

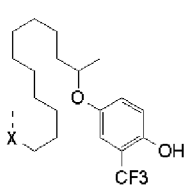
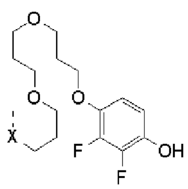
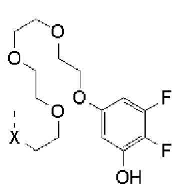
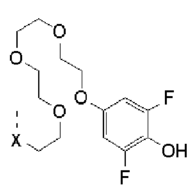
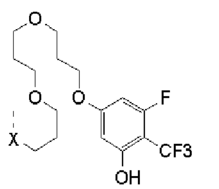

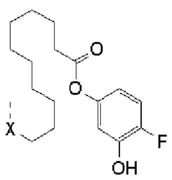
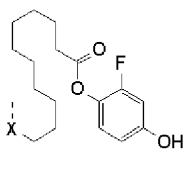
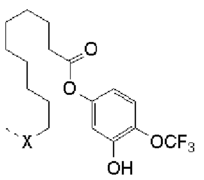
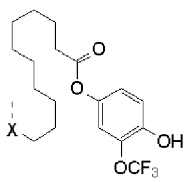
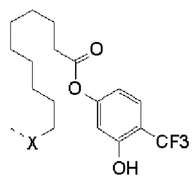

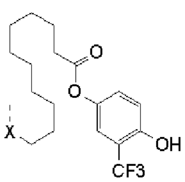
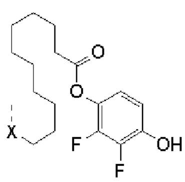
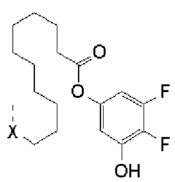
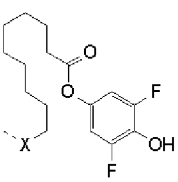
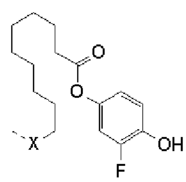

(wherein X represents an oxygen atom or $NR^4$; a dashed line represents a bonding arm; and $R^4$ is as defined above.)

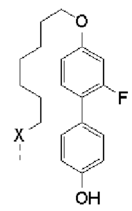 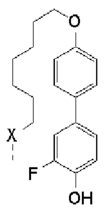 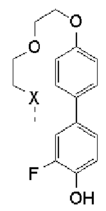 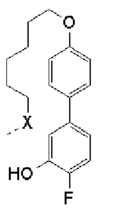 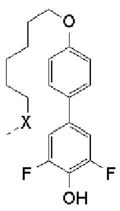 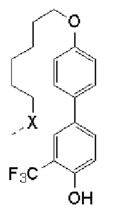 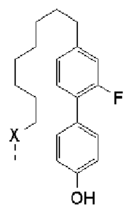 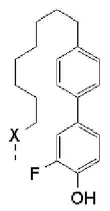

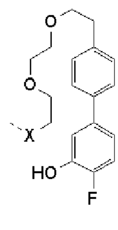 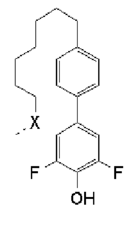 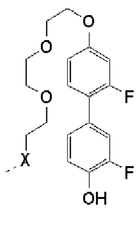 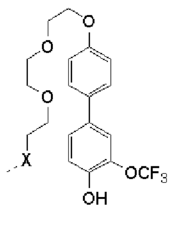 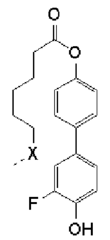 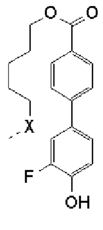 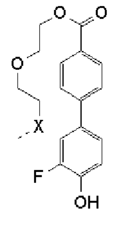 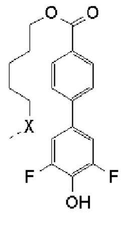

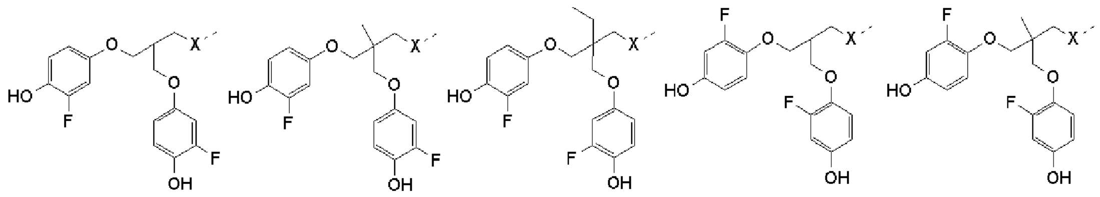

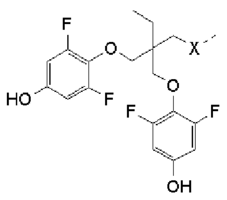 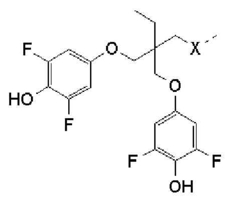 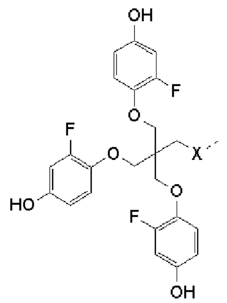 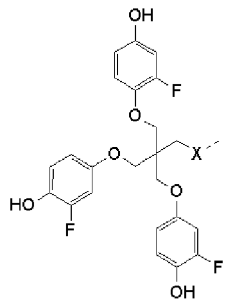

(wherein X represents an oxygen atom or $NR^4$; a dashed line represents a bonding arm; and $R^4$ is as defined above.)

Moreover, in the present invention, the aforementioned polyurethane preferably further contains one or more weakly acidic functional groups represented by the following general formulae (1a) to (1c),

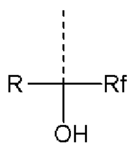 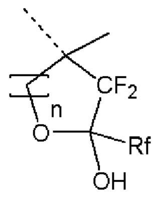 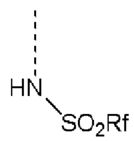

(1a) (1b) (1c)

wherein R represents a hydrogen atom, a fluorine atom, or a linear, branched, or cyclic hydrocarbon group having 1 to 10 carbon atoms optionally substituted with a fluorine atom; Rf represents a fluorine atom, or a linear, branched, or cyclic fluorinated hydrocarbon group having 1 to 10 carbon atoms; "n" is an integer of 1 or 2; and a dashed line represents a bonding arm.

R represents a hydrogen atom, a fluorine atom, or a linear, branched, or cyclic hydrocarbon group having 1 to 10 carbon atoms optionally substituted with a fluorine atom. Specific examples thereof include a hydrogen atom, fluorine atom, methyl group, ethyl group, propyl group, isopropyl group, n-butyl group, sec-butyl group, tert-butyl group, cyclopropyl group, cyclobutyl group, cyclopentyl group, cyclohexyl group, difluoromethyl group, trifluoromethyl group, 2,2,2-trifluoroethyl group, and the like.

Rf represents a fluorine atom, or a linear, branched, or cyclic fluorinated hydrocarbon group having 1 to 10 carbon atoms. Specific examples thereof preferably include a fluorine atom, difluoromethyl group, trifluoromethyl group, 2,2,2-trifluoroethyl group, and the like.

"n" is an integer of 1 or 2.

Specific examples of the weakly acidic functional group (1a) include the following fluoroalcohol-containing groups.

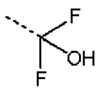 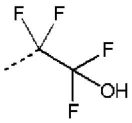 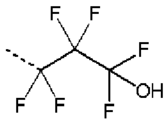 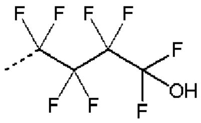 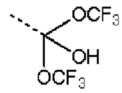

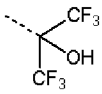 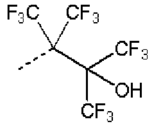 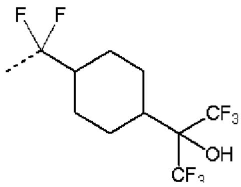 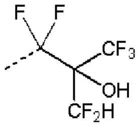 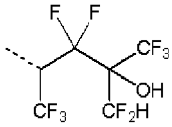

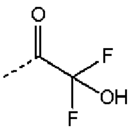 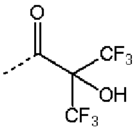 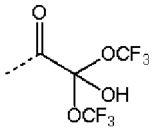 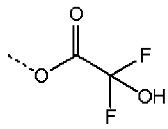 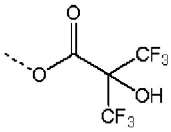 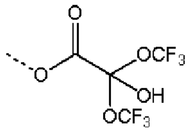

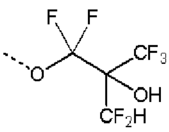 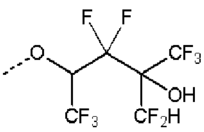 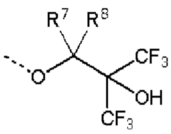 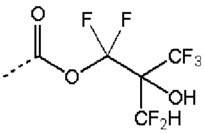 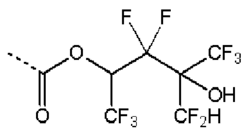

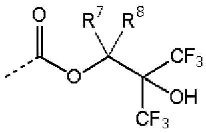 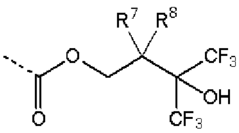 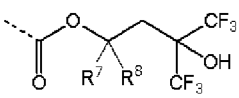 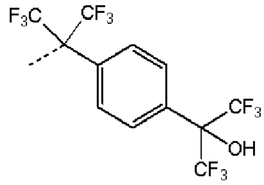

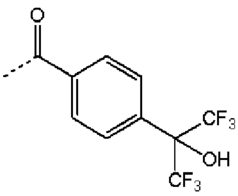 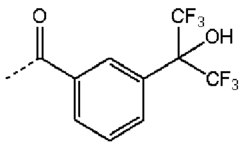 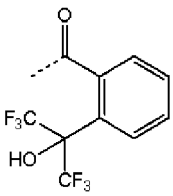 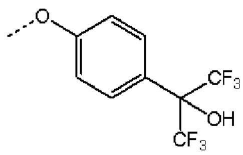

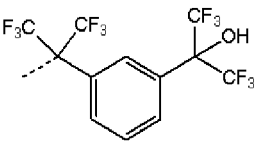 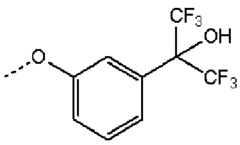 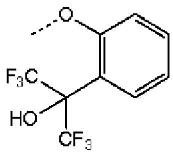

(wherein each of $R^7$ and $R^8$ independently represents a hydrogen atom, or a linear, branched, or cyclic hydrocarbon group having 1 to 6 carbon atoms; $R^7$ and $R^8$ are optionally bonded to each other to form a nonaromatic ring having 3 to 8 carbon atoms together with a carbon atom bonded to $R^7$ and $R^8$; and a dashed line represents a bonding arm.)

Specific examples of the weakly acidic functional group (1b) include the following cyclic fluoroalcoholcontaining groups.

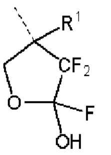 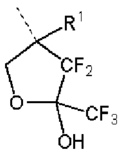 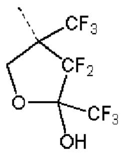 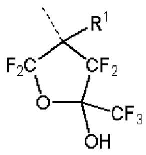 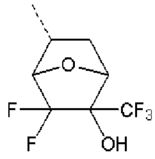 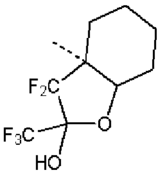

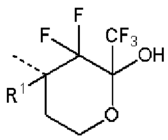 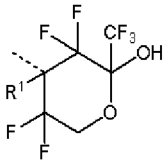 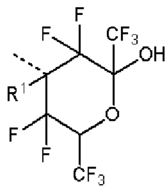 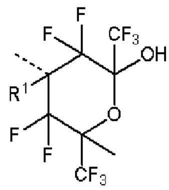 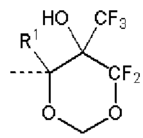

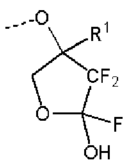 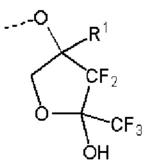 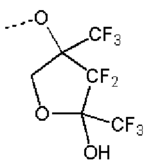 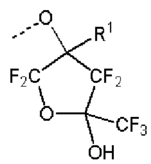 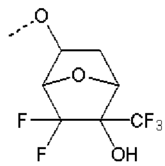 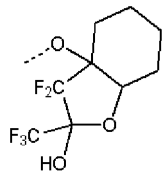

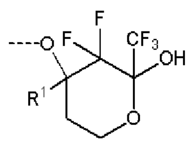 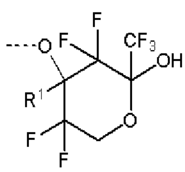 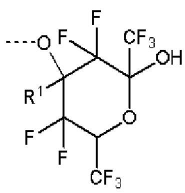 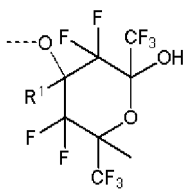 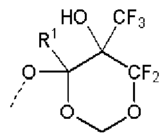

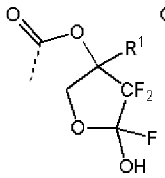 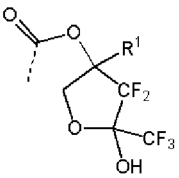 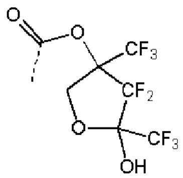 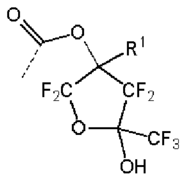 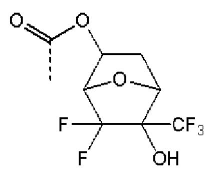 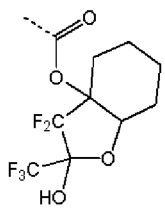

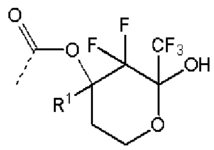 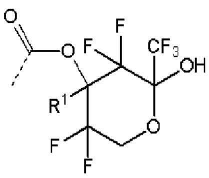 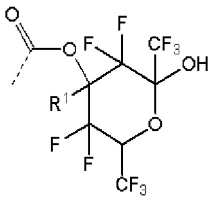 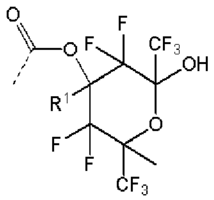 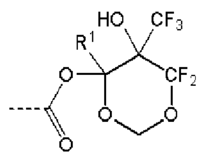

(wherein $R^1$ is a hydrogen atom or a monovalent hydrocarbon group having 1 to 3 carbon atoms; and a dashed line represents a bonding arm.)

Specific examples of the weakly acidic functional group (1c) include the following sulfonamide-containing groups.

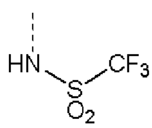 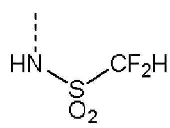 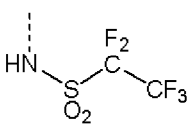 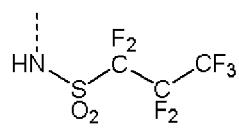

More preferably, the polyurethane of the present invention further contains one or more structures represented by the following general formulae (2a) to (2c),

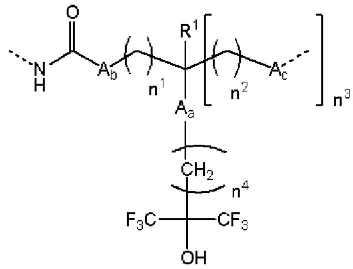
(2a)

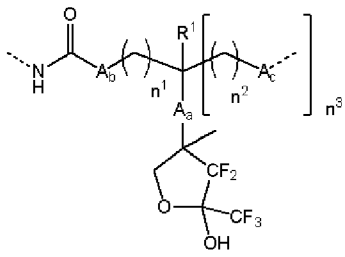
(2b)

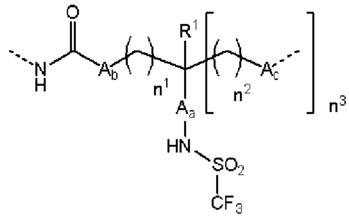
(2c)

wherein $R^1$ is a hydrogen atom, or a monovalent hydrocarbon group having 1 to 3 carbon atoms; $A_a$ represents a single bond, or a linear, branched, or cyclic divalent hydrocarbon group having 1 to 20 carbon atoms, and $-CH_2-$ constituting $A_a$ is optionally substituted with -O-, -C(=O)-, -C(=O)O-, or $-C_6H_4-$, or is optionally $-NR^4$-C(=O)-; each of $A_b$ and $A_c$ independently represents any group selected from the group consisting of -O-, -O-C(=O)-$NR^4$-, $-NR^4$-, and - C(=O)O-; each of $n^1$, $n^2$, and $n^4$ is an integer of 0 to 10; $n^3$ is an integer of 0 or 1; $R^4$ is as defined above; and a dashed line represents a bonding arm.

$R^1$ is a hydrogen atom, or a monovalent hydrocarbon group having 1 to 3 carbon atoms, and is preferably a hydrogen atom, a methyl group, or an ethyl group.

Method for Producing Polyurethane

The aforementioned polyurethane can be obtained, for example, by a reaction between a polyisocyanate and a polyol, polyamine, polycarboxylic acid, or the like according to a known method such as one-shot method or prepolymer method. Preferably, a prepolymer method is employed.

In the prepolymer method, a polyurethane is synthesized through steps of: (a) reacting a diisocyanate and a high-molecular-weight polyol with excess isocyanate groups to obtain a reaction mixture containing a prepolymer having an isocyanate group at a terminal; and (b) reacting the prepolymer with a low-molecular-weight diol, diamine, or dicarboxylic acid (chain extender) to polymerize the prepolymer. Further, a polyisocyanate having three or more reactive groups, or a polyol, polyamine, or polycarboxylic acid having three or more reactive groups can be added to obtain a polyurethane having a crosslink structure.

Alternatively, after the polymerization in (b) is performed with the excess isocyanate groups, a capping agent having a reactive group (such as a hydroxyl group, an amino group, a carboxyl group, or the like) that can react with an isocyanate group can be added to introduce a functional group derived from the capping agent to the urethane terminal.

The polyurethane containing the phenolic hydroxyl group of the present invention can be synthesized by substituting all or part of at least one of the high-molecular-weight polyol, the chain extender, and the capping agent with a compound containing the phenolic hydroxyl group in the reactions of (a) and (b) described above. However, the method preferably comprises: introducing the phenolic hydroxyl group as a capping agent after a chain extension reaction. That is, the method more preferably comprises: introducing the phenolic hydroxyl group to the polyurethane by using an alcohol or amine represented by the following general formula (1C) after the chain extension reaction,

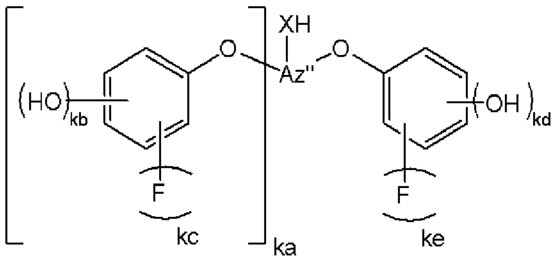
(1C)

wherein Az" represents a linear, branched, or cyclic (ka+2)-valent hydrocarbon group or a fluorinated hydrocarbon group having 1 to 19 carbon atoms, and $-CH_2$-constituting the (ka+2)-valent hydrocarbon group is optionally substituted with -O-, -C(=O)-, or -Si($R^2R^3$)-; X represents an oxygen atom or $NR^4$; "ka" represents an integer of 0 to 2; "kb", "kc", "kd", and "ke" each represent 1 or 2; and $R^2$, $R^3$, and $R^4$ are as defined above.

Az" represents a linear, branched, or cyclic (ka+2)-valent hydrocarbon group or a fluorinated hydrocarbon group having 1 to 19 carbon atoms, and $-CH_2$-constituting the (ka+2)-valent hydrocarbon group is optionally substituted with -O-, -C(=O)-, or -Si($R^2R^3$)-.

X represents an oxygen atom or $NR^4$.

"ka" represents an integer of 0 to 2, and "kb", "kc", "kd", and "ke" each represent 1 or 2.

$R^2$, $R^3$, and $R^4$ are as defined above.

Note that a phenolic hydroxyl group can react with an isocyanate, and thus, in a case of introducing the phenolic hydroxyl group to the high-molecular-weight polyol or the chain extender, it is preferable to form a polyurethan while protecting the phenolic hydroxyl group with a suitable protective group, followed by a deprotection reaction.

Further, in the reactions of (a) and (b), all or part of at least one of the high-molecular-weight polyol, the chain extender, and the capping agent can be substituted with a compound containing the weakly acidic functional group to synthesize the polyurethane containing the phenolic hydroxyl group and the weakly acidic functional group.

Polyisocyanate

Examples of the polyisocyanate as a constituent component of the polyurethane include: aliphatic and alicyclic polyisocyanates, such as ethylene diisocyanate, trimethylene diisocyanate, tetramethylene diisocyanate, pentamethylene diisocyanate, hexamethylene diisocyanate (HDI), octamethylene diisocyanate, nonamethylene diisocyanate, 2-methylpentane-1,5-diisocyanate, 3-methylpentane-1,5-diisocyanate, 2,2'-dimethylpentane diisocyanate, 2,2,4-trimethylhexane diisocyanate, decamethylene diisocyanate, butene diisocyanate, 1,3-butadiene-1,4-diisocyanate, 2,4,4-trimethylhexamethylene diisocyanate, 1,6,11-undecamethylene triisocyanate, 1,3,6-hexamethylene triisocyanate, 1,8-diisocyanate-4-isocyanatomethyloctane, 2,5,7-trimethyl-1,8-diisocyanate-5-isocyanatomethyloctane, bis (isocyanatoethyl)carbonate, bis(isocyanatoethyl)ether, 1,4-butylene glycol dipropyl ether-ω, w'-diisocyanate, lysine isocyanatomethyl ester, lysine triisocyanate, 2-isocyanatoethyl-2,6-diisocyanate hexanoate, 2-isocyanatopropyl-2,6-diisocyanate hexanoate, bis(4-isocyanate-n-butylidene)pentaerythritol, 2,6-diisocyanatemethyl caproate, isophorone diisocyanate (IPDI), 1,3-cyclohexyl diisocyanate, 1,4-cyclohexyl diisocyanate, 1,3-bis(isocyanatomethyl)cyclohexane, 1,4-bis(isocyanatomethyl)cyclohexane, 1,3-bis(isocyanatoethyl)cyclohexane, 1,4-bis(isocyanatoethyl)cyclohexane, methylcyclohexane diisocyanate, 2,2'-dimethyldicyclohexylmethane diisocyanate, 4,4'-dicyclohexylmethane diisocyanate, dimer acid diisocyanate, 2,5-diisocyanatomethylbicyclo[2,2,1]-heptane, 2,6-diisocyanatomethylbicyclo[2,2,1]-heptane, 2-isocyanatomethyl-2-(3-isocyanatopropyl)-5-isocyanatomethylbicyclo-[2,2,1]-heptane, 2-isocyanatomethyl-2-(3-isocyanatopropyl)-6-isocyanatomethylbicyclo-[2,2,1]-heptane, 2-isocyanatomethyl-3-(3-isocyanatopropyl)-5-(2-isocyanatoethyl)-bicyclo-[2,2,1]-heptane, 2-isocyanatomethyl-3-(3-isocyanatopropyl)-6-(2-isocyanatoethyl)-bicyclo-[2,2,1]-heptane, 2-isocyanatomethyl-2-(3-isocyanatopropyl)-5-(2-isocyanatoethyl)-bicyclo-[2,2,1]-heptane, and 2-isocyanatomethyl-2-(3-isocyanatopropyl)-6-(2-isocyanatoethyl)-bicyclo-[2,2,1]-heptane; aromatic polyisocyanates, such as 2,4-tolylene diisocyanate or 2,6-tolylene diisocyanate and isomer mixture thereof (TDI), 4,4'-diphenylmethane diisocyanate or 2,4'-diphenylmethane diisocyanate and isomer mixture thereof (MDI), toluidine diisocyanate (TODI), para-phenylene diisocyanate, naphthalene diisocyanate (NDI), and 4,4'-dibenzyl diisocyanate; aromatic-aliphatic polyisocyanates, such as ortho-xylylene diisocyanate, meta-xylylene diisocyanate, para-xylylene diisocyanate, 1,3-tetramethylxylylene diisocyanate, and 1,4-tetramethylxylylene diisocyanate; polymers thereof prepared as urethane-modified products, biuret-modified products, carbodiimide-modified products, uretoniminemodified products, uretdione-modified products, isocyanurate-modified products, or allophanate-modified products; and the like.

The above polyisocyanates may be used individually or two or more kinds thereof may be used in combination.

High-Molecular-Weight Polyol

A polyol having a number-average molecular weight of 500 to 5,000 and having two or more hydroxyl groups that can react with an isocyanate group can be used as the high-molecular-weight polyol that is a constituent component of the polyurethane. Examples thereof include polyether polyol, polyester polyol, polycarbonate polyol, acrylic polyol, polyolefin having a terminal hydroxyl group, silicone polyol, vegetable oil-based polyol, and the like.

Examples of the polyether polyol include polyoxypropylene glycols, polyoxyethylene glycols, polyoxytetramethylene glycols, copolymers thereof, and the like.

The polyoxypropylene glycols and polyoxyethylene glycols are alkylene-oxide addition polymers using a low-molecular-weight polyol, polyamine, or amino alcohol as an initiator. Examples of the low-molecular-weight polyol include: dihydric alcohols, such as ethylene glycol, 1,2-propanediol, 1,3-propanediol, 1,2-butanediol, 1,3-butanediol, 1,4-butanediol, 1,5-pentanediol, 1,6-hexanediol, 1,7-heptanediol, 1,8-octanediol, 1,9-nonanediol, neopentyl glycol, 3-methyl-1,5-pentanediol, 3,3-dimethylolheptane, 2,2,2-trimethylpentanediol, 1,4-dihydroxy-2-butene, 2,6-dimethyl-1-octene-3,8-diol, diethylene glycol, triethylene glycol, dipropylene glycol, cyclohexane-1,3-diol, cyclohexane-1,4-diol, cyclohexane-1,3-dimethanol, cyclohexane-1,4-dimethanol, 1,3-adamantanedimethanol, dimer acid diol, 1,2-benzenediol, 1,3-benzenediol, 1,4-benzenediol, hydroquinone di(2-hydroxyethyl ether), bisphenol A, bis(β-hydroxyethyl)benzene, and xylylene glycol; trihydric alcohols, such as glycerin, trimethylolpropane, and triisopropanolamine; tetrahydric alcohols, such as pentaerythritol, α-methyl glucoside, and diglycerin; and polyhydric alcohols, such as sorbitol and sucrose. Examples of

the low-molecular-weight amino alcohol include monoethanolamine, dimethanolamine, and triethanolamine. Examples of the low-molecular-weight polyamine include ethylenediamine, propylenediamine, butanediamine, pentamethylenediamine, hexamethylenediamine, isophorone diamine, piperazine, toluenediamine, metaphenylenediamine, diphenylmethanediamine, xylylenediamine, dimethylthiotoluenediamine, 4,4-methylenebis-o-chloroaniline, and the like. These initiators may be used individually or two or more kinds thereof may be used in combination.

Further, the low-molecular-weight polyol containing the aforementioned phenolic hydroxyl group or the weakly acidic functional group can be used to obtain a polyether polyol containing the phenolic hydroxyl group or the weakly acidic functional group. Note that polymerization is preferably performed while protecting the phenolic hydroxyl group and the weakly acidic functional group, followed by deprotection.

Examples of the alkylene oxide include ethylene oxide, propylene oxide, butylene oxide, and the like. One or a combination of two or more thereof can be used. Polyoxyalkylene polyol obtained from a combination of two or more kinds may have either a block or random structure.

The polyoxytetramethylene glycol is a ring-opening polymerization product obtained through cationic polymerization of tetrahydrofuran (THF). Examples thereof include crystalline polyoxytetramethylene glycol, amorphous polyoxytetramethylene glycol obtained by copolymerizing THF with alkyl-substituted tetrahydrofuran such as 3-methyltetrahydrofuran, the aforementioned dihydric alcohols, or the like.

Examples of the polyester polyol include: polymerization condensates between any of the aforementioned low-molecular-weight polyols and polyhydric carboxylic acid or oligomer acid; and ring-opening polymers of lactone or lactide using the low-molecular-weight polyol as an initiator.

Examples of the polyhydric carboxylic acid include oxalic acid, malonic acid, succinic acid, glutaric acid, adipic acid, pimelic acid, suberic acid, glutaconic acid, azelaic acid, sebacic acid, 1,1-dimethyl-1,3-dicarboxypropane, 3-methyl-3-ethylglutaric acid, 1,4-cyclohexyldicarboxylic acid, hexahydrophthalic acid, maleic acid, fumaric acid, itaconic acid, muconic acid, α-hydromuconic acid, β-hydromuconic acid, phthalic acid, ortho-phthalic acid, terephthalic acid, isophthalic acid, toluenedicarboxylic acid, naphthalenedicarboxylic acid, HET acid, dimer acid, and hydrogenated dimer acid. It is also possible to use a derivative, acid anhydride, or acid halide thereof.

Examples of the lactone include β-propiolactone, β-butyrolactone, γ-butyrolactone, γ-valerolactone, δ-valerolactone, ε-caprolactone, and the like. Examples of the lactide include L-lactide and D-lactide.

One kind of the low-molecular-weight polyol, polyhydric carboxylic acid, oligomer acid, lactone, or lactide, which are constituent components of the polyester polyol, may be used, or two or more kinds thereof may be used in combination.

Further, it is also possible to use polyesteramide polyol obtained by substituting a part of the low-molecular-weight polyol of the polyester polyol with the aforementioned low-molecular-weight polyamine or amino alcohol.

Examples of the polycarbonate polyol include polymerization condensates between the aforementioned polyols and the carbonate compound.

Examples of the carbonate compound include dimethyl carbonate, diethyl carbonate, ethylene carbonate, propylene carbonate, diphenyl carbonate, dinaphthyl carbonate, dianthryl carbonate, diphenanthryl carbonate, diindanyl carbonate, tetrahydronaphthyl carbonate, and the like.

One kind of the low-molecular-weight polyol or carbonate compound, which are constituent components of the polycarbonate polyol, may be used, or two or more kinds thereof may be used in combination.

Examples of the acrylic polyol include copolymers obtained by copolymerizing hydroxy group-containing (meth)acrylate with vinyl monomer.

Examples of the hydroxyl group-containing (meth)acrylate include 2-hydroxyethyl (meth)acrylate, hydroxypropyl (meth)acrylate, hydroxybutyl (meth)acrylate, 3-hydroxy-2,2-dimethylpropyl acrylate, 2,2-dihydroxymethylbutyl (meth)acrylate, pentaerythritol tri(meth)acrylate, polyhydroxyalkyl maleate, polyhydroxyalkyl fumarate, and the like. These hydroxyl group-containing (meth)acrylates may be used individually or two or more kinds thereof may be used in combination.

Examples of the vinyl monomer include:

(meth)acrylic esters, such as methyl (meth)acrylate, ethyl(meth)acrylate, propyl(meth)acrylate, isopropyl(meth)acrylate, butyl(meth)acrylate, isobutyl(meth)acrylate, s-butyl(meth)acrylate, t-butyl(meth)acrylate, pentyl(meth)acrylate, isopentyl(meth)acrylate, hexyl(meth)acrylate, 2-ethylhexl(meth)acrylate, octyl(meth)acrylate, nonyl(meth)acrylate, isononyl(meth)acrylate, decyl(meth)acrylate, dodecyl(meth)acrylate, cyclohexyl(meth)acrylate, phenyl(meth)acrylate, benzyl(meth)acrylate, allyl(meth)acrylate, ethylene glycol di(meth)acrylate, butylene glycol di(meth)acrylate, hexanediol di(meth)acrylate, and oligo ethylene glycol di(meth)acrylate; aromatic vinyls, such as styrene, vinyltoluene, and α-methylstyrene; vinyl cyanides, such as (meth)acrylonitrile; carboxyl group-containing vinyl monomers, such as fumaric acid, maleic acid, and itaconic acid, or alkyl esters thereof; isocyanate group-containing vinyl monomers, such as 3-(2-isocyanato-2-propyl)-α-methylstyrene; fluorinecontaining vinyl monomers, such as tetrafluoroethylene, chlorotrifluoroethylene, trichlorofluoroethylene, hexafluoropropylene, vinylidene fluoride, vinyl fluoride, and trifluoromethyltrifluoroethylene; silicone monomers, such as γ-(meth)acryloxypropyltrimethoxysilane; and the like. These vinyl monomers may be used individually or two or more kinds thereof may be used in combination.

Examples of the polyolefin having a terminal hydroxyl group include compounds obtained by attaching a hydroxyl group to a terminal of one or more olefin polymers. Examples of the olefin include ethylene, propylene, butadiene, isoprene, styrene, acrylonitrile, vinyl ether, and vinyl acetate. Further, part of these structures may be substituted with a halogen, such as fluorine, chlorine, or bromine.

Examples of the silicone polyol include: vinyl group-containing silicone compounds obtained by polymerizing $\gamma$-methacryloxypropyltrimethoxysilane or the like; and polysiloxanes having at least one terminal hydroxyl group per molecule, such as $\alpha,\omega$-dihydroxypolydimethylsiloxane and $\alpha,\omega$-dihydroxypolydiphenylsiloxane.

Examples of the vegetable oil-based polyol include: hydroxyl group-containing vegetable oils, such as castor oil and coconut oil; ester-modified castor oil polyol, dehydrated castor oil, partially dehydrated castor oil, and hydrogenated castor oil obtained from a reaction between castor oil fatty acid and polyol; and the like.

The high-molecular-weight polyol has a number-average molecular weight in a range of preferably 500 or more and 5,000 or less, more preferably 1,000 or more and 3,000 or less, further preferably 1,000 or more and 2,000 or less.

When the number-average molecular weight of the high-molecular-weight polyol is at the lower limit or more, it is possible to suppress excessive increase in the urethane group concentration in the polyurethane, increase in the hardness due to this concentration increase, and decrease in performance such as stretchability. In addition, when the number-average molecular weight is at the upper limit or less, it is possible to suppress excessive decrease in the urethane group concentration, prevent the strength attributable to the urethane bond from decreasing, and achieve both appropriate strength and stretchability.

The high-molecular-weight polyol is preferably bifunctional polyol or trifunctional polyol, more preferably bifunctional polyol.

Chain Extender

Examples of the chain extender to be employed include: dihydric alcohols, such as ethylene glycol, 1,2-propanediol, 1,3-propanediol, 1,2-butanediol, 1,3-butanediol, 1,4-butanediol, 1,5-pentanediol, 1,6-hexanediol, 1,7-heptanediol, 1,8-octanediol, 1,9-nonanediol, neopentyl glycol, 3-methyl-1,5-pentanediol, 3,3-dimethylolheptane, 2,2,2-trimethylpentanediol, 1,4-dihydroxy-2-butene, 2,6-dimethyl-1-octene-3,8-diol, diethylene glycol, triethylene glycol, dipropylene glycol, cyclohexane-1,3-diol, cyclohexane-1,4-diol, cyclohexane-1,3-dimethanol, cyclohexane-1,4-dimethanol, 1,3-adamantanedimethanol, dimer acid diol, 1,2-benzenediol, 1,3-benzenediol, 1,4-benzenediol, hydroquinone di(2-hydroxyethyl ether), bisphenol A, bis($\beta$-hydroxyethyl)benzene, and xylylene glycol; amines, such as ethanolamine, dimethanolamine, triethanolamine, ethylenediamine, propylenediamine, butanediamine, pentamethylenediamine, hexamethylenediamine, isophorone diamine, piperazine, toluenediamine, metaphenylenediamine, diphenylmethanediamine, xylylenediamine, dimethylthiotoluenediamine, and 4,4-methylenebis-o-chloroaniline; and the like.

Further, one or more alcohols represented by the following general formulae (3a) to (3c) may be used as the chain extender to introduce the weakly acidic functional group to the polyurethane. The chain extender may be the polyol represented by (3a) to (3c), or may be a combination with the above-listed chain extenders,

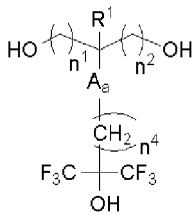
(3a)

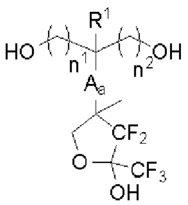
(3b)

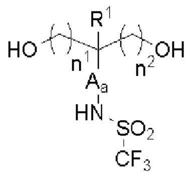
(3c)

wherein $R^1$ is a hydrogen atom, or a monovalent hydrocarbon group having 1 to 3 carbon atoms; $A_a$ represents a single bond, or a linear, branched, or cyclic divalent hydrocarbon group having 1 to 20 carbon atoms, and $-CH_2-$ constituting $A_a$ is optionally substituted with -O-, -C(=O)-, -C(=O)O-, or $-C_6H_4-$, or is optionally $-NR^4-C(=O)-$; $R^4$ is a hydrogen atom, or a linear or branched alkyl group having 1 to 4 carbon atoms; and each of $n^1$, $n^2$, and $n^4$ is an integer of 0 to 10.

By using such a chain extender, the polyurethane containing the structures represented by the above general formulae (2a) to (2c) can be obtained.

$R^1$ is a hydrogen atom or a monovalent hydrocarbon group having 1 to 3 carbon atoms, preferably a hydrogen atom, a methyl group, or an ethyl group.

$A_a$ represents a single bond, or a linear, branched, or cyclic divalent hydrocarbon group having 1 to 20 carbon atoms, and $-CH_2-$ constituting $A_a$ is optionally substituted with -O-, -C(=O)-, -C(=O)O-, or $-C_6H_4-$, or is optionally $-NR^4-C(=O)-$. Specific examples of the linear, branched, or cyclic divalent hydrocarbon group having 1 to 20 carbon atoms of $A_a$ include the following.

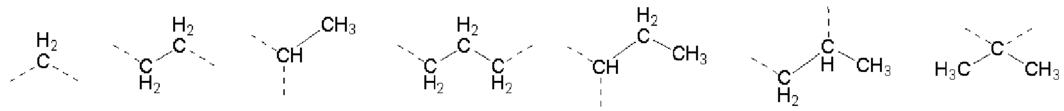

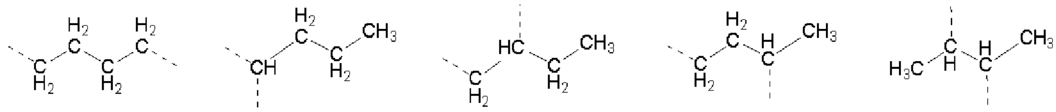

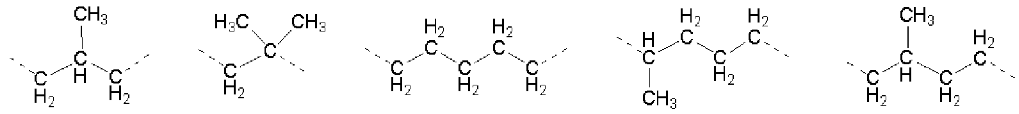

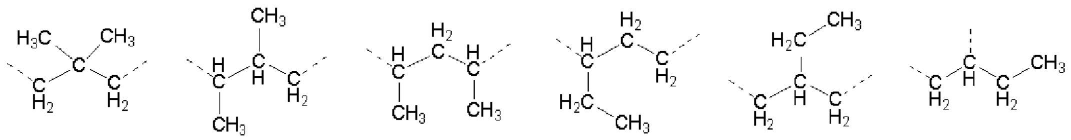

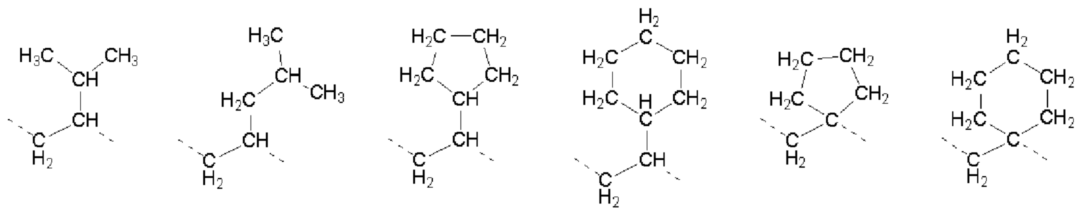

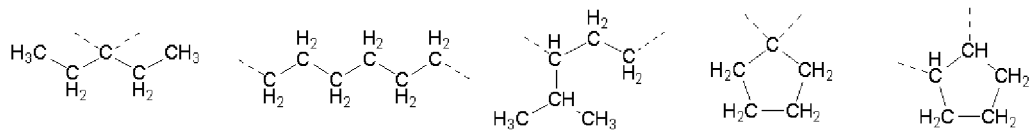

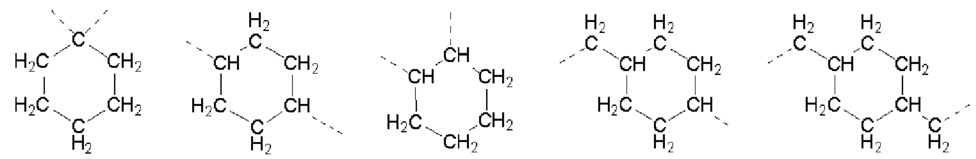

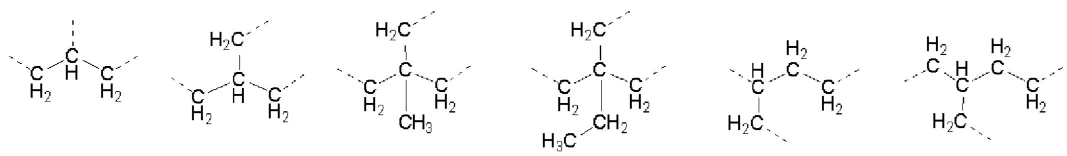

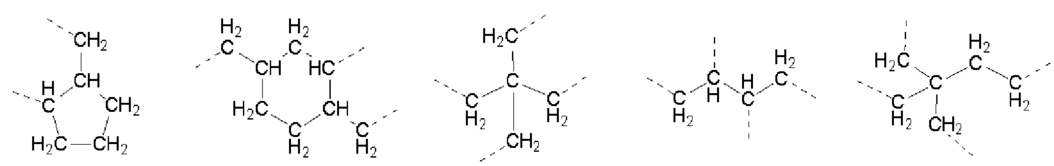

Further examples thereof include the one in which any of $-CH_2-$ of $A_a$ described above is substituted with - O-, -C(=O)-, -C(=O)O-, $-C_6H_4-$, or $-NR^4-C(=O)-$. $R^4$ is a hydrogen atom, or a linear or branched alkyl group having 1 to 4 carbon atoms, and each of $n^1$, $n^2$, and $n^4$ is an integer of 0 to 10.

Specific examples of the polyols represented by the above general formulae (3a) to (3c) include the following, but are not limited thereto.

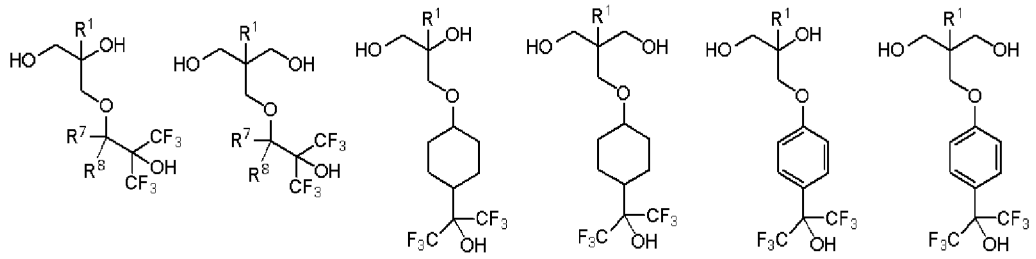

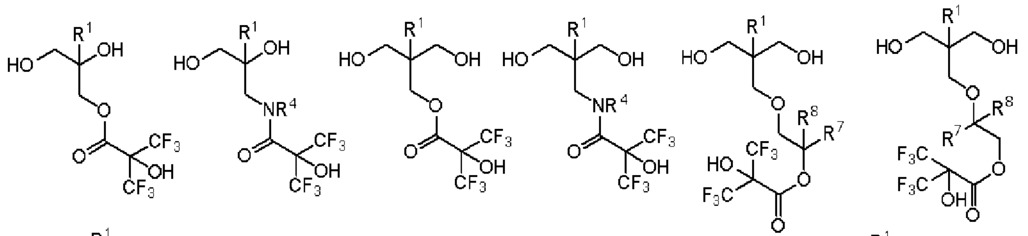

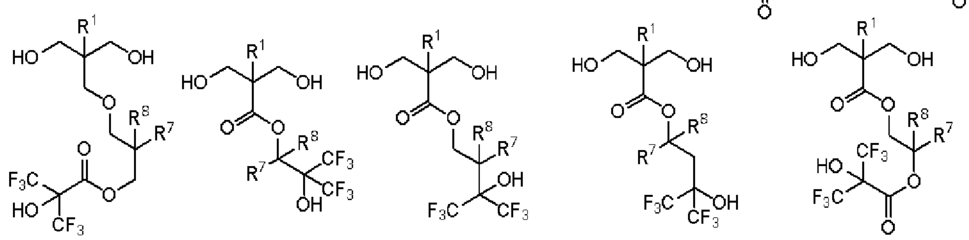

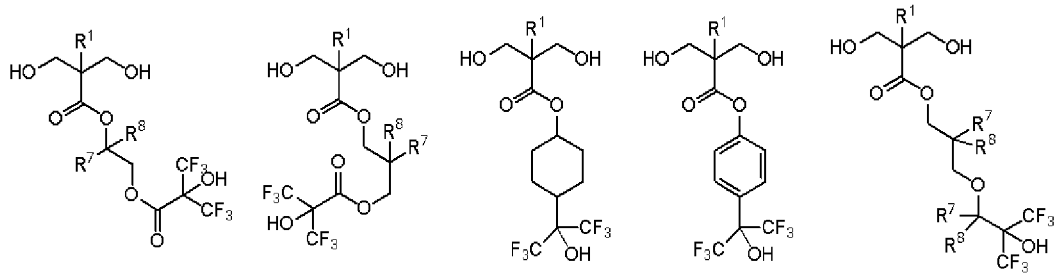

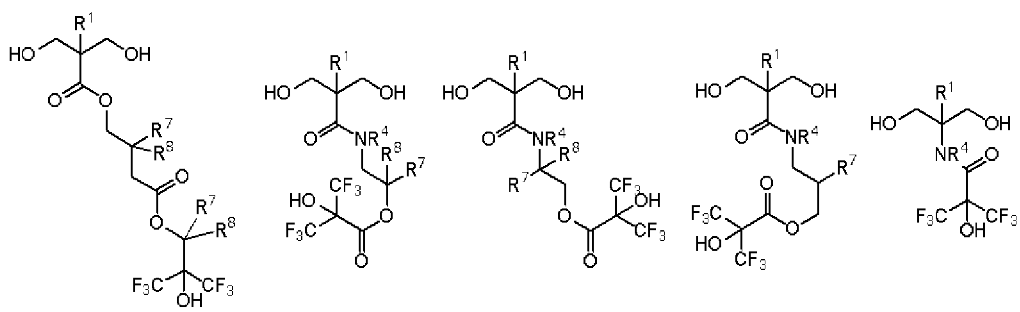

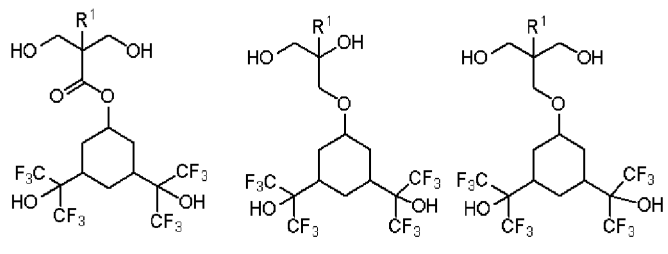

(wherein $R^1$, $R^4$, $R^7$, and $R^8$ are as defined above.)

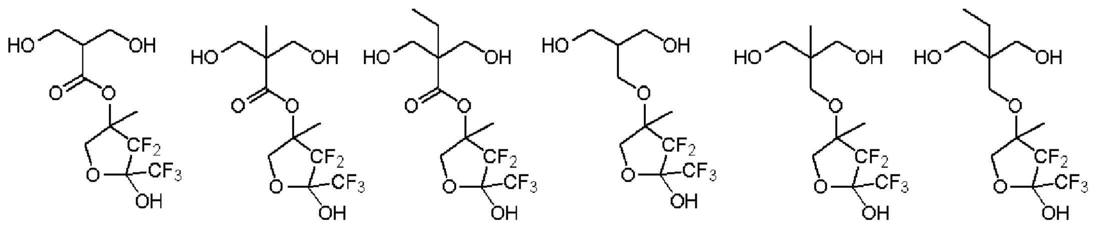

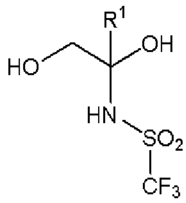

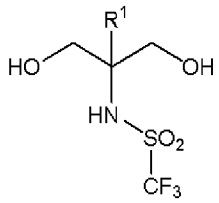

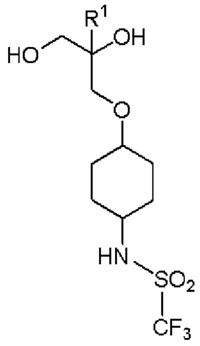

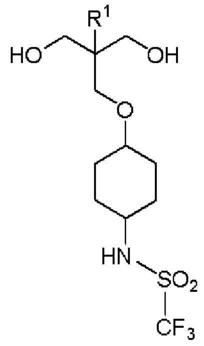

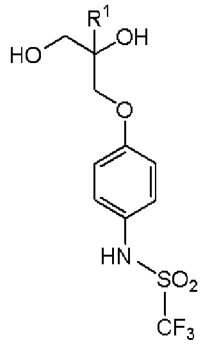

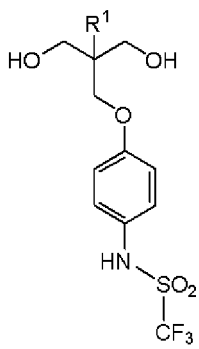

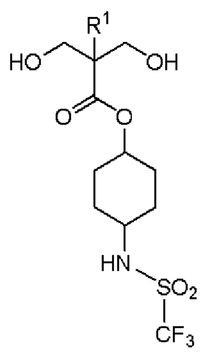

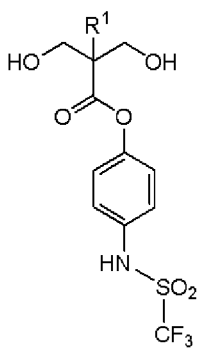

(wherein $R^1$ is as defined above.)

The chain extender having the weakly acidic functional group is used in an amount of preferably 5 to 50 mass%, more preferably 20 to 40 mass%, relative to a total amount of the constituent components of the urethane.

Crosslinking Agent

Examples of a crosslinking agent to be used include: trihydric alcohols, such as glycerin, trimethylolpropane, and triisopropanolamine; and polyhydric alcohols, such as pentaerythritol, α-methyl glucoside, and diglycerin.

The crosslinking agent is added in an amount within preferably 0 to 5 mass%, more preferably 0 to 3 mass%, relative to the total amount of the constituent components of the urethane.

When the crosslinking agent is added in an amount at the upper limit or less, the strength does not increase excessively, and the flexibility and stretchability would not be impaired. Thus, the crosslinking agent is suitable for use in the conductive paste composition containing the polyurethane of the present invention.

Organic Solvent

The polyurethane is synthesized by bulk polymerization or solution polymerization. Examples of an organic solvent used in the solution polymerization include: aromatic hydrocarbon solvents, such as toluene, xylene, cumene, 1,2,3-trimethylbenzene, 1,2,4-trimethylbenzene, 1,3,5-trimethylbenzene, styrene, α-methylstyrene, butylbenzene, sec-butylbenzene, isobutylbenzene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tert-amylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, and 1,3,5-triethylbenzene; aliphatic hydrocarbon solvents, such as n-heptane, isoheptane, n-hexane, octane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, cyclohexane, methylcyclohexane, 1-methyl-1-cyclohexene, 3-methyl-1-cyclohexene, methylenecyclohexane, 4-methyl-1-cyclohexene, 2-methyl-1-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-trimethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonyne, 2-nonyne, 3-nonyne, 4-nonyne, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, $\alpha$-phellandrene, pinene, terpinene, tetrahydrodicyclopentadiene, 5,6-dihydrodicyclopentadiene, dicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.0$^{2,7}$]undeca-4-ene, n-dodecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, and isoparaffin; ketone solvents, such as cyclohexanone, cyclopentanone, acetone, methyl ethyl ketone, 2-octanone, 2-nonanone, 2-heptanone, 3-heptanone, 4-heptanone, 2-hexanone, 3-hexanone, diisobutyl ketone, methyl isobutyl ketone, methylcyclohexanone, and methyl n-pentyl ketone; ether solvents, such as ethylene glycol dimethyl ether, propylene glycol dimethyl ether, diethylene glycol dimethyl ether, diethylene glycol diethyl ether, diethylene glycol dibutyl ether, diethylene glycol ethyl methyl ether, diethylene glycol butyl methyl ether, diethyl ether, diisopropyl ether, di-n-butyl ether, di-sec-butyl ether, diisobutyl ether, amyl ether, isoamyl ether, di-tert-amyl ether, methyl cyclopentyl ether, methyl cyclohexyl ether, methyl-tert-butyl ether, di-n-hexyl ether, anisole, dihydroterpinyl acetate, tetrahydrofuran, and dioxane; ester solvents, such as ethylene glycol monomethyl ether acetate, ethylene glycol monoethyl ether acetate, ethylene glycol monobutyl ether acetate, ethylene glycol monopropyl ether acetate, ethylene glycol diacetate, propylene glycol monomethyl ether acetate, propylene glycol monoethyl ether acetate, propylene glycol monopropyl ether acetate, propylene glycol monobutyl ether acetate, propylene glycol mono-tert-butyl ether acetate, propylene glycol diacetate, diethylene glycol monomethyl ether acetate, diethylene glycol monoethyl ether acetate, diethylene glycol monopropyl ether acetate, diethylene glycol monobutyl ether acetate, methyl acetate, ethyl acetate, butyl acetate, isobutyl acetate, acetate-tert-butyl, ethyl pyruvate, methyl 3-methoxypropionate, ethyl 3-ethoxypropionate, tert-butyl propionate, 3-methoxybutyl acetate, and ethyl-3-ethoxypropionate; lactone solvents such as $\gamma$-butyrolactone; nitrile solvents such as acetonitrile; halogen-based solvents, such as methyl chloride, methylene chloride, chloroform, carbon tetrachloride, 1,2-dichloroethane, and 1,1,2,2-tetrachloroethane; aprotic polar solvents, such as N-methylpyrrolidone, N,N'-dimethylformamide, N,N'-dimethylacetamide, dimethyl sulfoxide, and hexamethylphosphoramide; and the like.

Note that the organic solvent is added in an amount within preferably 20 parts by mass or more and 500 parts by mass or less, more preferably 25 parts by mass or more and 100 parts by mass or less, relative to 100 parts by mass of the total amount of the polyurethane constituent components (polyisocyanate, high-molecular-weight polyol, chain extender, crosslinking agent).

The organic solvent may be removed by distillation under reduced pressure or by crystallization after the polymerization reaction, or may be applied to a conductive paste composition in a form of polyurethane solution without removal.

Catalyst

In the polyurethane synthesis, a catalyst is preferably added to promote a urethane bond formation reaction, as necessary.

The catalyst to be used for the urethane formation can be appropriately selected from known catalysts. Examples thereof include amine-based catalysts, ammonium salt-based catalysts, potassium salt-based catalysts, organometallic catalysts, and the like.

Examples of the amine-based catalysts include triethylamine, N,N-dimethylcyclohexylamine, triethylenediamine, 2-methyltriethylenediamine, N,N,N',N'-tetramethylethylenediamine, N,N,N',N'-tetramethylpropylenediamine, N,N,N',N",N"-pentamethyldiethylenetriamine, N,N,N',N",N"-pentamethyl-(3-aminopropyl)ethylenediamine, N,N,N',N",N"-pentamethyldipropylenetriamine, N,N,N',N'-tetramethylhexamethylenediamine, bis(2-dimethylaminoethyl)ether, dimethylethanolamine, dimethylisopropanolamine, dimethylaminoethoxyethanol, N,N-dimethylhexanolamine, N,N-dimethyl-N'-(2-hydroxyethyl)ethylenediamine, N,N-dimethyl-N'-(2-hydroxyethyl)propanediamine, N,N,N'-trimethylaminoethylethanolamine, bis(dimethylaminopropyl)amine, bis(dimethylaminopropyl)isopropanolamine, N-methyl-N'-(2-dimethylaminoethyl)piperazine, N-methyl-N'-(2-hydroxyethyl)piperazine, N-methylmorpholine, N-ethylmorpholine, 1-methylimidazole, 1,2-dimethylimidazole, 1-isobutyl-2-methylimidazole, 1-dimethylaminopropylimidazole, 1-(2-hydroxyethyl)imidazole, 1-(2-hydroxypropyl)imidazole, 1-(2-hydroxyethyl)-2-methylimidazole, 1-(2-hydroxypropyl)-2-methylimidazole, and the like.

Examples of the ammonium salt-based catalysts include: quaternary ammonium salts, such as tetraethyl hydroxyl ammonium; ammonium salts of 1,8-diazabicyclo(5,4,0)-undecene-7 or 1,5-diazabicyclo(4,3,0)-nonene-5 with octylic acid, oleic acid, p-toluenesulfonic acid, formic acid, phenolic acid, ortho-phthalic acid, acetic acid, maleic acid, or boric acid; and the like.

Examples of the potassium salt-based catalysts include potassium carbonate, potassium acetate, potassium octylate, and the like.

Examples of the organometallic catalysts include: organotin compounds, such as tin acetate, tin octylate (tin 2-ethylhexaonate), tin oleate, tin laurate, dibutyltin diacetate, dimethyltin dilaurate, dibutyltin dilaurate, dibutyltin dimercaptide, dibutyltin maleate, dibutyltin dilaurate, dibutyltin dineodecanoate, dioctyltin dimercaptide, dioctyltin dilaurate, and dibutyltin dichloride; organolead compounds, such as lead octoate and lead naphthenate; organonickel compounds such as nickel naphthenate; organocobalt compounds, for example, cobalt naphthenate and the like; organocopper compounds such as copper octenoate; organobismuth compounds, such as bismuth octylate and bismuth neodecanoate; and the like.

Since the polyurethane of the present invention contains the weakly acidic functional group and thus may be capable of inhibiting the activity of a basic catalyst, it is preferable to use an organometallic catalyst, and more preferable example thereof is an organobismuth compound.

These catalysts for the urethane formation may be used individually or two or more kinds thereof may be used in combination. Note that the catalyst for the urethane formation is used in an amount within preferably 0 parts by mass or more and 5 parts by mass or less, more preferably 0.1 parts by mass or more and 2 parts by mass or less, relative to 100 parts by mass of the total amount of the polyurethane constituent components.

Capping Agent

To the polyurethane according to the present embodiments after the polymerization with excess isocyanate groups, a terminal capping agent can be added to introduce a functional group derived from the capping agent to the polyurethane terminal.

For example, as the capping agent for the polyurethane terminal, the following hydroxy(meth)acrylates can be used to synthesize urethane acrylate,

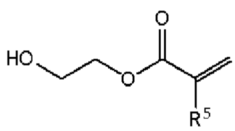 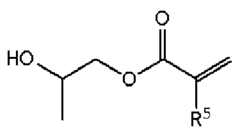 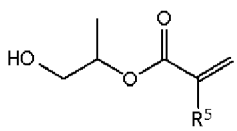 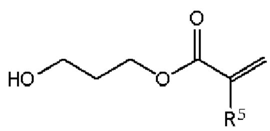

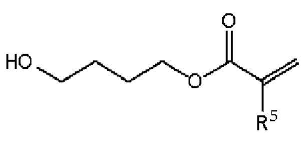 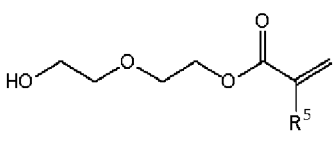 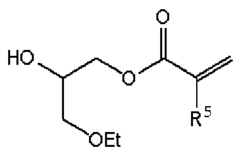

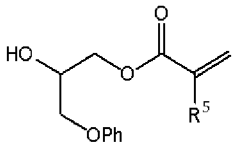 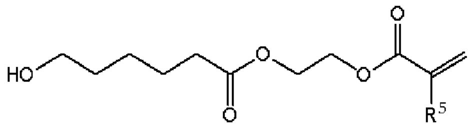 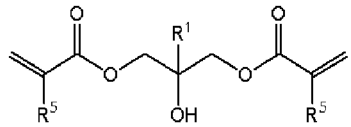

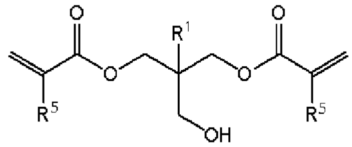 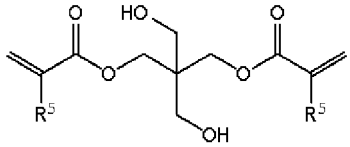 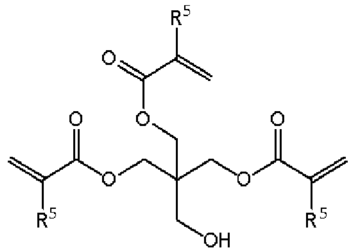

wherein $R^5$ is a hydrogen atom or a methyl group; Et is an ethyl group; Ph is a phenyl group; and $R^1$ is as defined above.

$R^5$ is a hydrogen atom or a methyl group.

The urethane acrylate, together with a reactive monomer and a polymerization initiator as necessary, can be heated or irradiated with active energy beam, such as ultraviolet ray, visible light, laser beam, electron beam, X-ray, $\gamma$-ray, plasma, or microwave, and then, polymerized and cured to form a cured product.

Examples of the reactive monomer to be used include: alkyl(meth)acrylates, such as methyl(meth)acrylate, ethyl(meth)acrylate, isopropyl(meth)acrylate, n-butyl(meth)acrylate, t-butyl(meth)acrylate, isobutyl(meth)acrylate, isoamyl(meth)acrylate, lauryl(meth)acrylate, dodecyl (meth)acrylate, stearyl acrylate, cyclohexyl(meth)acrylate, isobornyl(meth)acrylate, and adamantyl(meth)acrylate; aryloxyalkyl(meth)acrylates, such as phenoxymethyl(meth)acrylate and phenoxyethyl(meth)acrylate; (meth)acrylic aralkyl esters, such as benzyl(meth)acrylate and phenethyl(meth)acrylate; monofunctional (meth)acrylate compounds, such as phenyl(meth)acrylate, 2-hydroxyethyl(meth)acrylate, 2-hydroxypropyl(meth)acrylate, 3-hydroxypropyl(meth)acrylate, and 4-hydroxybutyl(meth)acrylate; tri(meth)acrylates, such as butanediol di(meth)acrylate, hexanediol di(meth)acrylate, octanediol di(meth)acrylate, nonanediol di(meth)acrylate, dodecanediol di(meth)acrylate, ethoxylated hexanediol di(meth)acrylate, propoxylated hexanediol di(meth)acrylate, diethylene glycol di(meth)acrylate, polyethylene glycol di(meth)acrylate, polypropylene glycol di(meth)acrylate, neopentyl glycol di(meth)acrylate, ethoxylated neopentyl glycol di(meth)acrylate, trimethylolpropane tri(meth)acrylate, ethoxylated trimethylolpropane tri(meth)acrylate, propoxylated trimethylolpropane tri(meth)acrylate, tris(2-hydroxyethyl)isocyanurate tri(meth)acrylate, and glycerin tri(meth)acrylate; polyfunctional (meth)acrylate compounds, such as pentaerythritol tri(meth)acrylate, dipentaerythritol tri(meth)acrylate, ditrimethylolpropane tri(meth)acrylate, pentaerythritol tetra(meth)acrylate, ditrimethylolpropane tetra(meth)acrylate, dipentaerythritol tetra(meth)acrylate, dipentaerythritol penta(meth)acrylate, ditrimethylolpropane penta(meth)acrylate, dipentaerythritol hexa(meth)acrylate, and ditrimethylolpropane hexa(meth)acrylate; and ethyleneoxy-modified products, propyleneoxy-modified products, and lactone-modified products thereof. One of these may be used alone, or two or more thereof may be used in combination.

Examples of the polymerization initiator include acetophenone, 2,2-diethoxyacetophenone, p-dimethylaminoacetophenone, benzophenone, 2-chlorobenzophenone, 4,4'-bisdiethylaminobenzophenone, benzoin ethyl ether, benzoin-n-propyl ether, benzoin isopropyl ether, benzoin isobutyl ether, benzoin-n-butyl ether, benzoin dimethyl ketal, thioxanthone, p-isopropyl-$\alpha$-hydroxyisobutylphenone, 2,2-dimethoxy-2-phenylacetophenone, 1-hydroxycyclohexyl phenyl ketone, 2-methyl-1-[4-(methylthio)phenyl]-2-morpholinopropan-1-one, 2-hydroxy-2-methyl-1-phenylpropan-1-one, 2,4,6-trimethylbenzophenone, 4-methylbenzophenone, (2,4,6-trimethylbenzoyl)-diphenylphosphine oxide, bis(2,4,6-trimethylbenzoyl)-phenylphosphine oxide, 2,2-dimethoxy-1,2-diphenylethanone, and the like. Preferably, 1-hydroxycyclohexyl phenyl ketone or bis(2,4,6-trimethylbenzoyl)phenylphosphine oxide is used. One of these may be used alone, or two or more thereof may be used in combination.

Further, the phenolic hydroxyl group can be introduced to a polyurethane by using an alcohol or amine represented by the following general formula (1C) after a chain extension reaction,

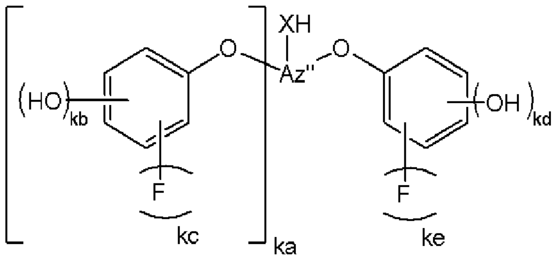 (1C)

wherein Az" represents a linear, branched, or cyclic (ka+2)-valent hydrocarbon group or a fluorinated hydrocarbon group having 1 to 19 carbon atoms, and $-CH_2-$constituting the (ka+2)-valent hydrocarbon group is optionally substituted with -O-, -C(=O)-, or $-Si(R^2R^3)-$; X represents an oxygen atom or $NR^4$; "ka" represents an integer of 0 to 2; "kb", "kc", "kd", and "ke" each represent 1 or 2; and $R^2$, $R^3$, and $R^4$ are as defined above.

Az" represents a linear, branched, or cyclic (ka+2)-valent hydrocarbon group or a fluorinated hydrocarbon group having 1 to 19 carbon atoms, and $-CH_2-$constituting the (ka+2)-valent hydrocarbon group is optionally substituted with -O-, -C(=O)-, or $-Si(R^2R^3)-$.

X represents an oxygen atom or $NR^4$.

"ka" represents an integer of 0 to 2, and "kb", "kc", "kd", and "ke" each represent 1 or 2.

$R^2$, $R^3$, and $R^4$ are as defined above.

Specific examples of the linear, branched, or cyclic (ka+2)-valent hydrocarbon group having 1 to 19 carbon atoms of Az" include the following.

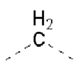 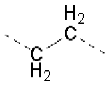 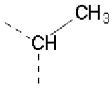 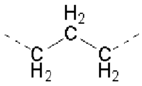 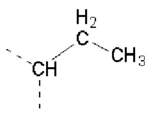 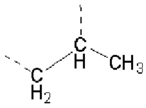 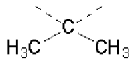

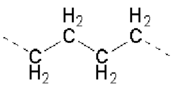 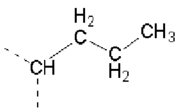 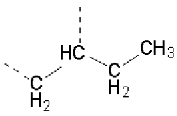 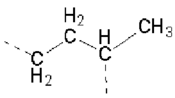 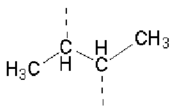

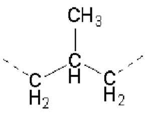 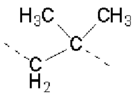 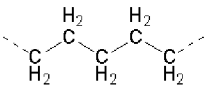 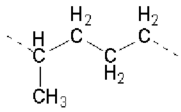 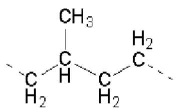

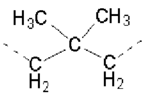 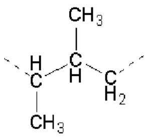 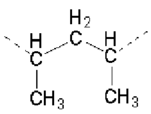 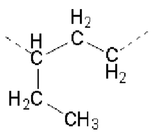 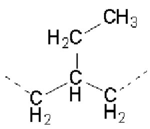 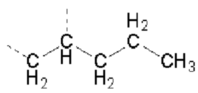

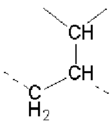 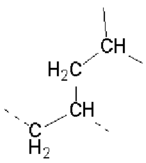 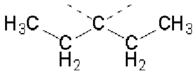 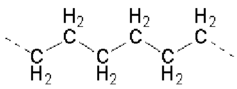 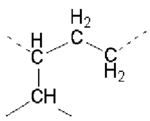

(wherein a dashed line represents a bonding arm.)

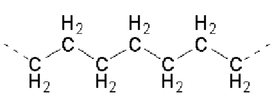 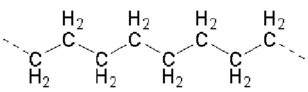 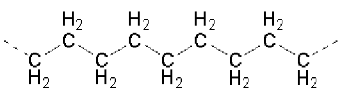

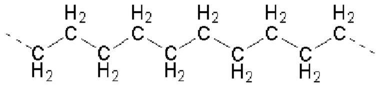
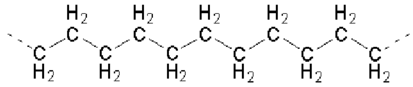
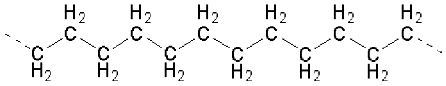
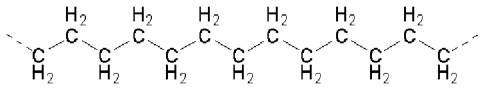
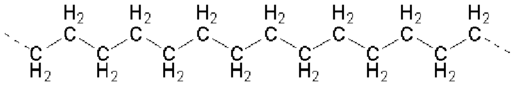
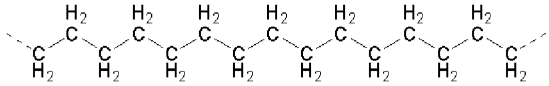
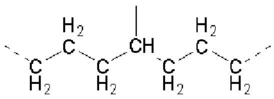
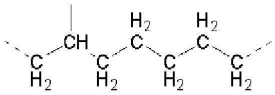
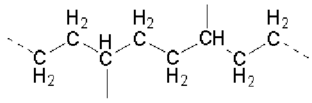
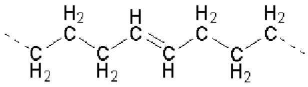
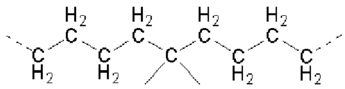
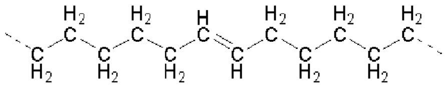
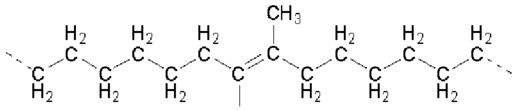

(wherein a dashed line represents a bonding arm.)

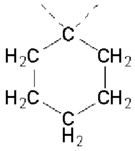
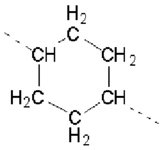
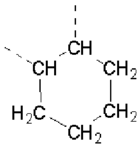
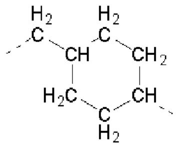
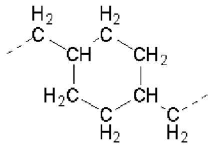
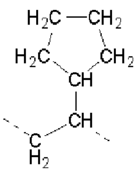
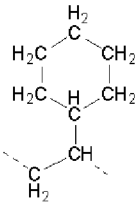
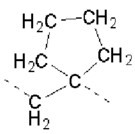
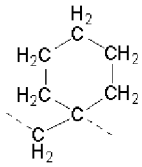
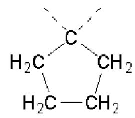
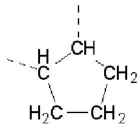
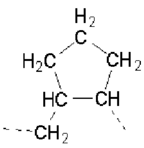
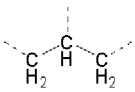
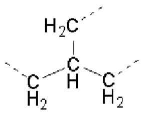
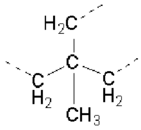
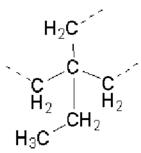
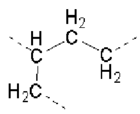
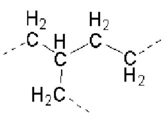
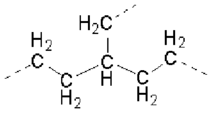
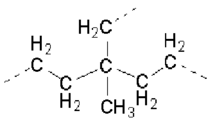
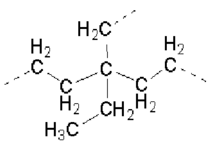
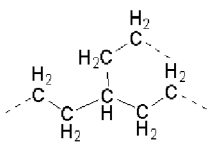

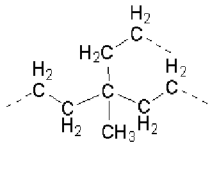 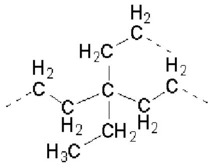 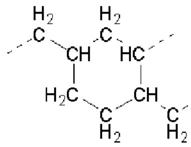 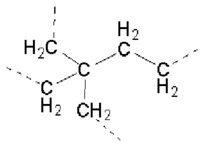 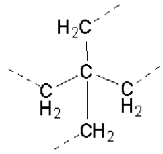

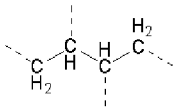 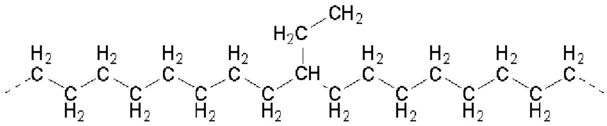

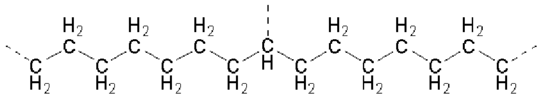 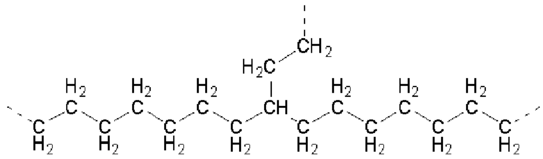

(wherein a dashed line represents a bonding arm.)

Specific examples of the alcohol or amine represented by the above general formula (1C) include the following, but are not limited thereto.

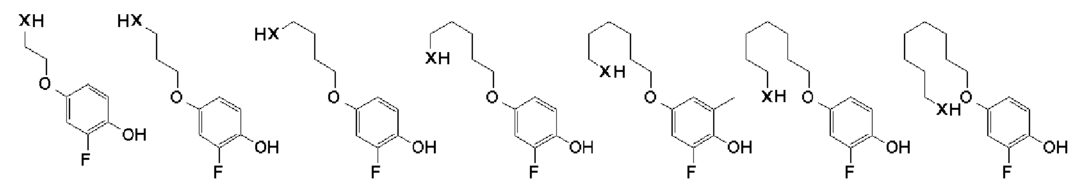

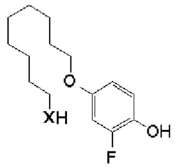 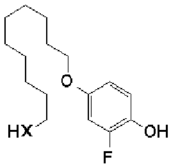 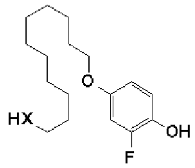 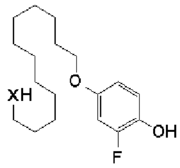 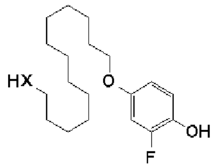

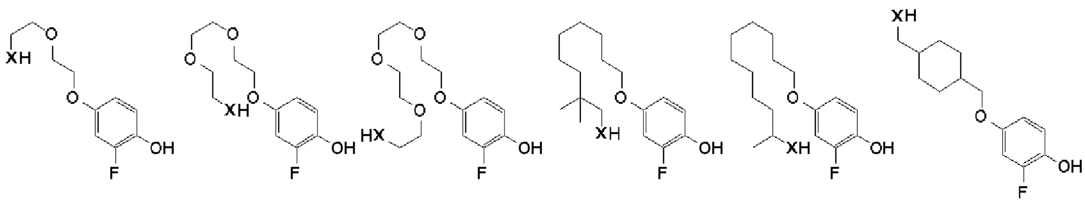

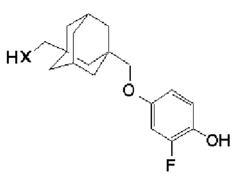 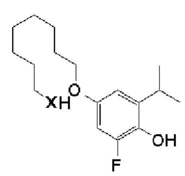 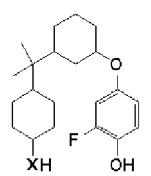 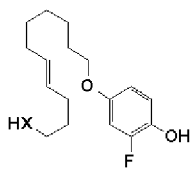

(wherein X represents an oxygen atom or $NR^4$, and $R^4$ is as defined above.)

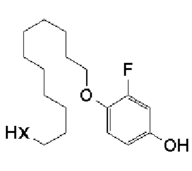
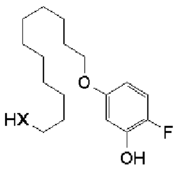
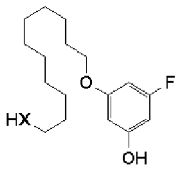
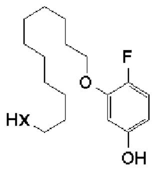
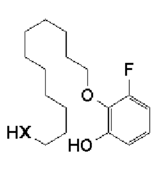
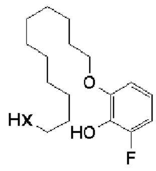

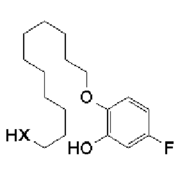
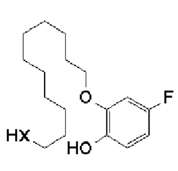
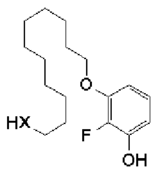
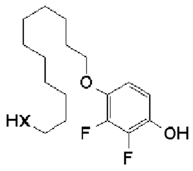
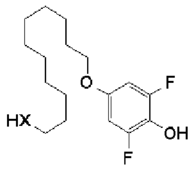
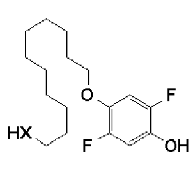

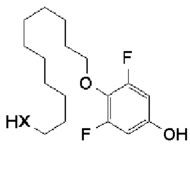
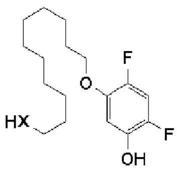
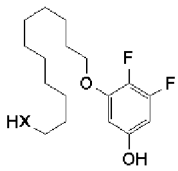
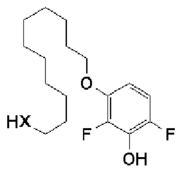
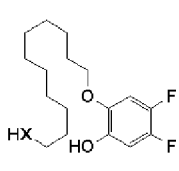
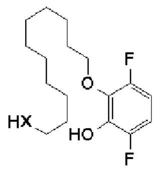

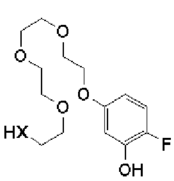
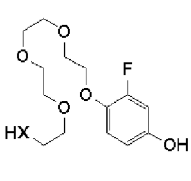
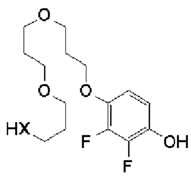
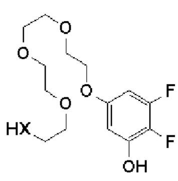
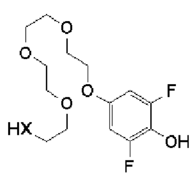

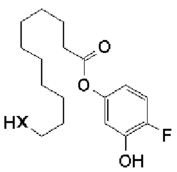
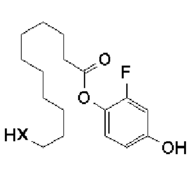
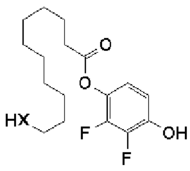
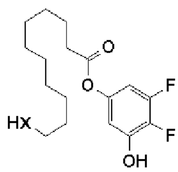
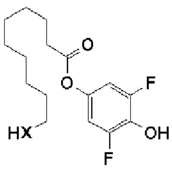
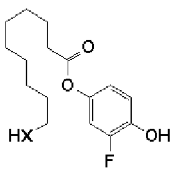

(wherein X represents an oxygen atom or $NR^4$, and $R^4$ is as defined above.)

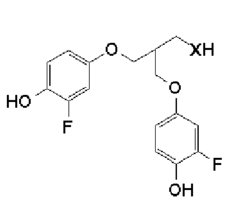
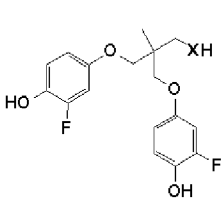
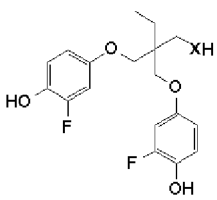
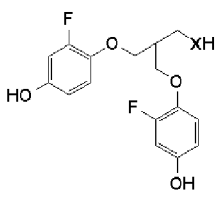
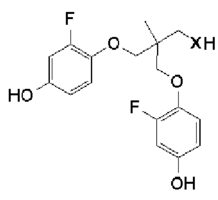

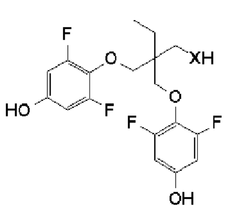
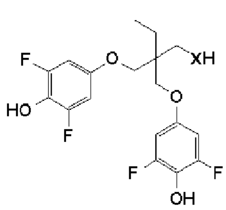
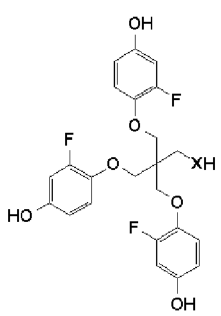
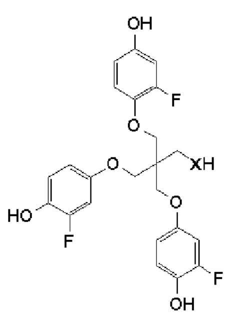

(wherein X represents an oxygen atom or $NR^4$, and $R^4$ is as defined above.)

The capping agent containing the phenolic hydroxyl group is preferably used in an amount of 1 to 20 mass%,

more preferably 1 to 5 mass%, relative to the total amount of the urethane constituent components.

The reaction temperature at the time of polyurethane synthesis is appropriately changed according to the kind of the reaction substrate. In general, the temperature is preferably 30°C to 200°C, more preferably 40°C to 120°C.

The polyurethane has a weight-average molecular weight of preferably 10,000 to 500,000, more preferably 15,000 to 200,000, further preferably 20,000 to 150,000. The weight-average molecular weight (Mw) is measured on polystyrene basis by gel permeation chromatography (GPC).

In the present embodiments, the polyurethane according to the present embodiments may further contain, as an additive, an antioxidant, defoamer, ultraviolet absorber, and the like, if necessary.

<Conductive Paste Composition>

The conductive paste composition of the present invention contains (A) a conductive filler, (B) a polyurethane containing the phenolic hydroxyl group of the present invention, and (C) a solvent, and preferably further contains (D) a phenol compound. Hereinafter, each component will be described in more details.

(A) Conductive Filler

Examples of the component (A) for enhancing electric conductivity include: metal particles or alloy particles, such as powders of gold, silver, platinum, copper, tin, iron, magnesium, titanium, nickel, palladium, aluminum, tungsten, molybdenum, ruthenium, chromium, indium, or solder, and silver-plated powders thereof; powders of carbon, carbon black, carbon nanotube, silver chloride, zinc oxide, titanium oxide, and indium tin oxide; and the like.

From the viewpoint of electric conductivity, gold, silver, or platinum is preferable. From the viewpoint of price, silver, copper, tin, iron, titanium, nickel, aluminum, tungsten, molybdenum, ruthenium, chromium, or stainless steel is preferable. Comprehensively, silver (silver powder) is most preferable.

The conductive filler particles may have any shape, such as spherical, granular, angular, dendritic, flaky, needle-like, or irregular shape. Several types of fillers may be used in combination.

The average particle size of the conductive filler is not particularly limited, but is preferably 5 nm to 10 μm.

The method for measuring the average particle size is not particularly limited. For example, a laser diffraction-type particle size distribution measurement apparatus can be used for the measurement.

The conductive filler is added in an amount within a range preferably exceeding 70 parts by mass, more preferably 80 parts by mass or more and 90 parts by mass or less, relative to 100 parts by mass of a total of the (A) conductive filler and (B) polyurethane containing the phenolic hydroxyl group.

(B) Polyurethane Containing Phenolic Hydroxyl Group

Examples of the polyurethane containing the phenolic hydroxyl group include those described above.

The polyurethane containing the phenolic hydroxyl group may be used individually, or a plurality of kinds thereof may be mixed for use. Further, a part of the polyurethane containing the phenolic hydroxyl group may be substituted with a polyurethane containing no phenolic hydroxyl group.

The polyurethane containing no phenolic hydroxyl group is contained in an amount of preferably 0 to 30 mass% relative to a total amount of the polyurethane containing the phenolic hydroxyl group and the polyurethane containing no phenolic hydroxyl group.

Preferably, the polyurethane containing the phenolic hydroxyl group further contains the aforementioned weakly acidic functional group.

In the conductive paste composition of the present invention, when the conductive filler is metal or alloy particles, particularly a silver powder, it is presumed that a silver salt is formed from an oxide film of the silver powder and the phenolic hydroxyl group or the weakly acidic functional group in the polyurethane, and the silver salt is reduced by heat to form silver nanoparticles. In this event, it is presumed that a stable silver salt is formed in case of the functional group exhibiting strong acidity so that the reduction by heat hardly progresses, while the silver salt and thus silver nanoparticles are hardly formed in case of weak acidity. Therefore, it is presumed that using a polyurethane containing a functional group with appropriate acidity enables silver salt formation and silver salt reduction even by baking at relatively low temperature, thereby easily generating silver nanoparticles.

(C) Solvent

The component (C) is a solvent. By containing the component (C), the conductive paste composition has suitable viscosity and enhanced workability.

Specific examples of the solvent as the component (C) include: aromatic hydrocarbon-based solvents, such

as toluene, xylene, cumene, 1,2,3-trimethylbenzene, 1,2,4-trimethylbenzene, 1,3,5-trimethylbenzene, styrene, α-methylstyrene, butylbenzene, sec-butylbenzene, isobutylbenzene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tert-amylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, and 1,3,5-triethylbenzene; aliphatic hydrocarbon-based solvents, such as n-heptane, isoheptane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, methylcyclohexane, 1-methyl-1-cyclohexene, 3-methyl-1-cyclohexene, methylenecyclohexane, 4-methyl-1-cyclohexene, 2-methyl-1-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-trimethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonyne, 2-nonyne, 3-nonyne, 4-nonyne, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, α-phellandrene, pinene, terpinene, tetrahydrodicyclopentadiene, 5,6-dihydrodicyclopentadiene, dicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.$0^{2,7}$]undeca-4-ene, n-dodecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, and isoparaffin; ketone solvents, such as cyclohexanone, cyclopentanone, 2-octanone, 2-nonanone, 2-heptanone, 3-heptanone, 4-heptanone, 2-hexanone, 3-hexanone, diisobutyl ketone, methylcyclohexanone, methyl n-pentyl ketone, methyl isobutyl ketone, and isophorone; alcohol solvents, such as 3-methoxybutanol, 3-methyl-3-methoxybutanol, 1-methoxy-2-propanol, and 1-ethoxy-2-propanol; ether solvents, such as propylene glycol monomethyl ether, ethylene glycol monomethyl ether, propylene glycol monoethyl ether, ethylene glycol monoethyl ether, propylene glycol dimethyl ether, diethylene glycol dimethyl ether, diethylene glycol diethyl ether, diethylene glycol monomethyl ether, diethylene glycol monoethyl ether, diethylene glycol monopropyl ether, diethylene glycol monobutyl ether, diisopropyl ether, diisobutyl ether, diisopentyl ether, di-n-pentyl ether, methyl cyclopentyl ether, methyl cyclohexyl ether, di-n-butyl ether, di-sec-butyl ether, di-sec-pentyl ether, di-tert-amyl ether, di-n-hexyl ether, and anisole; ester solvents, such as ethylene glycol monobutyl ether acetate, propylene glycol monomethyl ether acetate, propylene glycol monoethyl ether acetate, propylene glycol monopropyl ether acetate, propylene glycol monobutyl ether acetate, propylene glycol diacetate, diethylene glycol monomethyl ether acetate, diethylene glycol monoethyl ether acetate, diethylene glycol monobutyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactone solvents such as γ-butyrolactone; terpene solvents, such as α-terpineol, α-pinene, dihydroterpineol, and dihydroterpinyl acetate; and the like. These solvents may be used individually or as a mixture of two or more kinds thereof.

Diethylene glycol monobutyl ether, diethylene glycol monoethyl ether acetate, or diethylene glycol monobutyl ether acetate is particularly preferable because the solvents hardly evaporate during printing and impart suitable viscosity for printing.

The solvent (organic solvent) is preferably added in an amount within a range of 100 to 1,000 parts by mass relative to 100 parts by mass of (B) the polyurethane.

(D) Phenol Compound

Further, it is preferable to contain a phenol compound containing a structure represented by the following general formula (2A) as the phenol compound,

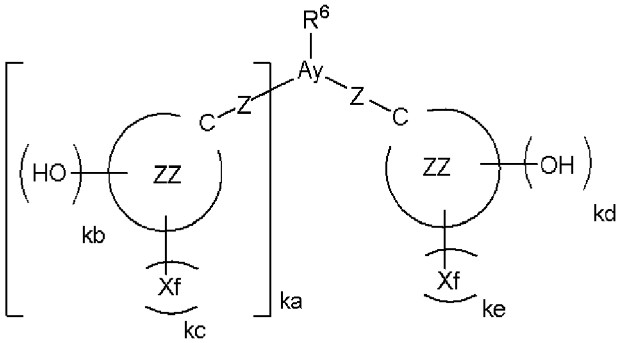 (2A)

wherein $R^6$ represents a hydrogen atom, a halogen atom, a cyano group, or a hydroxyl group; Ay represents a linear, branched, or cyclic (ka+2)-valent hydrocarbon group or a fluorinated hydrocarbon group having 1 to 20 carbon atoms, and $-CH_2-$ constituting the (ka+2)-valent hydrocarbon group is optionally substituted with -O-, - C(=O)-, or $-Si(R^2R^3)-$; "ka" represents an integer of 0 to 2; "kb" and "kd" each represent 1 or 2; "kc" and "ke" represent an integer of 0 to 2; and Z, Xf, ZZ, $R^2$, and $R^3$ are as defined above.

$R^6$ represents a hydrogen atom, a halogen atom, a cyano group, or a hydroxyl group.

Ay represents a linear, branched, or cyclic (ka+2)-valent hydrocarbon group or a fluorinated hydrocarbon group having 1 to 20 carbon atoms, and $-CH_2-$constituting the (ka+2)-valent hydrocarbon group is optionally substituted with -O-, -C(=O)-, or $-Si(R^2R^3)-$. Specific examples of the linear, branched, or cyclic (ka+2)-valent hydrocarbon group having 1 to 20 carbon atoms as Ay include the following.

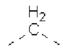 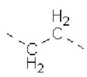 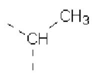 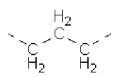 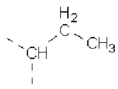 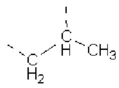 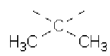

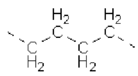 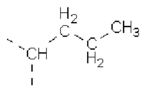 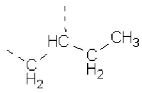 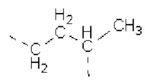 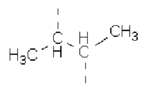

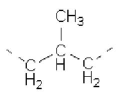 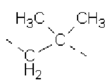 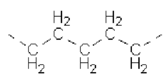 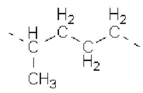 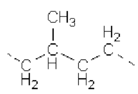

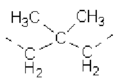 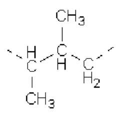 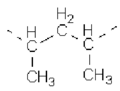 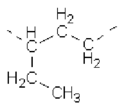 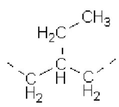 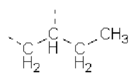

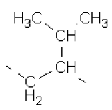 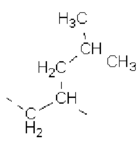 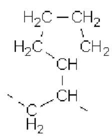 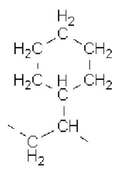 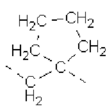 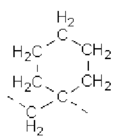

(wherein a dashed line represents a bonding arm.)

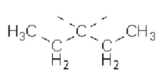 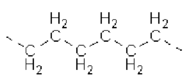 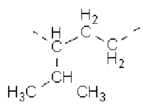 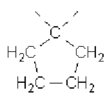 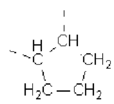

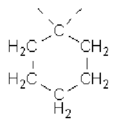 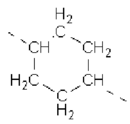 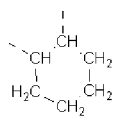 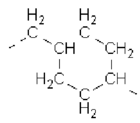 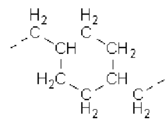

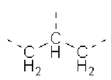 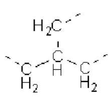 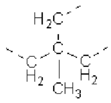 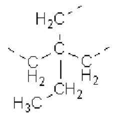 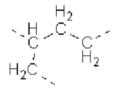 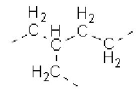

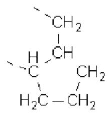 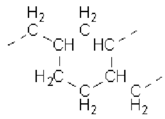 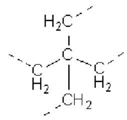 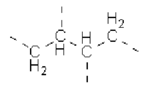 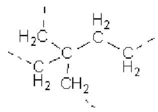

(wherein a dashed line represents a bonding arm.)

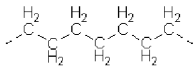 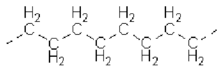 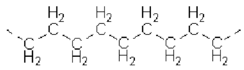 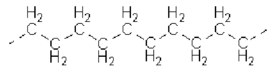

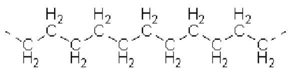 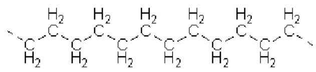 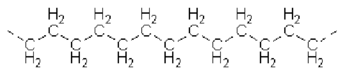

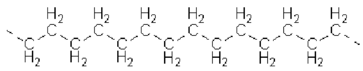 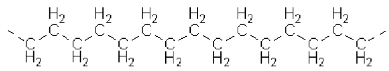

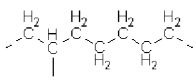 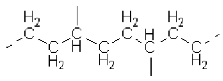 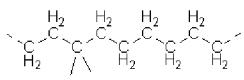 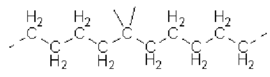

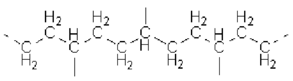 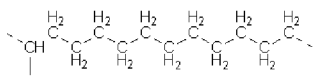 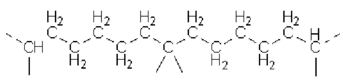

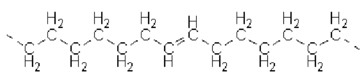 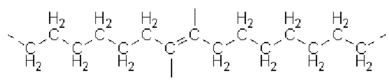

(wherein a dashed line represents a bonding arm.)

Specific examples of the linear, branched, or cyclic (ka+2)-valent fluorinated hydrocarbon group having 1 to 20 carbon atoms as Ay include the one in which a part or all of the hydrogen atoms in the hydrocarbon group are substituted with a fluorine atom.

"ka" represents an integer of 0 to 2, "kb" and "kd" each represent 1 or 2, and "kc" and "ke" each represent an integer of 0 to 2.

Z, Xf, ZZ, $R^2$, and $R^3$ are as defined above.

The phenol compound represented by the above general formula (2A) is preferably a phenol compound represented by the following general formula (2B),

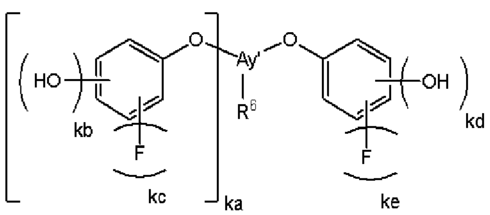 (2B)

wherein Ay' represents a linear, branched, or cyclic (ka+2)-valent hydrocarbon group or a fluorinated hydrocarbon group having 1 to 19 carbon atoms, and $-CH_2$-constituting the (ka+2)-valent hydrocarbon group is optionally substituted with -O-, -C(=O)-, or $-Si(R^2R^3)-$; "ka" represents an integer of 0 to 2; "kb", "kc", "kd", and "ke" each represent 1 or 2; and $R^2$, $R^3$, and $R^6$ are as defined above.

Ay' represents a linear, branched, or cyclic (ka+2)-valent hydrocarbon group or a fluorinated hydrocarbon group having 1 to 19 carbon atoms, and $-CH_2$-constituting the (ka+2)-valent hydrocarbon group is optionally substituted with -O-, -C(=O)-, or $-Si(R^2R^3)-$.

"ka" represents an integer of 0 to 2, and "kb", "kc", "kd", and "ke" each represent 1 or 2.

$R^2$, $R^3$, and $R^6$ are as defined above.

Specific examples of the compounds represented by the above general formulae (2A) and (2B) include the following.

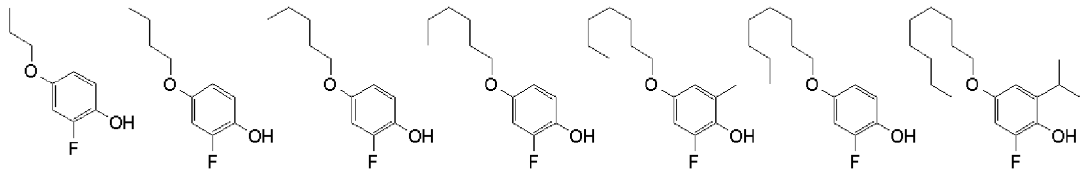

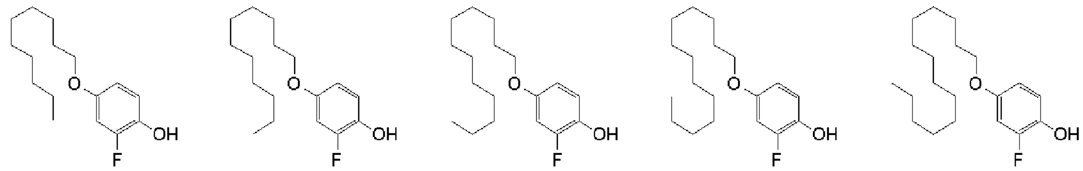

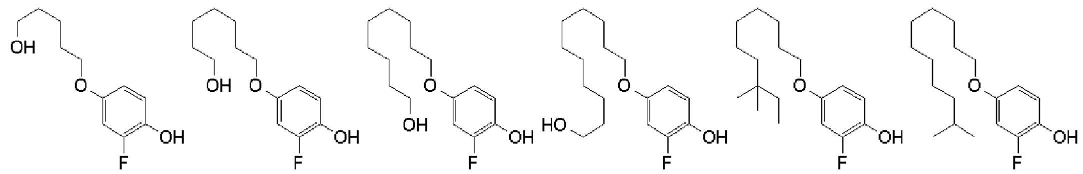

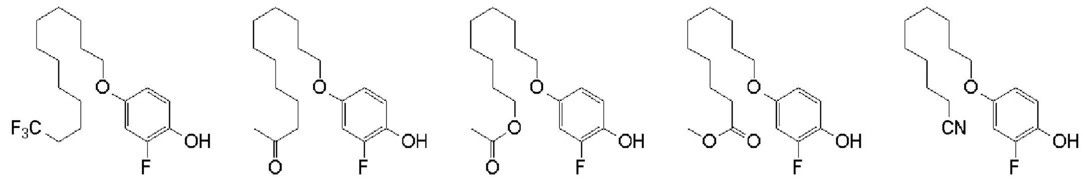

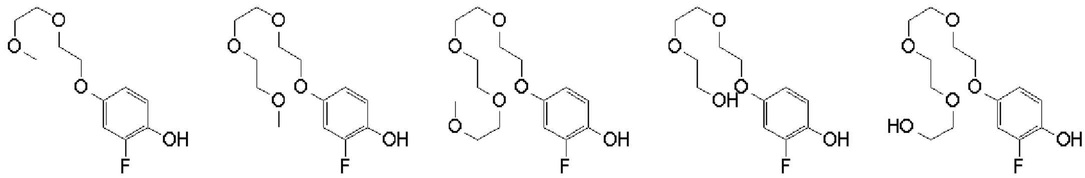

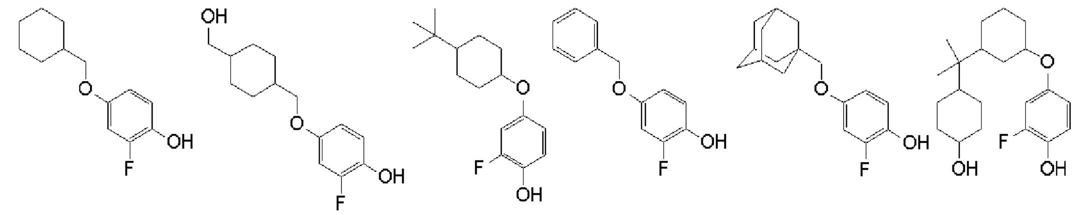

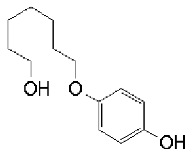 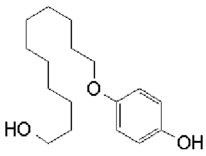 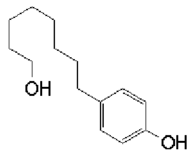 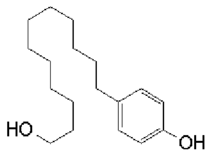 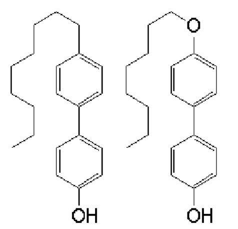

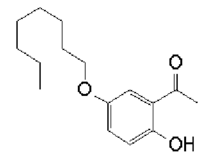 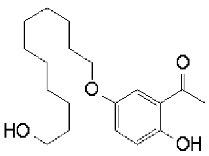 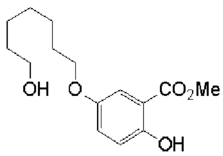 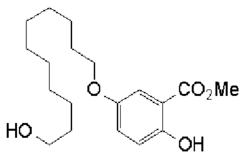 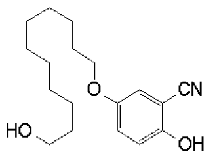

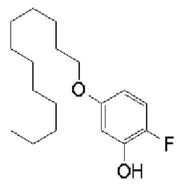 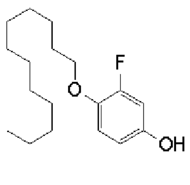 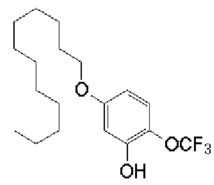 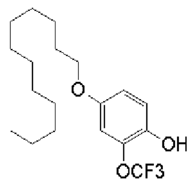 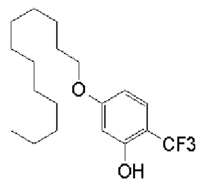

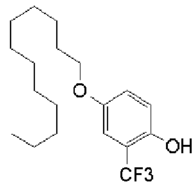 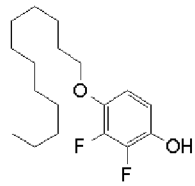 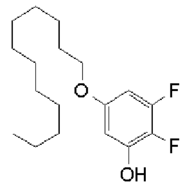 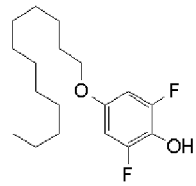 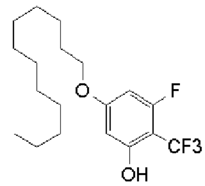

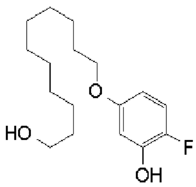 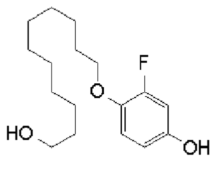 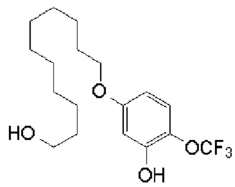 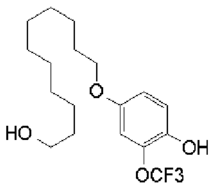 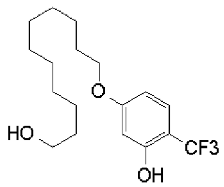

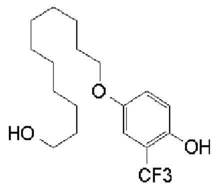 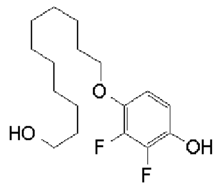 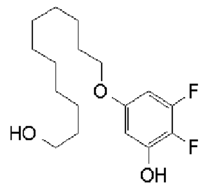 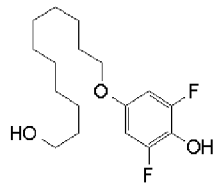 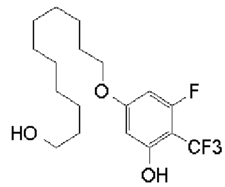

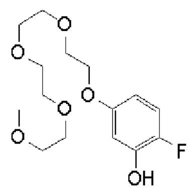 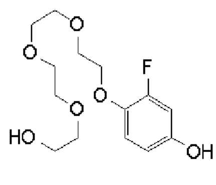 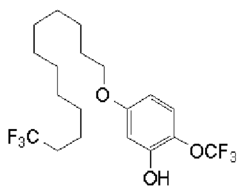 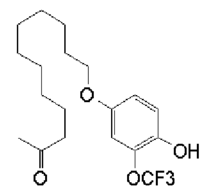 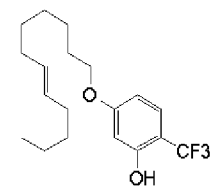

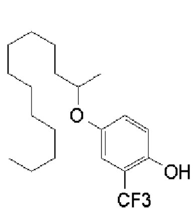 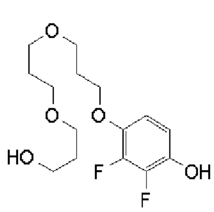 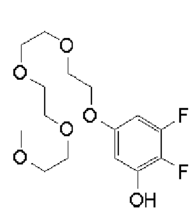 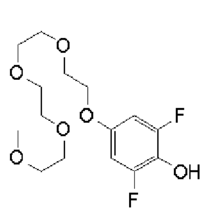 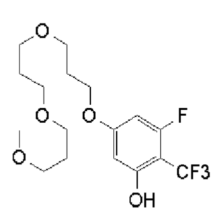

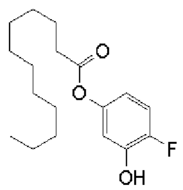 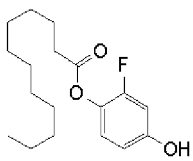 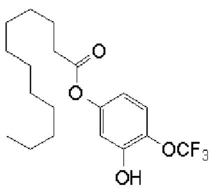 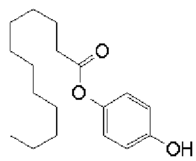 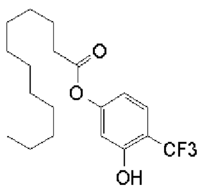

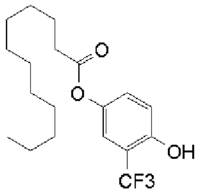 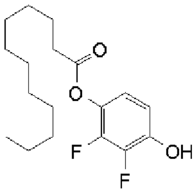 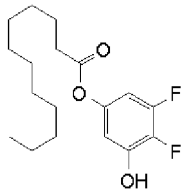 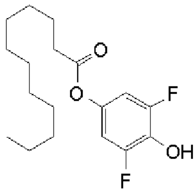 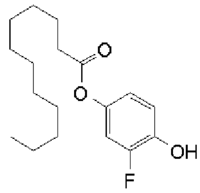

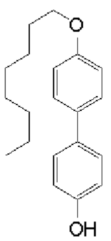 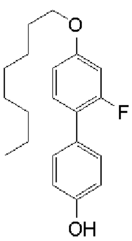 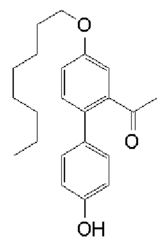 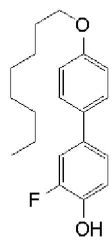 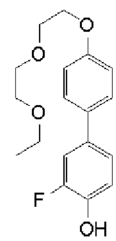 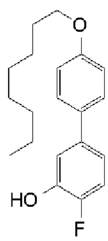 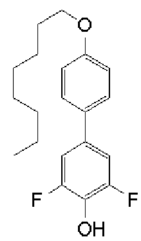

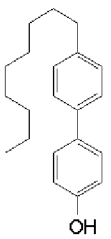 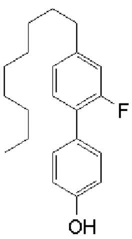 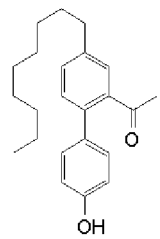 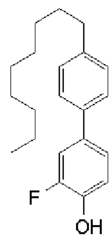 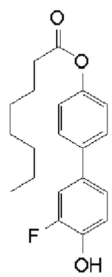 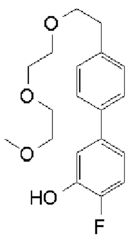 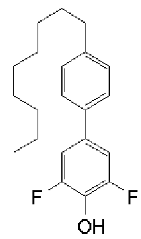

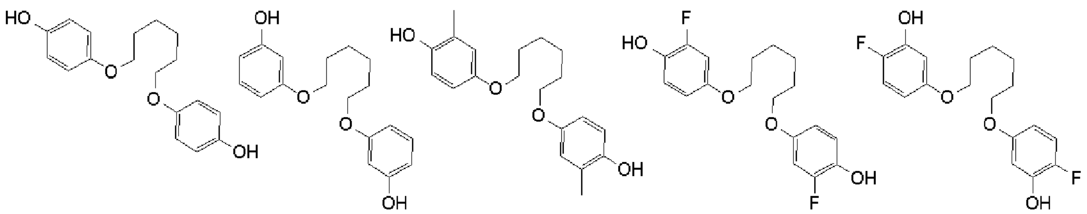

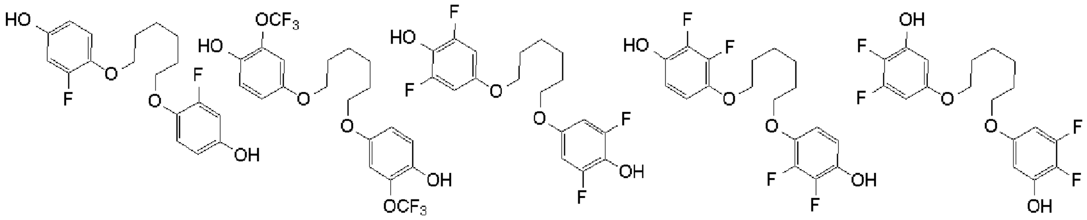

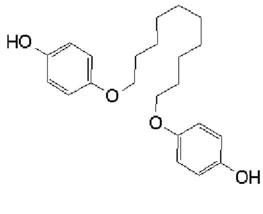
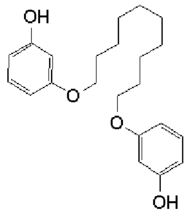
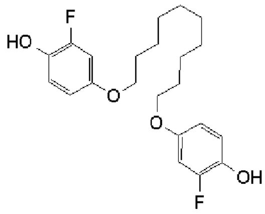
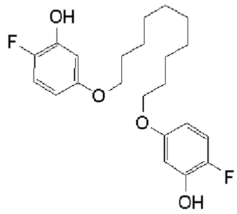

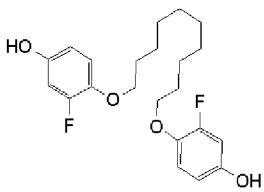
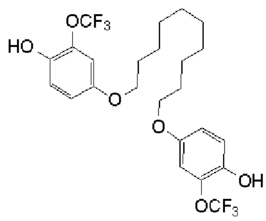
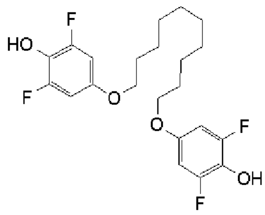
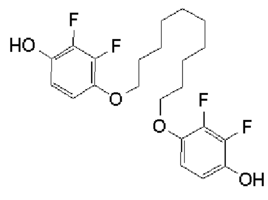

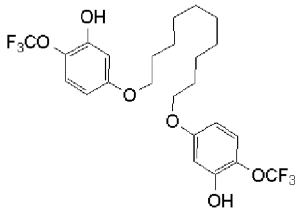
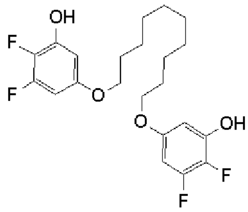
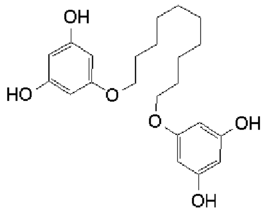
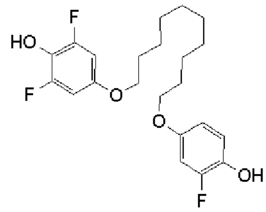

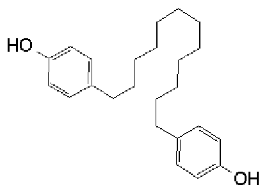
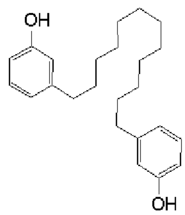
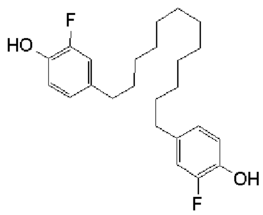
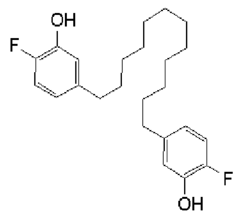

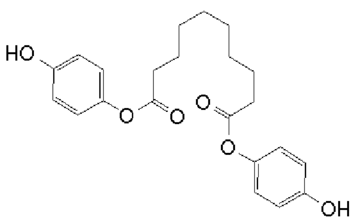
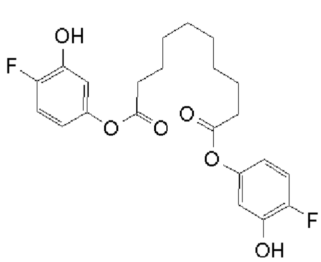
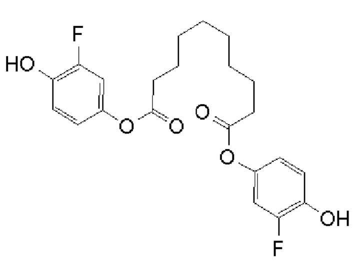

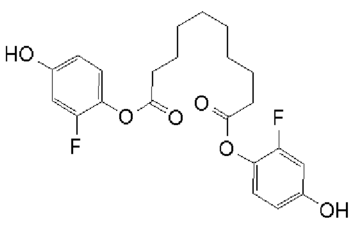
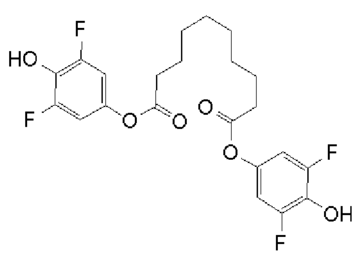
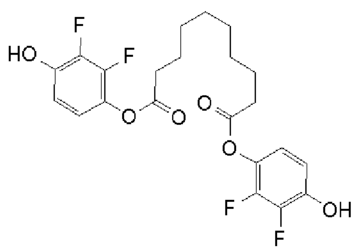

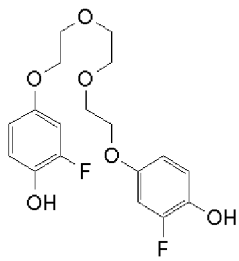
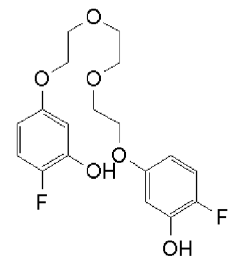
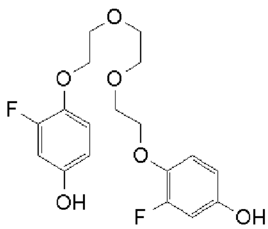
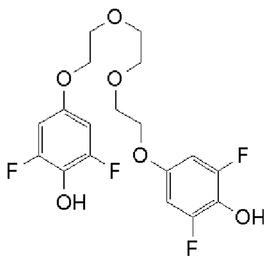

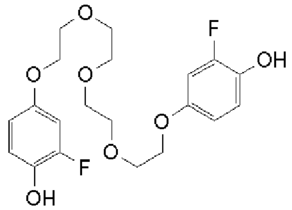
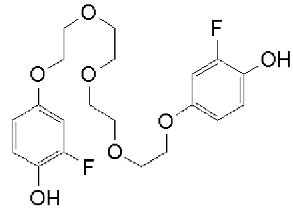
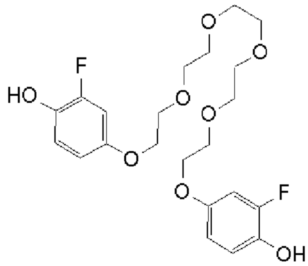

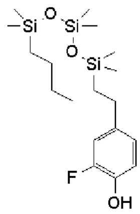
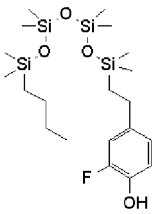
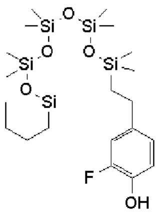
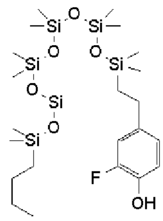
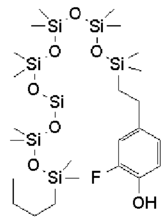
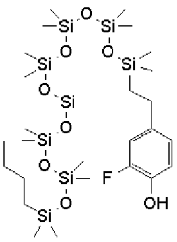

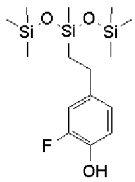
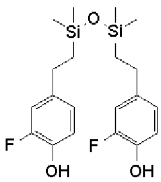
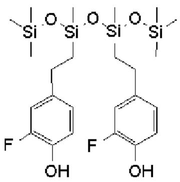
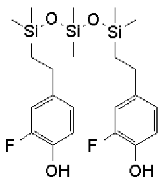
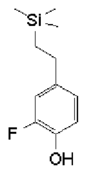
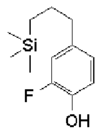
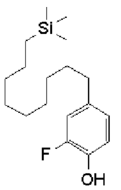

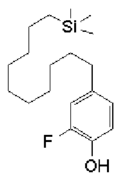
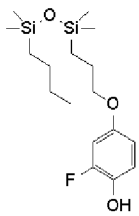
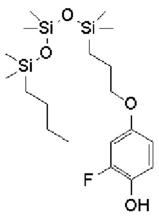
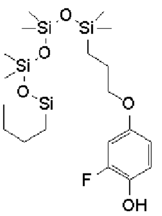
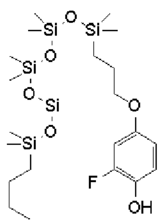
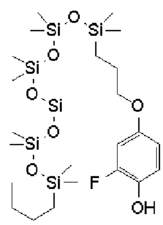
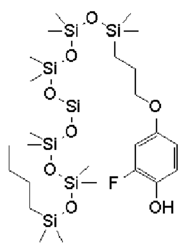

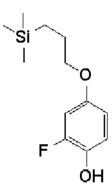
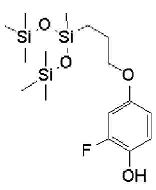
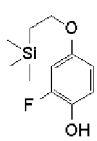
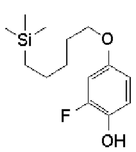
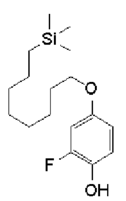
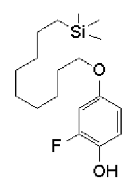
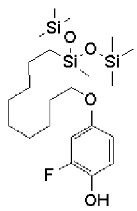

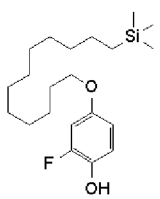
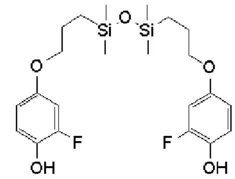
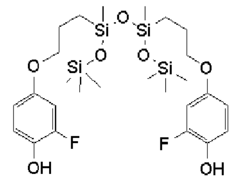

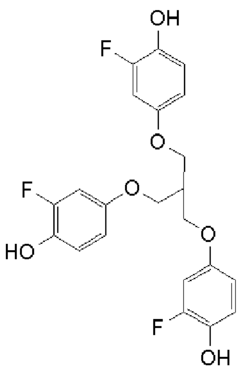
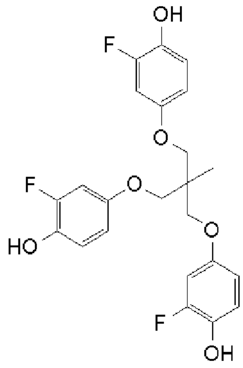
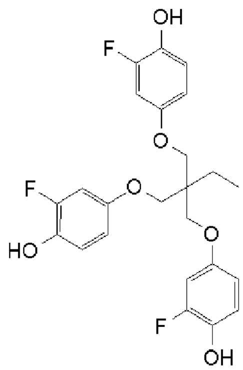
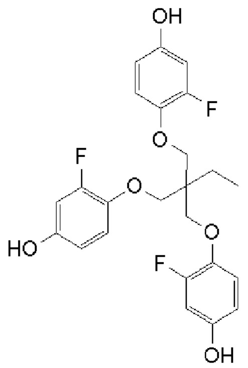

The method for synthesizing the phenol compounds represented by the above general formulae (2A) and (2B) is not particularly limited. The phenol compounds can be synthesized by an appropriate method depending on the structure thereof. For example, when "kb" and "kc" in the formula (2B) represent 1, the synthesis can be performed by the steps (i) to (v) shown in the following reaction formula,

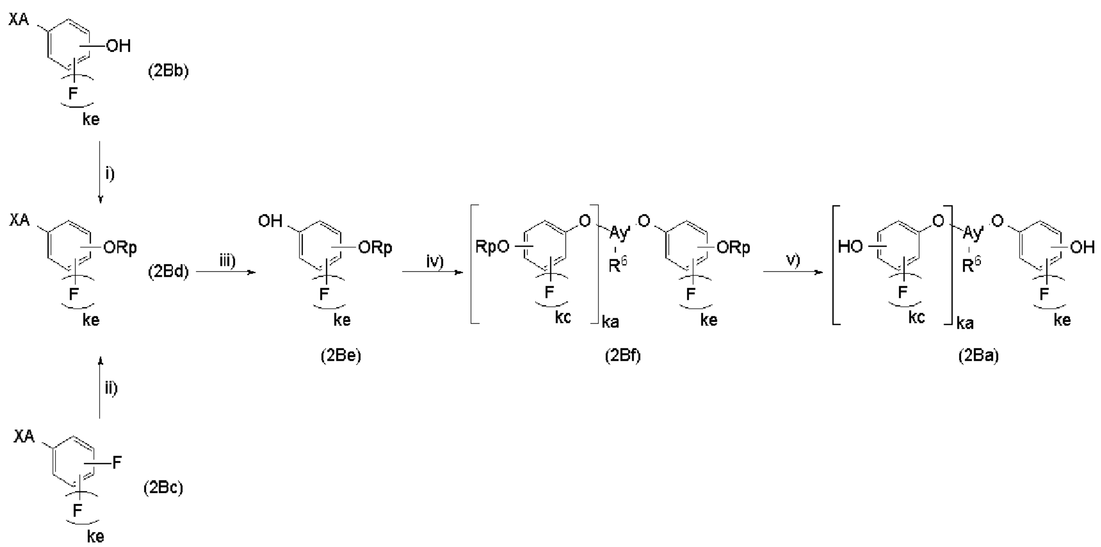


wherein $R^1$, Ay', "ka", "kc" and "ke" are as defined above; Rp represents an acid-labile group; and XA represents a halogen atom.

The step (i) is a step of protecting a halogenated phenol compound (2Bb) to give an intermediate halogenated aryl compound (2Bd).

The reaction of the step (i) easily proceeds under known conditions. For example, when Rp is a tertiary alkyl group such as a t-butyl group, t-amyl group, methylcyclopentyl group, ethylcyclopentyl group, methylcyclohexyl group, ethylcyclohexyl group, methyladamantyl group, and ethyladamantyl group, the reaction is preferably carried out at a reaction temperature of -20°C to 50°C without a solvent or in a solvent such as toluene and hexane using a halogenated phenol compound (2Bb) and olefins corresponding to Rp such as isobutene and isoamylene in the presence of an acid catalyst. Examples of the acid catalyst to be used include: inorganic acids such as hydrochloric acid, sulfuric acid, nitric acid, and perchloric acid; and organic acids such as methanesulfonic acid, trifluoromethanesulfonic acid, p-toluenesulfonic acid, and benzenesulfonic acid.

The step (ii) is a step of subjecting a fluorobenzene compound (2Bc) to a nucleophilic substitution reaction to

give the intermediate halogenated aryl compound (2Bd).

The reaction of the step (ii) easily proceeds under known conditions. When Rp is a tertiary alkyl group such as, for example, a t-butyl group, t-amyl group, methylcyclopentyl group, ethylcyclopentyl group, methylcyclohexyl group, ethylcyclohexyl group, and methyladamantyl group, the reaction is preferably carried out at a reaction temperature of 10°C to 80°C in a solvent such as tetrahydrofuran and N-methyl-2-pyrrolidone using the fluorobenzene compound (2Bc) and alcohol corresponding to Rp such as t-butyl alcohol and t-amyl alcohol, or corresponding alkoxides such as potassium t-butoxide in the presence of a base. Examples of the base to be used include: metal hydrides, such as borane, alkylborane, sodium hydride, lithium hydride, potassium hydride, and calcium hydride; alkyl metal compounds, such as trityllithium, tritylsodium, tritylpotassium, methyllithium, phenyllithium, n-butyllithium, sec-butyllithium, tert-butyllithium, and ethyl magnesium bromide; alkoxides, such as sodium methoxide, sodium ethoxide, lithium methoxide, lithium ethoxide, lithium tert-butoxide, and potassium tert-butoxide; and the like.

The step (iii) is a step of oxidizing the halogenated aryl compound (2Bd) to give an intermediate phenol compound (2Be).

The reaction easily proceeds by a known method. Examples of the method include the following reaction formula,

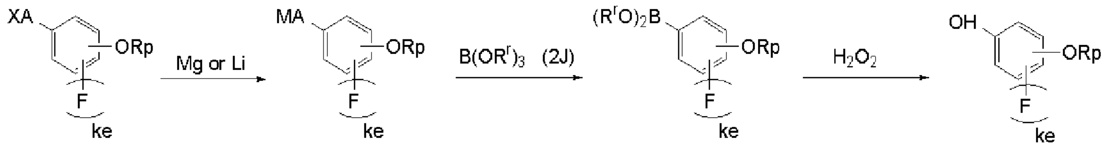


(2Bd) (2Bg) (2Bh) (2Be)

wherein Rp, "ke", and XA are as defined above; MA represents Li, MgCl, MgBr, and MgI; and $R^r$ represents a linear, branched, or cyclic monovalent hydrocarbon group having 1 to 6 carbon atoms.

First, in a solvent such as tetrahydrofuran and diethyl ether, an organometallic reagent (2Bg) is prepared from the halogenated aryl compound (2Bd) and Li or Mg. Then, the reagent is reacted with a boric acid ester compound (2J) to give an arylboronic acid derivative (2Bh). Finally, an oxidant such as hydrogen peroxide, performic acid, peracetic acid, or m-chloroperbenzoic acid is used to obtain the intermediate phenol compound (2Be). In this step, the reaction can usually proceed in one-pot without a purification step.

The step (iv) is a step of etherifying the intermediate phenol compound (2Be) to give an aryl ether compound (2Bf).

The reaction easily proceeds by a known method. Examples of the method include the following reaction formula,

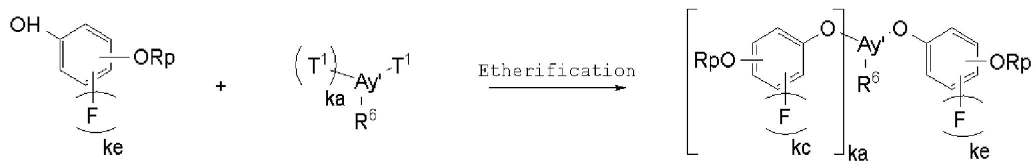


(2Be) (2Bi) (2Bf)

wherein Rp, "ka", "ke", and Ay' are as defined above; and each $T^1$ independently represents a hydroxyl group, a halogen atom, an alkane sulfonyloxy group, or an arene sulfonyloxy group.

Examples of the etherification method include an etherification method by base treatment of the intermediate phenol compound (2Be) and a leaving group-containing compound (2Bi).

Examples of the halogen atom of $T^1$ include a chloride atom, a bromine atom, and an iodine atom. Further, examples of the alkane sulfonyloxy group and arene sulfonyloxy group of $T^1$ include methane sulfonyloxy, trifluoromethanesulfonyloxy, benzenesulfonyloxy, and p-toluenesulfonyloxy group.

Specific examples of the base to be used include: alkoxides, such as sodium methoxide, sodium ethoxide, lithium methoxide, lithium ethoxide, lithium tert-butoxide, and potassium tert-butoxide; organic amines, such as pyridine, triethylamine, N,N-dimethylaniline, and 4-dimethylaminopyridine; inorganic hydroxides, such as sodium hydroxide, lithium hydroxide, potassium hydroxide, barium hydroxide, and tetra-n-butylammonium hydroxide; inorganic carbonates, such as sodium carbonate, sodium bicarbonate, lithium carbonate, and potassium carbonate; alkoxides, such as sodium methoxide, sodium ethoxide, lithium methoxide, lithium ethoxide, lithium tert-butoxide, and potassium tert-butoxide; metal hydrides, such as borane, alkylborane, sodium hydride, lithium hydride, potassium hydride, and calcium hydride; alkyl metal compounds, such as trityllithium, tritylsodium, tritylpotassium, methyllithium, phenyllithium, sec-butyllithium,

tert-butyllithium, methylmagnesium chloride, ethylmagnesium chloride, and ethylmagnesium bromide; metal amides, such as sodium amide, potassium amide, lithium diisopropylamide, potassium diisopropylamide, lithium dicyclohexylamide, potassium dicyclohexylamide, lithium 2,2,6,6-tetramethylpiperidine, lithium bistrimethylsilylamide, sodium bistrimethylsilylamide, potassium bistrimethylsilylamide, lithium isopropylcyclohexylamide, and bromomagnesium diisopropylamide; and the like. The amount of the base to be used is preferably 0.9 to 10 moles, particularly 1.0 to 5.0 moles per mole of the intermediate phenol compound (2Be).

Examples of the solvent include: water; ethers, such as tetrahydrofuran, diethylether, di-n-butylether, and 1,4-dioxane; hydrocarbons, such as n-hexane, n-heptane, benzene, toluene, xylene, and cumene; alcohols, such as methanol, ethanol, isopropyl alcohol, and tert-butyl alcohol; aprotic polar solvents, such as dimethyl sulfoxide (DMSO) and N,N-dimethylformamide (DMF); and chlorine-based organic solvents, such as methylene chloride, chloroform, and carbon tetrachloride. One kind or a combination thereof can be selected and used depending on the reaction conditions. The bases listed above themselves may be used as a solvent.

The reaction temperature and time vary depending on the reagents and conditions. For example, when $T^1$ is a bromine atom and potassium carbonate is used as a base to perform the reaction, the reaction temperature is from room temperature to 120°C, preferably 30°C to 90°C, from the viewpoint of rapid completion of the reaction. The reaction time is usually about 1 to 60 hours, though it is preferable, in terms of yields, to monitor the reaction by gas chromatography (GC) or silica gel thin-layer chromatography (TLC) to complete the reaction. The aryl ether compound (2Bf) can be obtained from the reaction mixture by typical aqueous post-treatment (aqueous work-up), and if necessary, it can be purified according to a standard method such as distillation and chromatography.

The step (v) is a step of subjecting the aryl ether compound (2Bf) to a deprotection reaction to give the phenol compound of the present invention.

Examples of the solvent include: hydrocarbons, such as toluene, xylene, hexane, and heptane; chlorine-based solvents, such as methylene chloride, chloroform, and dichloroethane; ethers, such as diethylether, tetrahydrofuran, and dibutylether; ketones such as acetone and 2-butanone; esters such as ethyl acetate and butyl acetate; nitriles such as acetonitrile; alcohols such as methanol and ethanol; aprotic polar solvents, such as N,N-dimethylformamide, N,N-dimethylacetamide, and dimethyl sulfoxide; and water. One kind or a combination of two or more kinds thereof can be selected and used. Alternatively, the reaction can be performed without a solvent.

Examples of the acid for use include: inorganic acids, such as hydrochloric acid, sulfuric acid, nitric acid, and perchloric acid; organic acids, such as methanesulfonic acid, trifluoromethanesulfonic acid, p-toluenesulfonic acid, and benzenesulfonic acid; and Lewis acids, such as boron trifluoride, trimethylsilyl triflate, aluminum chloride, magnesium chloride, iron chloride, zinc chloride, and titanium chloride; and the like. Further, acid is used in an amount of preferably 0.001 to 5 moles, particularly 0.01 to 0.5 moles, per mole of the aryl ether compound (2Bf). When the amount to be used is 0.001 moles or more, the reaction rate does not decrease and thus, there is no cost disadvantage due to increased reaction time. When the amount to be used is 5 moles or less, no side reactions due to strong acidity occur and thus, the yields does not decrease. In addition, to reduce acidity, a base, for example, amines such as ammonia, triethylamine, pyridine, lutidine, collidine, and N,N-dimethylaniline, may be added.

In the aforementioned deprotection reaction, an appropriate reaction temperature can be selected depending on the reaction conditions. Since the reaction may not proceed under low temperature conditions, the reaction temperature is preferably 40°C to 120°C. Moreover, the reaction time is preferably determined by monitoring the progress of the reaction by thin-layer chromatography, gas chromatography or the like to improve the yields. The reaction time is usually around 2 hours to 1 day. The reaction can proceed by diluting the aryl ether compound (2Bf) with a solvent, followed by adding acid and stirring with heat. After completion of the reaction, the phenol compound of the present invention can be obtained by a typical aqueous post-treatment (aqueous work-up), and if necessary, it can be purified according to a standard method such as distillation, recrystallization, chromatography, or the like.

When blending the aforementioned phenol compound with the conductive paste composition of the present invention, it is contained preferably in a range exceeding 0.5 parts by mass, more preferably 1 parts by mass or more and 10 parts by mass or less relative to 100 parts by mass of a total of the (A) conductive filler, (B) polyurethane containing the phenolic hydroxyl group, and (D) phenol compound.

The aforementioned phenol compound has the same effect as the phenolic hydroxyl group and the weakly acidic functional group in the polyurethane of the present invention. In other words, when the conductive filler is metal or alloy particles, especially a silver powder, it is presumed that a silver salt is formed by the phenolic hydroxyl group of the phenol compound and an oxide film of the silver powder, and the silver salt is reduced by heat to generate silver nanoparticles. In particular, the phenol compound with an electron-withdrawing group on an aromatic ring has improved acidity and facilitates formation of the silver salt as compared to a non-functional phenol. Therefore, it is expected that silver nanoparticles are formed even at a low temperature. In addition, when the phenol compound is the phenol compound represented by the above general formula (2B), it has a fluorine atom on the aromatic ring, and it is expected that a nucleophilic substitution reaction proceeds by the silver salt, and a silver fluoride byproduct of the reaction is reduced by heat or the like, thereby forming silver nanoparticles.

Conductive Wire

Moreover, the present invention provides a conductive wire formed on a substrate, the conductive wire containing a baked product of the conductive paste composition. A stretchable conductive wire is provided when the substrate has stretchability.

Hereinafter, the conductive wire according to the present invention will be described, but the present invention is not limited thereto.

Examples of the substrate on which the conductive wire is formed include polyurethane, polyester, silicone, nitrile rubber, butadiene rubber, polyethylene, polypropylene, polyolefin, PTFE, PFA, and the like. The substrate is preferably stretchable, more preferably a stretchable sheet or film, further preferably a stretchable polyurethane substrate, particularly preferably a thermoplastic polyurethane substrate. The surface of the sheet may be flat or uneven. An uneven surface allows formation of a stretchable wire having a bellows-like structure in a direction orthogonal to the substrate, and makes it possible to suppress change in electric conductivity at the time of elongation and shrinkage. Alternatively, it is also possible to use a nonwoven fabric or a fabric made of stretchable fibers.

The stretchable substrate preferably has a stretchability of 1000% at maximum. Human skins are known to stretch, according to the movements, by 10% on bones of chest and so forth, by 20% on abdominal portions and so forth, and by 50% on joints. The stretchability required for conductive wires vary depending on the attachment location on skin.

Hereinafter, description will be given of the case employing a substrate having stretchability (stretchable substrate). The same applies to a substrate having no stretchability, and the present invention is not limited to the following.

A conductive wire made from the conductive paste composition of the present invention is formed on a stretchable substrate. Suitable examples of the method for applying the conductive wire onto the stretchable substrate includes, but not particularly limited to, dip coating, spray coating, spin coating, roll coating, flow coating, doctor coating, screen printing, flexographic printing, gravure printing, inkjet printing, and the like. Particularly, forming a wire pattern by printing can enhance the productivity and enables free design, including wiring width.

The conductive wire to be used preferably has a film thickness in a range of 10 nm to 1000 μm, more preferably 5 um to 50 μm.

The conductive paste composition is applied onto the stretchable substrate by printing, and then baked. In other words, a wire pattern is formed on the substrate by printing the conductive paste composition. The baking temperature for forming the wire pattern is in a range of 60°C to 160°C, preferably 120°C to 150°C, and the baking time is 1 second to 10 hours, preferably 10 minutes to 5 hours. The baking can be performed on a hot plate or in an oven, or can be performed by flash annealing in a shorter period of time at a higher temperature than the aforementioned temperature. The baking can also be performed by irradiation with infrared light.

The electric conductivity of the stretchable conductive wire can be evaluated by measuring the electric resistance between both ends of the wire formed on the substrate. The stretchable conductive wire can be evaluated as excellent when the change in electric resistance before or during elongation of the substrate is small and the degradation of the electric conductivity after the elongated substrate is shrunk and returned to the original state is small. It is more preferable that no wire breakage occurs and the change in electric resistance is small in repetitive elongations and shrinkages.

Further, upon 20% elongation, the conductive wire of the present invention preferably has electric resistance of 500% or less of electric resistance before the elongation. The lower limit value is not particularly limited, and is preferably as low as possible; for example, 150% or more. When elongations and shrinkages are repeated 1000 times at an elongation ratio of 20%, the conductive wire preferably has a maximum electric resistance of 5000% or less of electric resistance before the elongations and shrinkages. The lower limit value is not particularly limited, and is preferably as low as possible; for example, 300% or more. These electric resistances can be measured according to a measurement method described later.

Further, a cover film may be provided to cover the conductive wire. Providing a cover film can improve the water resistance and mechanical strength. The cover film also needs to be stretchable since both of the substrate and the conductive wire are stretchable. As with the substrate, the material of the cover film can be selected from polyurethane, urethane acrylate, polyester, silicone, nitrile rubber, butadiene rubber, polyethylene, polypropylene, polyolefin, PTFE, PFA, and the like. The cover film to be used preferably has a thickness in a range of 10 nm to 1 mm.

The conductive wire formed by using the conductive paste composition of the present invention shows high electric conductivity, especially when it contains a silver powder, since silver nanoparticles formed in the wire baking process are dispersed in the insulating polymer among the silver powder particles. Furthermore, even when the distances between the silver powder particles are increased as a result of the wire elongation, the electric conduction path is not easily cut off because of the silver nanoparticles dispersed among the silver powders, so that the wire rarely breaks and the electric conductivity slightly changes.

EXAMPLE

Hereinafter, the present invention will be specifically described with reference to Synthesis Examples, Examples, and Comparative Examples. However, the present invention is not limited thereto. Note that weight-average molecular weight (Mw) and number-average molecular weight (Mn) were measured on polystyrene basis by gel permeation chromatography (GPC). The GPC measurement conditions are as follows.

Column: TSKgel G4000$H_{XL}$, TSKgel G3000$H_{XL}$, TSKgel G2000$H_{XL}$ (two)
Mobile phase: tetrahydrofuran
Column oven temperature: 40°C
Sample concentration: 0.20 mass%
Sample injection amount: 100 μL
Flow rate: 1 mL/min

A polyurethane containing a phenolic hydroxyl group was synthesized as follows.

[1] Synthesis of Phenolic Hydroxyl Group-Containing Capping Agent

Synthesis Example 1-1: Synthesis of Capping Agent 1

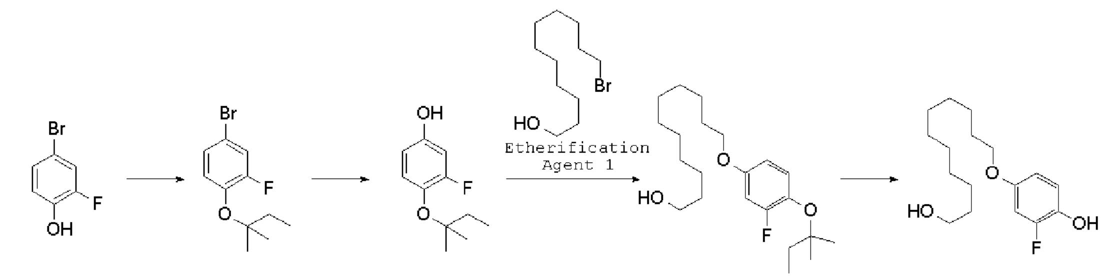


Bromophenol 1 Bromobenzene 1 Intermediate Phenol 1 Protecting Phenol 1 Capping Agent 1

Synthesis Example 1-1-1: Synthesis of Bromobenzene 1

Under a nitrogen atmosphere, methanesulfonic acid (4.8 g) was added to a toluene (10 g) solution of bromophenol 1 (76.4 g) and isoamylene (112.2 g) at -20°C to -10°C. After stirring at the same temperature for 3 hours, triethylamine (10.1 g) followed by 25 mass% of caustic soda water (32.0 g) were added dropwise to stop the reaction. Typical aqueous post-treatment (aqueous work-up) was performed. By distillation under reduced pressure, bromobenzene 1 was obtained (84.6 g, yield: 81%).
Boiling point: 67°C/10Pa.

Synthesis Example 1-1-2: Synthesis of Intermediate Phenol 1

Under a nitrogen atmosphere, a Grignard reagent prepared in advance using bromobenzene 1 (52.2 g), magnesium (5.1 g), and 140 mL of tetrahydrofuran was added dropwise to a solution of trimethyl borate (22.9 g) and tetrahydrofuran (310 mL) at an internal temperature of -5°C or less. The stirring was continued at a reaction temperature of 5°C for 3 hours. Then, acetic acid (18.0 g) and 35% hydrogen peroxide (25.3 g) were added at an internal temperature of 30°C or less. The stirring was continued at room temperature for 3 hours, followed by an ordinary post-treatment method and recrystallization with a mixed solution of toluene/n-hexane to obtain intermediate phenol 1 (29.7 g, yield: 75%) .

IR(D-ATR):$\nu$=3233, 3071, 2941, 2929, 2853, 1622, 1601, 1512, 1480, 1465, 1447, 1378, 1334, 1311, 1205, 1196, 1158, 1111, 1097, 975, 966, 870, 816$cm^{-1}$.
$^1$H-NMR(600MHz in DMSO-d6):$\delta$=9.52(1H, s), 6.88(1H, t), 6.55(1H, dd), 6.46(1H, dd), 1.62(2H, q), 1.14(6H, s), 0.93 (3H, t)ppm.
$^{19}$F-NMR(565MHz in DMSO-d6):$\delta$=-126.6(1F, s)ppm.

Synthesis Example 1-1-3: Synthesis of Protecting Phenol 1

Under a nitrogen atmosphere, etherification agent 1 (36.9 g) was added at 60°C to 80°C to a slurry solution of intermediate phenol 1 (29.5 g), potassium carbonate (20.5 g), sodium iodide (40 mg), and dimethylformaldehyde (74 g). After stirring at the same temperature for 20 hours, water (160 g) was added dropwise to stop the reaction. Typical aqueous post-treatment (aqueous work-up) was performed to obtain protecting phenol 1 (53.2 g, crude yield: 91%).

Synthesis Example 1-1-4: Synthesis of Capping Agent 1

A solution of the protecting phenol 1 (53.2 g) thus obtained, p-toluenesulfonic acid monohydrate (0.2 g), and toluene (140 g) was continuously heated and stirred at an internal temperature of 70°C to 100°C for 3 hours. Then, 50 g of water was added at an internal temperature of 30°C or less to stop the reaction. Thereafter, typical aqueous post-treatment (aqueous work-up) was performed, followed by recrystallization with a mixed solution of ethyl acetate/n-hexane to obtain capping agent 1 (31.6 g, yield of two steps: 71%). IR(D-ATR):$\nu$=3483, 3189, 2919, 2852, 1605, 1521, 1477, 1468, 1449, 1395, 1313, 1275, 1263, 1243, 1205, 1163, 1113, 1046, 1032, 1008, 955, 864, 840, 799, 788cm$^{-1}$. $^{1}$H-NMR(600MHz in DMSO-d6):$\delta$=9.18(1H, s), 6.82(1H, t), 6.74(1H, dd), 6.54(1H, dd), 4.29(1H, s), 3.83(2H, t), 3.36(2H, t), 1.63(2H, m), 1.16-1.44(16H, m)ppm. $^{19}$F-NMR(565MHz in DMSO-d6):5=-133.7(1F, t)ppm.

Synthesis Example 1-2: Synthesis of Capping Agent 2

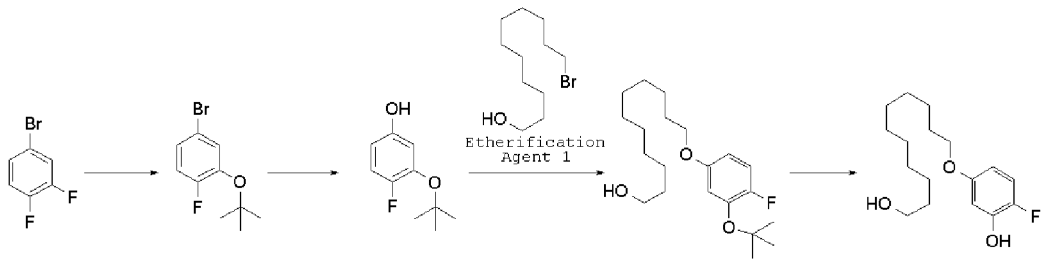


Fluorobenzene 1 Bromobenzene 2 Intermediate Phenol 2 Protecting Phenol 2 Capping Agent 2

Synthesis Example 1-2-1: Synthesis of Bromobenzene 2

Under a nitrogen atmosphere, fluorobenzene 1 (100 g) was added dropwise at 0°C to 10°C to a solution obtained by dissolving t-butoxy potassium (63.9 g) in THF (360 g). After stirring at the same temperature for 10 hours, water (150 g) was added dropwise to stop the reaction. Typical aqueous post-treatment (aqueous work-up) was performed. By distillation under reduced pressure, bromobenzene 2 was obtained (98.6 g, yield: 77%).
Boiling point: 97-100°C /700Pa.

Synthesis Example 1-2-2: Synthesis of Intermediate Phenol 2

Intermediate phenol 2 was obtained in the same manner as in Synthesis Example 1-1-2, except that bromobenzene 2 was used instead of bromobenzene 1 (yield: 77%).

Synthesis Example 1-2-3: Synthesis of Protecting Phenol 2

Protecting phenol 2 was obtained in the same manner as in Synthesis Example 1-1-3, except that intermediate phenol 2 was used instead of intermediate phenol 1 (yield: 92%).

Synthesis Example 1-2-4: Synthesis of Capping Agent 2

Capping agent 2 was obtained in the same manner as in Synthesis Example 1-1-4, except that protecting phenol 2 was used instead of protecting phenol 1 (yield: 78%).

Synthesis Example 1-3: Synthesis of Capping Agent 3

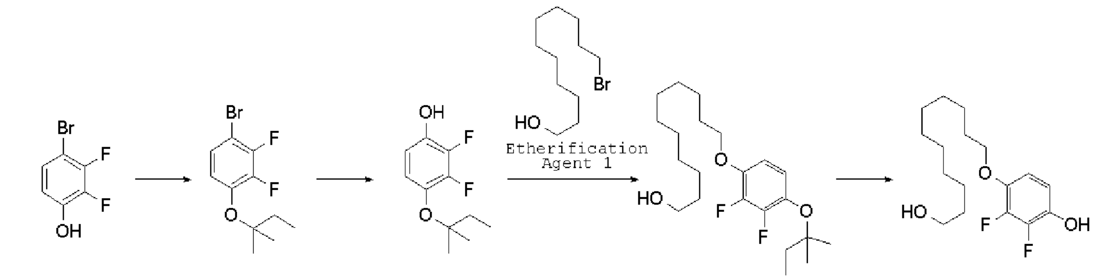


Bromophenol 3 Bromobenzene 3 Intermediate Phenol 3 Protecting Phenol 3 Capping Agent 3

Synthesis Example 1-3-1: Synthesis of Bromobenzene 3

Bromobenzene 3 was obtained in the same manner as in Synthesis Example 1-1-1, except that bromophenol 3 was used instead of bromophenol 1 (yield: 77%). Boiling point: 66°C/6Pa.

Synthesis Example 1-3-2: Synthesis of Intermediate Phenol 3

Intermediate phenol 3 was obtained in the same manner as in Synthesis Example 1-1-2, except that bromobenzene 3 was used instead of bromobenzene 1 (yield: 62%).

Synthesis Example 1-3-3: Synthesis of Protecting Phenol 3

Protecting phenol 3 was obtained in the same manner as in Synthesis Example 1-1-3, except that intermediate phenol 3 was used instead of intermediate phenol 1 (yield: 82%).

Synthesis Example 1-3-4: Synthesis of Capping Agent 3

Capping agent 3 was obtained in the same manner as in Synthesis Example 1-1-4, except that protecting phenol 3 was used instead of protecting phenol 1 (yield: 92%) .

IR(D-ATR):$\nu$=3494, 3215, 2932, 2918, 2849, 1613, 1526, 1503, 1480, 1470, 1434, 1400, 1287, 1264, 1212, 1183, 1091, 1077, 1054, 1020, 1004, 967, 942, 804cm$^{-1}$.
$^1$H-NMR(600MHz in DMSO-d6):$\delta$=9.75(1H, s), 6.70(2H, dt), 4.28(1H, t), 3.92(2H, t), 3.35(2H, m), 1.65(2H, m), 1.32-1.42(4H, m), 1.20-1.32(12H, m)ppm.
$^{19}$F-NMR(565MHz in DMSO-d6):$\delta$=-158.1~-157.9(1F, m), - 159.3~-159.1(1F, m)ppm.

Synthesis Example 1-4: Synthesis of Capping Agent 4

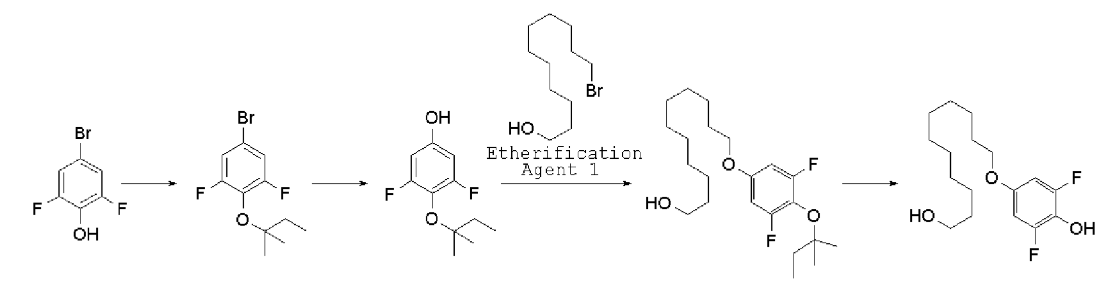


Bromophenol 4 Bromobenzene 4 Intermediate Phenol 4 Protecting Phenol 4 Capping Agent 4

Synthesis Example 1-4-1: Synthesis of Bromobenzene 4

Bromobenzene 4 was obtained in the same manner as in Synthesis Example 1-1-1, except that bromophenol 4 was used instead of bromophenol 1 (yield: 72%). Boiling point: 53°C/15Pa.

Synthesis Example 1-4-2: Synthesis of Intermediate Phenol 4

Intermediate phenol 4 was obtained in the same manner as in Synthesis Example 1-1-2, except that bromobenzene 4 was used instead of bromobenzene 1 (yield: 64%).
$^{1}$H-NMR(600MHz in DMSO-d6):$\delta$=9.99(1H, s), 6.44(2H, m), 1.62(2H, m), 1.15(6H, s), 0.94(3H, t)ppm. $^{19}$F-NMR(565MHz in DMSO-d6):$\delta$=-122.9(2F, s)ppm.

Synthesis Example 1-4-3: Synthesis of Protecting Phenol 4

Protecting phenol 4 was obtained in the same manner as in Synthesis Example 1-1-3, except that intermediate phenol 4 was used instead of intermediate phenol 1 (yield: 89%).

Synthesis Example 1-4-4: Synthesis of Capping Agent 4

Capping agent 4 was obtained in the same manner as in Synthesis Example 1-1-4, except that protecting phenol 4 was used instead of protecting phenol 1 (yield: 73%).

IR(D-ATR):$\nu$=3532, 3101, 2922, 2853, 2801, 1649, 1613, 1530, 1468, 1457, 1426, 1397, 1268, 1254, 1239, 1200, 1156, 1046, 1036, 1018, 855, 817, 804cm$^{-1}$.
$^{1}$H-NMR(600MHz in DMSO-d6):$\delta$=9.38(1H, s), 6.63(2H, m), 4.30(1H, s), 3.85(2H, t), 3.36(2H, t), 1.63(2H, m), 1.32-1.42(4H, m), 1.20-1.32(12H, m)ppm.
$^{19}$F-NMR(565MHz in DMSO-d6):$\delta$=-158.1(1F, m)ppm.

Synthesis Example 1-5: Synthesis of Capping Agent 5

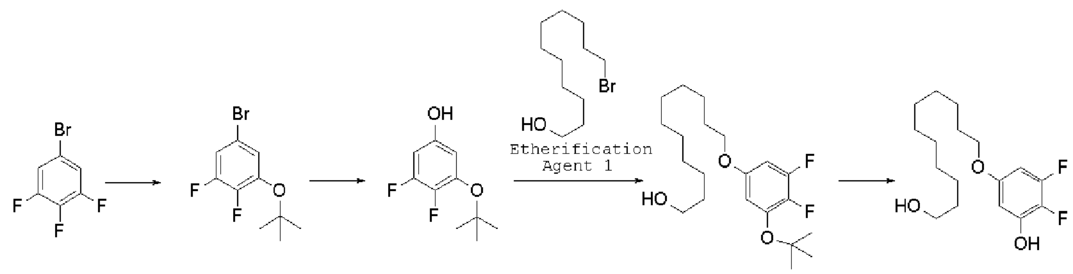


Fluorobenzene 2 Bromobenzene 5 Intermediate Phenol 5 Protecting Phenol 5 Capping Agent 5

Synthesis Example 1-5-1: Synthesis of Bromobenzene 5

Bromobenzene 5 was obtained in the same manner as in Synthesis Example 1-2-1, except that fluorobenzene 2 was used instead of fluorobenzene 1 (yield: 94%). Boiling point: 82-84°C/300Pa.

Synthesis Example 1-5-2: Synthesis of Intermediate Phenol 5

Intermediate phenol 5 was obtained in the same manner as in Synthesis Example 1-1-2, except that bromobenzene 5 was used instead of bromobenzene 1 (yield: 62%).

Synthesis Example 1-5-3: Synthesis of Protecting Phenol 5

Protecting phenol 5 was obtained in the same manner as in Synthesis Example 1-1-3, except that intermediate phenol 5 was used instead of intermediate phenol 1 (yield: 81%).

Synthesis Example 1-5-4: Synthesis of Capping Agent 5

Capping agent 5 was obtained in the same manner as in Synthesis Example 1-1-4, except that protecting phenol 5 was used instead of protecting phenol 1 (yield: 77%).

Synthesis Example 1-6: Synthesis of Capping Agent 6

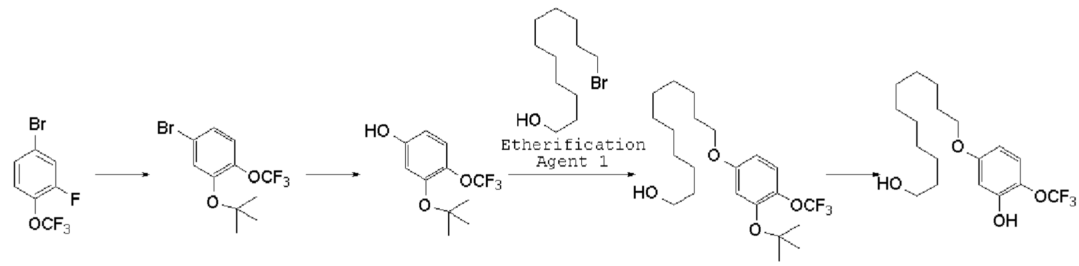


Fluorobenzene 3 Bromobenzene 6 Intermediate Phenol 6 Protecting Phenol 6 Capping Agent 6

Synthesis Example 1-6-1: Synthesis of Bromobenzene 6

Bromobenzene 6 was obtained in the same manner as in Synthesis Example 1-2-1, except that fluorobenzene 3 was used instead of fluorobenzene 1 (yield: 77%). Boiling point: 99-100°C/1KPa.

Synthesis Example 1-6-2: Synthesis of Intermediate Phenol 6

Intermediate phenol 6 was obtained in the same manner as in Synthesis Example 1-1-2, except that bromobenzene 6 was used instead of bromobenzene 1 (yield: 68%).

Synthesis Example 1-6-3: Synthesis of Protecting Phenol 6

Protecting phenol 6 was obtained in the same manner as in Synthesis Example 1-1-3, except that intermediate phenol 6 was used instead of intermediate phenol 1 (yield: 86%).

Synthesis Example 1-6-4: Synthesis of Capping Agent 6

Capping agent 6 was obtained in the same manner as in Synthesis Example 1-1-4, except that protecting phenol 6 was used instead of protecting phenol 1 (yield: 86%) .

Synthesis Example 1-7: Synthesis of Capping Agent 7

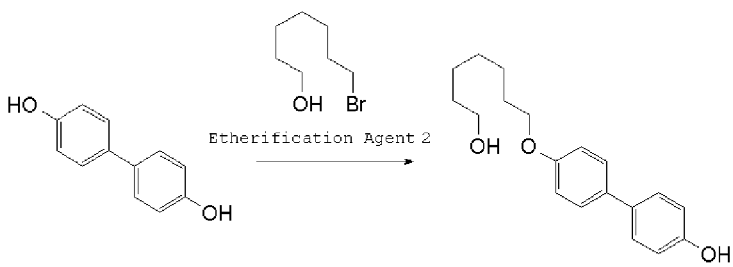


Dihydroxybenzene 1 Capping Agent 7

Synthesis Example 1-7-1: Synthesis of Capping Agent 7

Under a nitrogen atmosphere, etherification agent 2 (19.5 g) was added at 60°C to 80°C to a slurry solution of

dihydroxybenzene 1 (55.9 g), potassium carbonate (41.5 g), sodium iodide (100 mg), and dimethylformaldehyde (300 g). After stirring at the same temperature for 3 hours, water (400 g) was added dropwise to stop the reaction. Typical aqueous post-treatment (aqueous work-up) was performed. Recrystallization was performed with a mixed solution of ethyl acetate/n-hexane to obtain capping agent 7 (18.6 g, yield: 62%).

Synthesis Example 1-8: Synthesis of Capping Agent 8

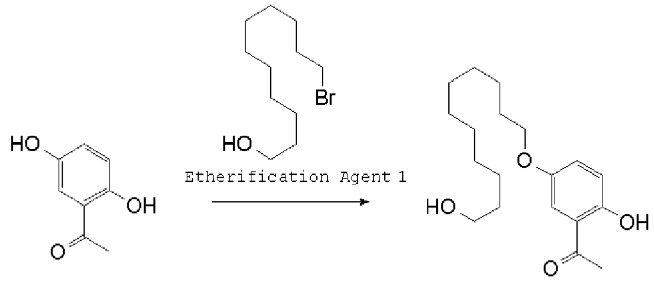


Dihydroxybenzene 2

Capping Agent 8

Synthesis Example 1-8-1: Synthesis of Capping Agent 8

Capping agent 8 was obtained in the same manner as in Synthesis Example 1-7-1, except that dihydroxybenzene 2 and etherification agent 1 were used instead of dihydroxybenzene 1 and etherification agent 2 respectively (yield: 43%).

Synthesis Example 1-9: Synthesis of Capping Agent 9

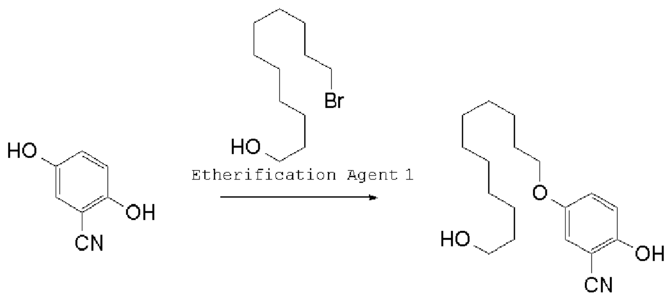


Dihydroxybenzene 3

Capping Agent 9

Synthesis Example 1-9-1: Synthesis of Capping Agent 9

Capping agent 9 was obtained in the same manner as in Synthesis Example 1-7-1, except that dihydroxybenzene 3 and etherification agent 1 were used instead of dihydroxybenzene 1 and etherification agent 2 respectively (yield: 45%).

Synthesis Example 1-10: Synthesis of Capping Agent 10

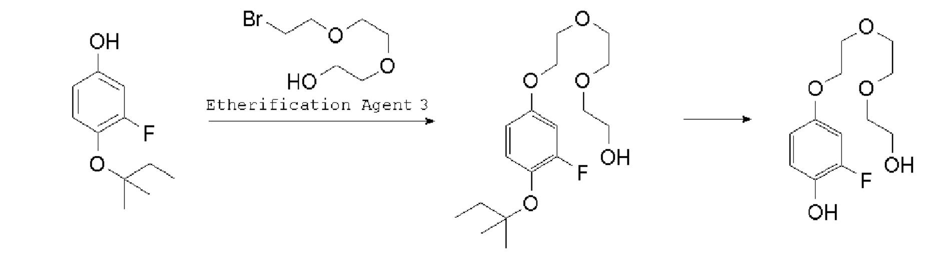


Intermediate Phenol 1 Protecting Phenol 10 Capping Agent 10

Synthesis Example 1-10-1: Synthesis of Protecting Phenol 10

Protecting phenol 10 was obtained in the same manner as in Synthesis Example 1-1-3, except that etherification agent 3 was used instead of etherification agent 1 (yield: 81%).

Synthesis Example 1-10-2: Synthesis of Capping Agent 10

Capping agent 10 was obtained in the same manner as in Synthesis Example 1-1-4, except that protecting phenol 10 was used instead of protecting phenol 1 (yield 86%) .

Synthesis Example 1-11: Synthesis of Capping Agent 11

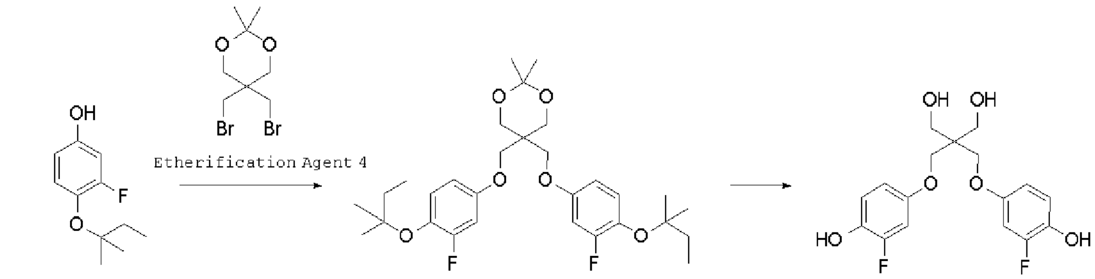


Intermediate Phenol 1 Protecting Phenol 11 Capping Agent 11

Synthesis Example 1-11-1: Synthesis of Protecting Phenol 11

Protecting phenol 11 was obtained in the same manner as in Synthesis Example 1-1-3, except that etherification agent 4 was used instead of etherification agent 1 (yield: 82%).

Synthesis Example 1-11-2: Synthesis of Capping Agent 11

Capping agent 11 was obtained in the same manner as in Synthesis Example 1-1-4, except that protecting phenol 11 was used instead of protecting phenol 1 (yield: 75%).
IR(D-ATR):ν=3619, 3292, 2948, 2890, 1608, 1516, 1452, 1386, 1280, 1234, 1191, 1157, 1109, 1101, 1052, 1023, 966, 945, 912, 851, 841, 823, 798, 756, 740cm$^{-1}$. $^{1}$H-NMR(600MHz in DMSO-d6):5=9.20(2H, s), 6.82(2H, t), 6.76(2H, dd), 6.57(2H,dd), 4.55(2H, t), 3.85(4H, s), 3.54(4H, d)ppm.

[2] Synthesis of Weakly Acidic Functional Group-Containing Polyol

Synthesis Example 2-1: Synthesis of Hexafluoroalcohol-Containing Chain Extender 1

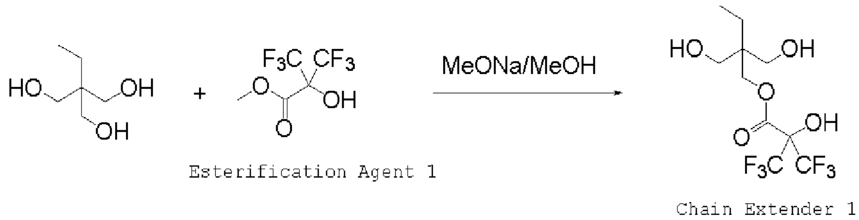


Synthesis Example 2-1-1: Synthesis of Chain Extender 1

Under a nitrogen atmosphere, trimethylolpropane (1260 g), esterification agent 1 (1415 g), and a methanol solution (60.7 g) of 28 mass% of sodium methoxide were stirred at 100°C to 140°C. Ester exchange was performed while methanol was being distilled off. After the reaction was completed, the resultant was cooled to 50°C or less and neutralized with hydrochloric acid. Thereafter, typical aqueous post-treatment (aqueous work-up) was performed, followed by recrystallization with a mixed solution of IPE/n-hexane to obtain 949 g of chain extender 1 (purity: 99.1%, yield: 46.2%).
Chain extender 1:

White powder
$^1$H-NMR(DMSO-d6):δ=0.78 (3H,t), 1.26 (2H,q), 3.27 (4H,m), 4.17 (2H,s), 4.49 (2H,t), 9.08 (1H,s)

Synthesis Example 2-2: Synthesis of Hexafluoroalcohol-Containing Chain Extender 2

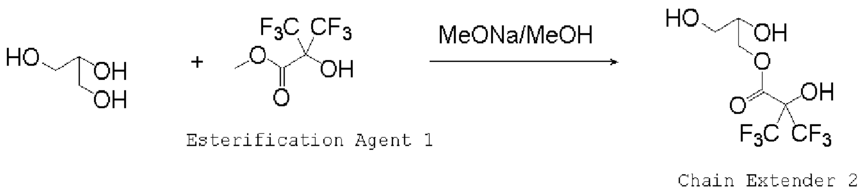


Synthesis Example 2-2-1: Synthesis of Chain Extender 2

Chain extender 2 was obtained in the same manner as in Synthesis Example 2-1-1, except that glycerol was used instead of trimethylolpropane as a raw material (yield: 40%).

Synthesis Example 2-3: Synthesis of Hexafluoroalcohol-Containing Chain Extender 3

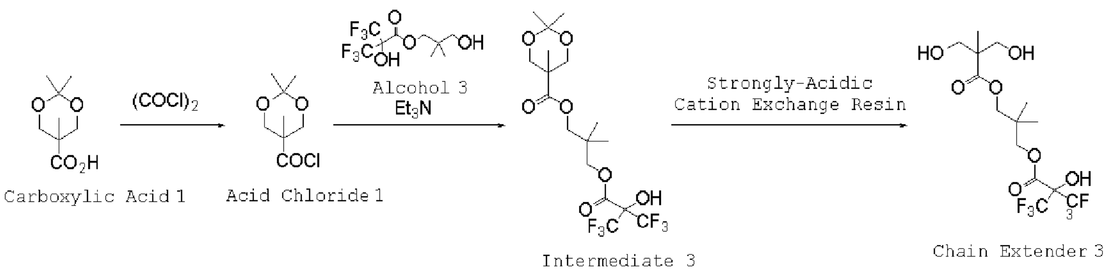


Synthesis Example 2-3-1: Synthesis of Intermediate 3

Under a nitrogen atmosphere, oxalyl chloride (8.7 g) was added to a suspension containing carboxylic acid 1 (10.0 g), toluene (50.0 g), and 2 drops of N,N-dimethylformamide. After stirring at room temperature for 5 hours, the solvent was distilled off under reduced pressure to obtain acid chloride 1.

Under a nitrogen atmosphere, an acetonitrile solution of acid chloride 1 was added to a solution of ice-cooled alcohol 3 (15.6 g), triethylamine (7.9 g), and acetonitrile (20 mL), and stirred at room temperature overnight. After the reaction was completed, water was added. Typical post-treatment was performed to obtain 19.0 g of intermediate 3 (crude yield: 80%).

Synthesis Example 2-3-2: Synthesis of Chain Extender 3

The intermediate 3 (15.0 g) thus obtained, methanol (30 g), and strongly-acidic cation exchange resin (1 g) were heated under reflux. While acetone was distilled off, deprotection was performed. After the reaction was completed, the ion exchange resin was removed and the solvent was distilled off. Thus 13.3 g of chain extender 3 was obtained (crude yield: 97%).

Synthesis Example 2-4: Synthesis of Hexafluoroalcohol-Containing Chain Extender 4

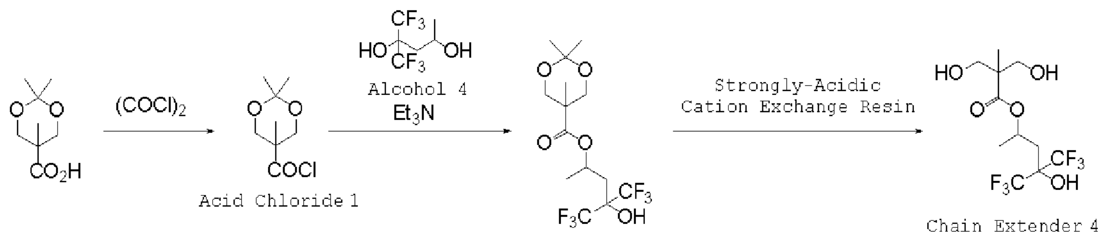


Acid Chloride 1 Intermediate 4 Chain Extender 4

Synthesis Example 2-4-1: Synthesis of Intermediate 4

Intermediate 4 was obtained in the same manner as in Synthesis Example 2-3-1, except that alcohol 4 was used instead of alcohol 3.

Synthesis Example 2-4-2: Synthesis of Chain Extender 4

Chain extender 4 was obtained in the same manner as in Synthesis Example 2-3-2, except that intermediate 4 was used instead of intermediate 3 (yield of two steps: 75%).

Synthesis Example 2-5: Synthesis of Pentafluoroalcohol-Containing Chain Extender 5

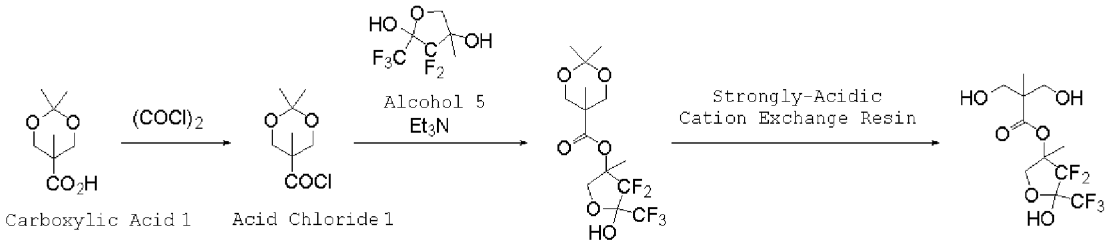


Carboxylic Acid 1 Acid Chloride 1 Intermediate 5 Chain Extender 5

Synthesis Example 2-5-1: Synthesis of Intermediate 5

Intermediate 5 was obtained in the same manner as in Synthesis Example 2-3-1, except that alcohol 5 was used instead of alcohol 3.

Synthesis Example 2-5-2: Synthesis of Chain Extender 5

Chain extender 5 was obtained in the same manner as in Synthesis Example 2-3-2, except that intermediate

5 was used instead of intermediate 3 (yield of two steps: 75%).

Synthesis Example 2-6: Synthesis of Trifluoromethanesulfonamide-Containing Chain Extender 6

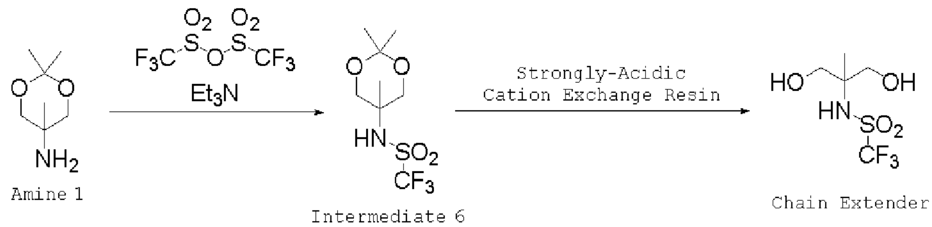


Amine 1 Intermediate 6 Chain Extender 6

Synthesis Example 2-6-1: Synthesis of Intermediate 6

Under a nitrogen atmosphere, trifluoromethanesulfonic anhydride (32.1 g) was added dropwise at -78°C to a mixed solution containing amine 1 (15.0 g), dichloromethane (45.0 g), and triethylamine (12.5 g), and stirred at the same temperature for 2 hours. The temperature was increased to room temperature, and the mixture was further stirred for 1 hour. Then, water was added while the mixture was being cooled with ice to stop the reaction. Thereafter, typical post-treatment was performed to obtain 22.7 g of intermediate 6 (crude yield: 78%).

Synthesis Example 2-6-2: Synthesis of Chain Extender 6

Chain extender 6 was obtained in the same manner as in Synthesis Example 2-3-2, except that intermediate 6 was used instead of intermediate 3 (yield: 87%).

[3] Synthesis of Weakly Acidic Functional Group-Containing Polyurethane

Raw materials

<High-Molecular-Weight Polyols>

- NIPPOLAN 4010 (manufactured by Tosoh Corporation): polyester polyol, number-average molecular weight: 2000
- NIPPOLAN 4009 (manufactured by Tosoh Corporation): polyester polyol, number-average molecular weight: 1000
- KURARAY POLYOL P-2010 (manufactured by KURARAY CO., LTD.): polyester polyol, number-average molecular weight: 2000
- KURARAY POLYOL C-2090 (manufactured by KURARAY CO., LTD.): polycarbonate polyol, number-average molecular weight: 2000
- PLACCEL 210 (manufactured by DAICEL CORPORATION): polycaprolactone diol, number-average molecular weight: 1000

<Diisocyanate>

- 2,4-tolylene diisocyanate or 2,6-tolylene diisocyanate, and isomer mixture thereof (TDI)
- isophorone diisocyanate (IPDI)

<Chain Extender>

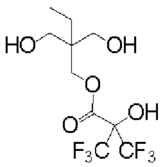 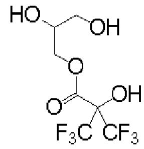 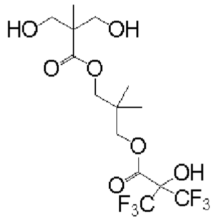 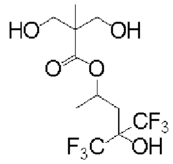 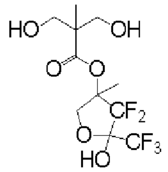 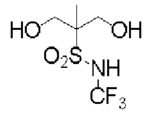

Chain Extender 6

Chain Extender1 Chain Extender 2 Chain Extender 3 Chain Extender 4 Chain Extender 5

<Capping Agent>

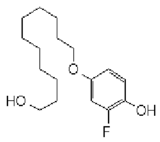 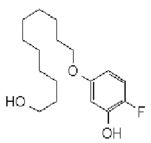 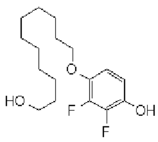 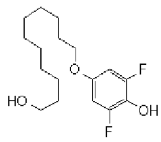 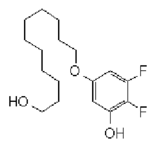 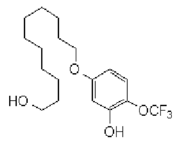

Capping Agent 1 Capping Agent 2 Capping Agent 3 Capping Agent 4 Capping Agent 5 Capping Agent 6

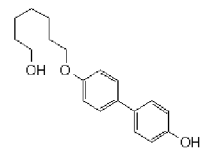 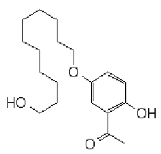 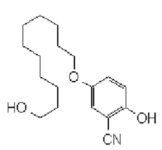 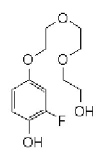 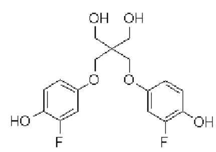 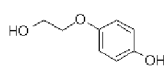

Capping Agent 7 Capping Agent 8 Capping Agent 9 Capping Agent 10 Capping Agent 11 Capping Agent 12

Commercially available capping agent 12 was used.

<Catalyst>

- XK-640 (manufactured by KING INDUSTRIES, Inc.)

Synthesis Example 3

Under a nitrogen atmosphere, 40 mass% of NIPPOLAN 4009 in 445.8 g of BCA solution was added dropwise to 86.0 g of TDI, 134.2 g of diethylene glycol monobutyl ether acetate (BCA), and 0.36 g of XK-640 (manufactured by KING INDUSTRIES, Inc.), and the mixture was stirred for 1 hour to prepare prepolymer. The prepolymer solution was heated to 90°C, and 50 mass% of chain extender 1 in 232.9 g of BCA solution was added dropwise thereto. After it was matured at the same temperature for 10 hours, 10 mass% of capping agent 1 in 91.1 g of BCA solution was added and matured for 1 hour.
PU1:
Mw=125,190, Mw/Mn=3.53

PU2 to PU17 and Comparative PU1 to Comparative PU3 were synthesized by the same procedure as in Synthesis Example 3, except that the kinds and proportions of diisocyanate, high-molecular-weight polyol, chain extender, and capping agent, as well as the amount of catalyst used were changed to those shown in Tables 1 and 2.

[TABLE 1]

| | High-Molecular-Weight Polyols [part by mass] | Diisocyanate [part by mass] | Chain Extender [part by mass] | Capping Agent [part by mass] | Catalyst [part by mass] | Mw | Mw/Mn |
|---|---|---|---|---|---|---|---|
| PU1 | NIPPOLAN 4009 [45] | TDI [24] | 1[29] | 1[2] | XK-640 [0.1] | 125,190 | 3.53 |

(continued)

| | High-Molecular-Weight Polyols [part by mass] | Diisocyanate [part by mass] | Chain Extender [part by mass] | Capping Agent [part by mass] | Catalyst [part by mass] | Mw | Mw/ Mn |
|---|---|---|---|---|---|---|---|
| PU2 | NIPPOLAN 4009 [43] | IPDI [31] | 2 [24] | 1[2] | XK-640 [2.00] | 72,110 | 3.22 |
| PU3 | NIPPOLAN 4010 [46] | TDI [31] | 3[31] | 1[2] | XK-640 [0.1] | 98,690 | 4.15 |
| PU4 | P-2010 [46] | TDI [32] | 4[30] | 1[2] | XK-640 [0.1] | 89,330 | 4.24 |
| PU5 | C-2090 [46] | TDI [32] | 5[30] | 1[2] | XK-640 [0.1] | 78,980 | 3.61 |
| PU6 | PLACCEL 210 [45] | TDI [24] | 6[29] | 1[2] | XK-640 [0.1] | 82,630 | 3.99 |
| PU7 | NIPPOLAN 4009 [45] | TDI [24] | 1[29] | 2[2] | XK-640 [0.1] | 111,430 | 4.22 |
| PU8 | NIPPOLAN 4009 [45] | TDI [24] | 1[29] | 3[2] | XK-640 [0.1] | 110,260 | 4.12 |
| PU9 | NIPPOLAN 4009 [45] | TDI [24] | 1[29] | 4[2] | XK-640 [0.1] | 127,670 | 4.65 |
| PU10 | NIPPOLAN 4009 [45] | TDI [24] | 1[29] | 5[2] | XK-640 [0.1] | 95,410 | 4.11 |

[TABLE 2]

| | High-Molecular-Weight Polyols [part by mass] | Diisocyanate [part by mass] | Chain Extender [part by mass] | Capping Agent [part by mass] | Catalyst [part by mass] | Mw | Mw/ Mn |
|---|---|---|---|---|---|---|---|
| PU11 | NIPPOLAN 4009 [45] | TDI [24] | 1[29] | 6[2] | XK-640 [0.1] | 100, 990 | 4.73 |
| PU12 | NIPPOLAN 4009 [45] | TDI [24] | 1[29] | 7[2] | XK-640 [0.1] | 99,860 | 3. 93 |
| PU13 | NIPPOLAN 4009 [45] | TDI [24] | 1[29] | 8[2] | XK-640 [0.1] | 102,770 | 4.33 |
| PU14 | NIPPOLAN 4009 [45] | TDI [24] | 1[29] | 9[2] | XK-640 [0.1] | 115,040 | 4.88 |
| PU15 | NIPPOLAN 4009 [45] | TDI [24] | 1[29] | 10[2] | XK-640 [0.1] | 111,130 | 5.15 |
| PU16 | NIPPOLAN 4009 [45] | TDI [24] | 1[29] | 11[2] | XK-640 [0.1] | 104,950 | 4.44 |
| PU17 | NIPPOLAN 4009 [45] | TDI [24] | 1[29] | 12 [2] | XK-640 [0.1] | 121,670 | 4.57 |
| Comparative PU1 | NIPPOLAN 4009 [46] | TDI [24] | 1[30] | | XK-640 [0.5] | 119, 220 | 4.22 |
| Comparative PU2 | NIPPOLAN 4009 [43] | IPDI [30] | 2 [27] | | XK-640 [2.00] | 63,690 | 2.05 |

(continued)

| | High-Molecular-Weight Polyols [part by mass] | Diisocyanate [part by mass] | Chain Extender [part by mass] | Capping Agent [part by mass] | Catalyst [part by mass] | Mw | Mw/Mn |
|---|---|---|---|---|---|---|---|
| Comparative PU3 | NIPPOLAN 4009 [45] | TDI [24] | 5[31] | | XK-640 [0.05] | 79,680 | 2.42 |

A phenol compound to be used as an additive was synthesized as follows.

[4] Synthesis of Phenol Compound

Synthesis Example 4-1: Synthesis of Phenol 1

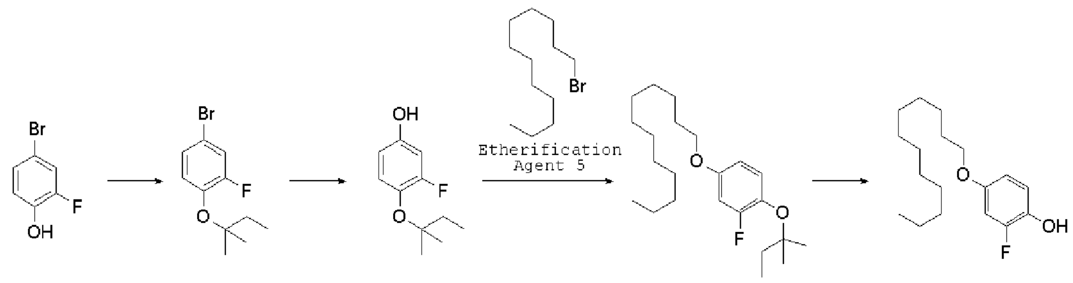


Bromophenol 1 Bromobenzene 1 Intermediate Phenol 1 Protecting Phenol 12 Phenol 1

Synthesis Example 4-1-1: Synthesis of Protecting Phenol 12

Protecting phenol 12 was obtained in the same manner as in Synthesis Example 1-1-3, except that etherification agent 5 was used instead of etherification agent 1 (yield: 85%).

Synthesis Example 4-1-2: Synthesis of Phenol 1

Phenol 1 was obtained in the same manner as in Synthesis Example 1-1-4, except that protecting phenol 12 was used instead of protecting phenol 1 (yield: 84%). IR(D-ATR):ν=3402, 2953, 2942, 2920, 2870, 2854, 1607, 1516, 1479, 1471, 1460, 1398, 1313, 1275, 1255, 1205, 1158, 1111, 1028, 840, 788cm$^{-1}$.

$^1$H-NMR(600MHz in DMSO-d6):δ=9.18(1H, s), 6.82(1H, t), 6.72(1H, dd), 6.54(1H, dd), 3.82(2H, t), 1.62(2H, m), 1.14-1.38(18H, m), 0.83(3H, t)ppm.
$^{19}$F-NMR(565MHz in DMSO-d6):5=-133.7(1F, t)ppm.

Synthesis Example 4-2: Synthesis of Phenol 2

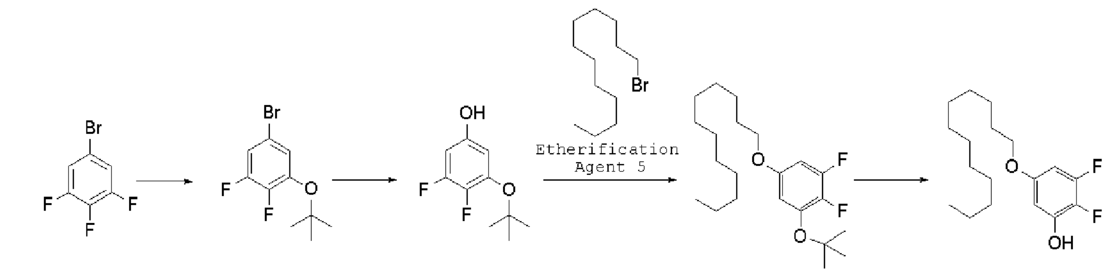


Fluorobenzene 2 Bromobenzene 5 Intermediate Phenol 5 Protecting Phenol 13 Phenol 2

Synthesis Example 4-2-1: Synthesis of Protecting Phenol 13

Protecting phenol 13 was obtained in the same manner as in Synthesis Example 4-1-1, except that intermediate phenol 5 was used instead of intermediate phenol 1 (yield: 88%).

Synthesis Example 4-2-2: Synthesis of Phenol 2

Phenol 2 was obtained in the same manner as in Synthesis Example 1-1-4, except that protecting phenol 13 was used instead of protecting phenol 1 (yield: 72%). IR(D-ATR):ν=3423, 2954, 2942, 2920, 2853, 1639, 1616, 1531, 1478, 1472, 1379, 1400, 1379, 1244, 1214, 1152, 1054, 1032, 1020, 828, 822, 809, 789cm$^{-1}$.

$^{1}$H-NMR (major isomers only, 600MHz in DMSO-d6) :δ=10.32(1H, s), 6.38(1H, m), 6.27(1H, m), 3.82 (2H, t), 1.63(2H, m), 1.16-1.40(18H, m), 0.82(3H, t)ppm.
$^{19}$F-NMR (major isomers only, 565MHz in DMSO-d6):5=-137.4(1F, m), -171.7(1F, m)ppm.

Synthesis Example 4-3: Synthesis of Phenol 3

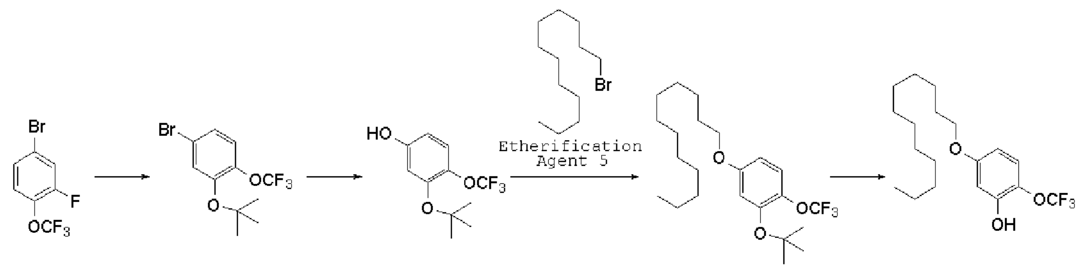


Fluorobenzene 3 Bromobenzene 6 Intermediate Phenol 6 Protecting Phenol 14 Phenol 3

Synthesis Example 4-3-1: Synthesis of Protecting Phenol 14

Protecting phenol 14 was obtained in the same manner as in Synthesis Example 4-1-1, except that intermediate phenol 6 was used instead of intermediate phenol 1 (yield: 90%).

Synthesis Example 4-3-2: Synthesis of Phenol 3

Phenol 3 was obtained in the same manner as in Synthesis Example 1-1-4, except that protecting phenol 14 was used instead of protecting phenol 1 (yield: 81%). IR(D-ATR):ν=3591, 3446, 2926, 2855, 1605, 1507, 1468, 1391, 1254, 1204, 1172, 1026, 983, 912, 836, 792cm$^{-1}$. $^{1}$H-NMR(600MHz in DMSO-d6):δ=10.06(1H, s), 7.08(1H, d), 6.49(1H, d), 6.34(1H, dd), 3.86(2H, t), 1.64(2H, m), 1.15-1.38(18H, m), 0.83(3H, t)ppm.
$^{19}$F-NMR(565MHz in DMSO-d6):5=-57.5(3F, t)ppm.

Synthesis Example 4-4: Synthesis of Phenol 4

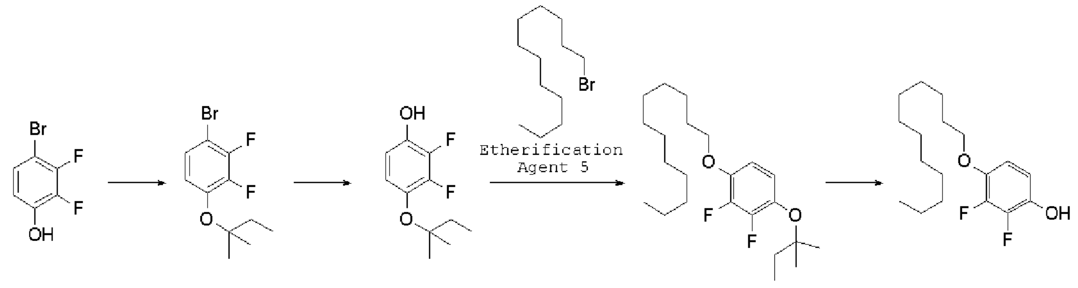


Bromophenol 3 Bromobenzene 3 Intermediate Phenol 3 Protecting Phenol 15 Phenol 4

Synthesis Example 4-4-1: Synthesis of Protecting Phenol 15

Protecting phenol 15 was obtained in the same manner as in Synthesis Example 4-1-1, except that intermediate phenol 3 was used instead of intermediate phenol 1 (yield: 91%).

Synthesis Example 4-4-2: Synthesis of Phenol 4

Phenol 4 was obtained in the same manner as in Synthesis Example 1-1-4, except that protecting phenol 15 was used instead of protecting phenol 1 (yield: 76%). IR(D-ATR):$\nu$=3290, 2956, 2918, 2873, 2849, 1615, 1526, 1502, 1470, 1420, 1398, 1284, 1265, 1216, 1180, 1089, 1062, 1053, 1042, 1030, 1016, 975, 945, 802cm$^{-1}$.

$^{1}$H-NMR(600MHz in DMSO-d6):$\delta$=9.74(1H, s), 6.70(2H, dt), 3.91(2H, t), 1.64(2H, m), 1.32-1.39(2H, m), 1.16-1.32(16H, m), 0.82(3H, t)ppm.
$^{19}$F-NMR(565MHz in DMSO-d6):$\delta$=-158.1~-157.9(1F, m), - 159.3~-159.1(1F, m)ppm.

Synthesis Example 4-5: Synthesis of Phenol 5

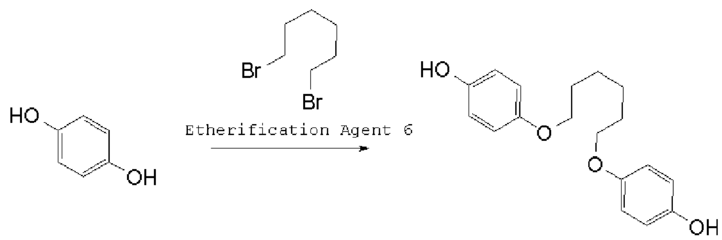


Hydroquinone Phenol 5

Synthesis Example 4-5-1: Synthesis of Phenol 5

Phenol 5 was obtained in the same manner as in Synthesis Example 1-7-1, except that hydroquinone and etherification agent 6 were used instead of dihydroxybenzene 1 and etherification agent 2 respectively (yield: 59%).

Synthesis Example 4-6: Synthesis of Phenol 6

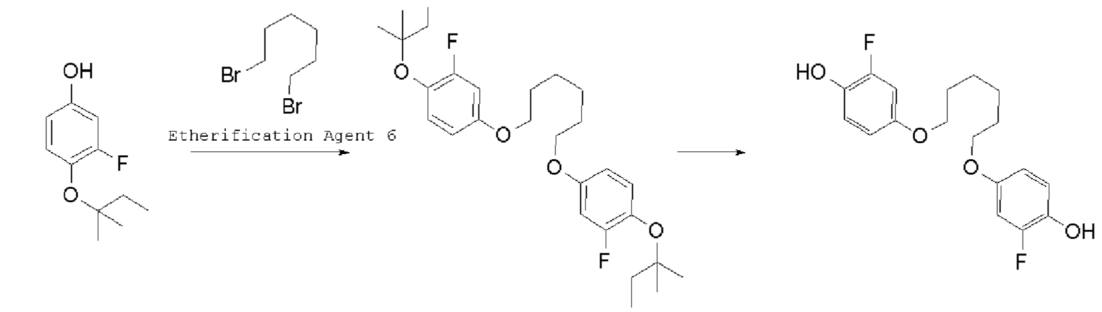


Intermediate Phenol 1 Protecting Phenol 16 Phenol 6

Synthesis Example 4-6-1: Synthesis of Protecting Phenol 16

Protecting phenol 16 was obtained in the same manner as in Synthesis Example 1-1-3, except that etherification agent 6 was used instead of etherification agent 1 (yield: 94%).

Synthesis Example 4-6-2: Synthesis of Phenol 6

Phenol 6 was obtained in the same manner as in Synthesis Example 1-1-4, except that protecting phenol 16 was used instead of protecting phenol 1 (yield: 90%). IR(D-ATR):ν=3380, 2944, 2869, 1642, 1606, 1516, 1477, 1446, 1394, 1368, 1317, 1263, 1242, 1195, 1162, 1109, 1023, 969, 956, 867, 838, 796$cm^{-1}$.

$^{1}$H-NMR(600MHz in DMSO-d6):δ=9.19(2H, s), 6.82(2H, t), 6.75(2H, dd), 6.55(2H, dd), 3.85(4H, t), 1.66(4H, quin), 1.42 (4H, m)ppm.
$^{19}$F-NMR(565MHz in DMSO-d6):δ=-133.6(2F, t)ppm.

Synthesis Example 4-7: Synthesis of Phenol 7

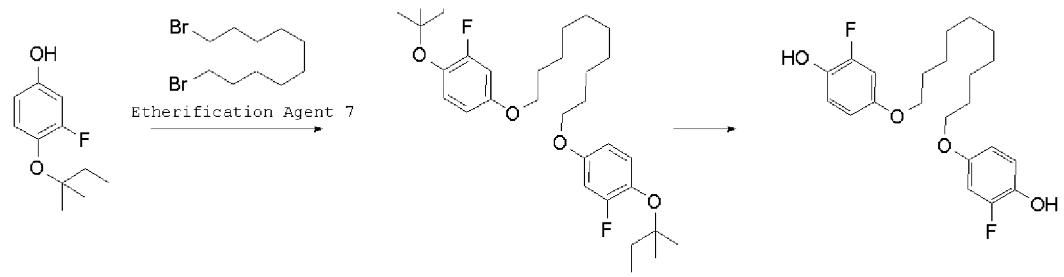


Intermediate Phenol 1 Protecting Phenol 17 Phenol 7

Synthesis Example 4-7-1: Synthesis of Protecting Phenol 17

Protecting phenol 17 was obtained in the same manner as in Synthesis Example 1-1-3, except that etherification agent 7 was used instead of etherification agent 1 (yield: 92%).

Synthesis Example 4-7-2: Synthesis of Phenol 7

Phenol 7 was obtained in the same manner as in Synthesis Example 1-1-4, except that protecting phenol 17 was used instead of protecting phenol 1 (yield: 87%). IR(D-ATR):ν=3385, 2941, 2923, 2856, 1608, 1514, 1479, 1468, 1455, 1288, 1276, 1253, 1203, 1156, 1110, 1042, 1022, 988, 956, 842, 818, 802, 787, 748$cm^{-1}$.

$^{1}$H-NMR(600MHz in DMSO-d6):δ=9.18(2H, s), 6.82(2H, t), 6.74(2H, dd), 6.55(2H, dd), 3.83(4H, t), 1.66(4H, quin), 1.20-1.41(12H, m)ppm.
$^{19}$F-NMR(565MHz in DMSO-d6):δ=-133.7(2F, t)ppm.

Synthesis Example 4-8: Synthesis of Phenol 8

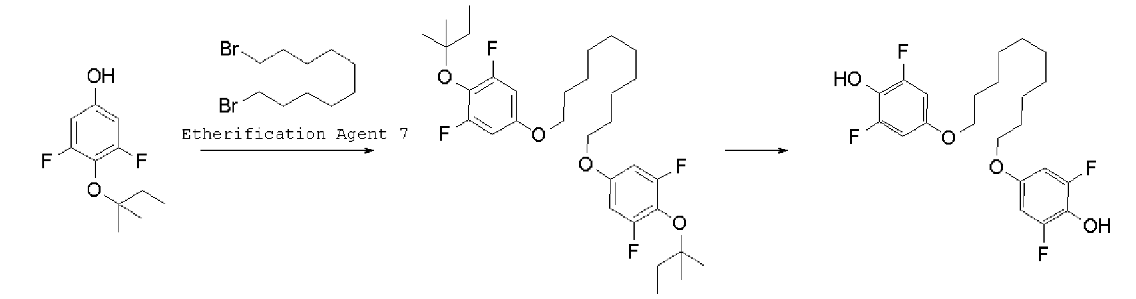


Intermediate Phenol 4 Protecting Phenol 18 Phenol 8

Synthesis Example 4-8-1: Synthesis of Protecting Phenol 18

Protecting phenol 18 was obtained in the same manner as in Synthesis Example 1-1-3, except that intermediate phenol 4 and etherification agent 7 were used instead of intermediate phenol 1 and etherification agent 1 respectively (yield: 88%).

Synthesis Example 4-8-2: Synthesis of Phenol 8

Phenol 8 was obtained in the same manner as in Synthesis Example 1-1-4, except that protecting phenol 18 was used instead of protecting phenol 1 (yield: 84%). IR(D-ATR):ν=3401, 2942, 2923, 2856, 1648, 1616, 1528, 1479, 1469, 1459, 1378, 1247, 1202, 1148, 1045, 1023, 1017, 823, 802cm$^{-1}$.

$^{1}$H-NMR(600MHz in DMSO-d6):5=9.38(2H, s), 6.64(4H, m), 3.85(4H, t), 3.36(2H, t), 1.64(4H, m), 1.32-1.42(4H, m), 1.18-1.40(12H, m)ppm.
$^{19}$F-NMR(565MHz in DMSO-d6):δ=-131.0(4F, d)ppm.

Synthesis Example 4-9: Synthesis of Phenol 9

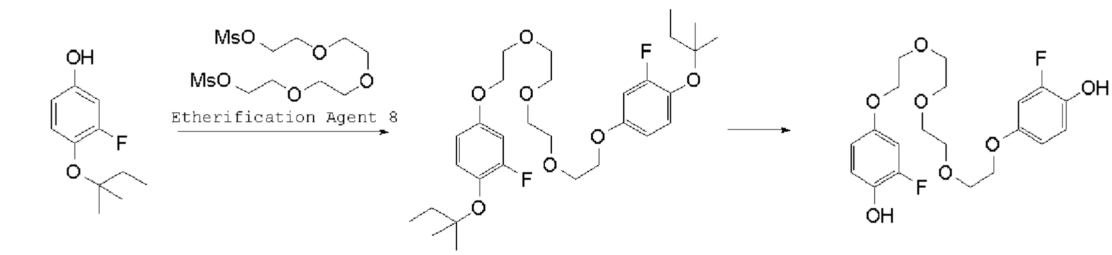


Intermediate Phenol 1 Protecting Phenol 19 Phenol 9

(wherein Ms represents a mesyl group.)

Synthesis Example 4-9-1: Synthesis of Protecting Phenol 19

Protecting phenol 19 was obtained in the same manner as in Synthesis Example 1-1-3, except that etherification agent 8 was used instead of etherification agent 1 (yield: 85%).

Synthesis Example 4-9-2: Synthesis of Phenol 9

Phenol 9 was obtained in the same manner as in Synthesis Example 1-1-4, except that protecting phenol 19 was used instead of protecting phenol 1 (yield: 81%). $^{1}$H-NMR(600MHz in DMSO-d6):δ=9.22(2H, s), 6.83(2H, dd),

6.78(2H, dd), 6.57(2H, ddd), 3.95~3.98(4H, m), 3.65-3.70(4H, m), 3.50-3.56(8H, m)ppm.
$^{19}$F-NMR(565MHz in DMSO-d6):δ=-136.8(2F, t)ppm.

Synthesis Example 4-10: Synthesis of Phenol 10

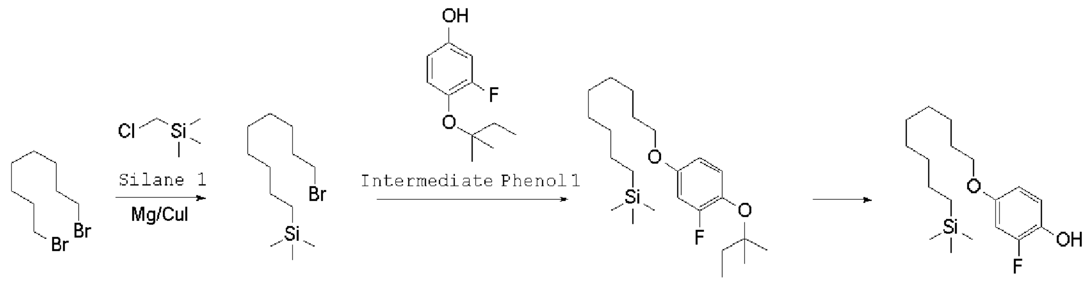


Dibromo Compound 1 Etherification Agent 9 Protecting Phenol 20 Phenol 10

Synthesis Example 4-10-1: Synthesis of Etherification Agent 9

Under a nitrogen atmosphere, a Grignard reagent prepared in advance using silane 1 (30.7 g), magnesium (6.7 g), and 120 mL of tetrahydrofuran was added dropwise at an internal temperature of 10°C to 30°C to a solution of dibromo compound 1 (75.0 g), cuprous iodide (0.52 g), triethyl phosphite (0.96 g), and tetrahydrofuran (100 mL). Stirring was continued at the same reaction temperature for 20 hours. Then, a saturated aqueous solution of ammonium chloride (200 g) was added at an internal temperature of 30°C or less to stop the reaction. A typical post-treatment method was performed to obtain etherification agent 9 (43.3 g, yield: 62%).
Boiling point: 72°C/20Pa.

Synthesis Example 4-10-2: Synthesis of Protecting Phenol 20

Protecting phenol 20 was obtained in the same manner as in Synthesis Example 1-1-3, except that etherification agent 9 was used instead of etherification agent 1 (yield: 85%).

Synthesis Example 4-10-3: Synthesis of Phenol 10

Phenol 10 was obtained in the same manner as in Synthesis Example 1-1-4, except that protecting phenol 20 was used instead of protecting phenol 1 (yield: 93%). $^{1}$H-NMR(500MHz in DMSO-d6):δ=9.18(1H, s), 6.81(1H, t), 6.72(1H, dd), 6.53(1H, dd), 3.82(2H, t), 1.63(2H, m), 1.19-1.40(12H, m), 0.44(2H, m), -0.06(9H, s)ppm. $^{19}$F-NMR(470MHz in DMSO-d6):δ=-133.7(1F, t)ppm.

Examples 1 to 26 and Comparative Examples 1 to 5

<Preparation of Conductive Paste Compositions>

As polyurethanes for preparing conductive paste compositions, PU1 to PU17 and Comparative PU1 to Comparative PU3 shown in Tables 1 and 2 as well as the following resins were used.

- Fluorine rubber (G801, manufactured by DAIKIN INDUSTRIES, Ltd.)
- Polyester (UE-9200, manufactured by UNITIKA Ltd.)

As conductive fillers, the following silver powders A to E and copper powder A were prepared.

- Silver powder A: average particle diameter ($D_{L50}$) is 2.1 um
- Silver powder B: average particle diameter ($D_{L50}$) is 5.3 um
- Silver powder C: average particle diameter ($D_{L50}$) is 1.2 um
- Silver powder D: average particle diameter ($D_{L50}$) is 0.67 um
- Silver powder E: average particle diameter ($D_{L50}$) is 1.72 um

- Copper powder A: average particle diameter ($D_{L50}$) is 1.30 um

In the average particle diameter measurement, a laser-diffraction particle size distribution measurement apparatus was used to measure the particle size distribution, and the cumulative value up to 50% of the particle diameters was determined as an average particle diameter.

The following phenols 1 to 13 were used as the phenol compounds as additives. Here, phenol 11 is same as the capping agent 1, phenol 12 is same as the capping agent 4, and phenol 13 is same as the capping agent 11.

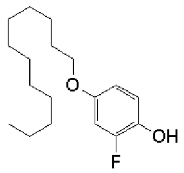
Phenol 1

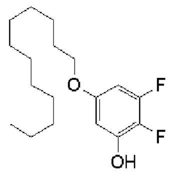
Phenol 2

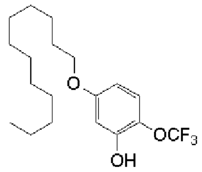
Phenol 3

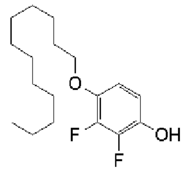
Phenol 4

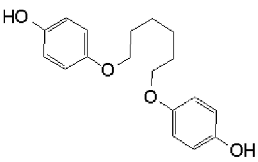
Phenol 5

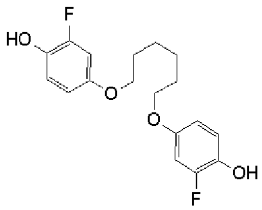
Phenol 6

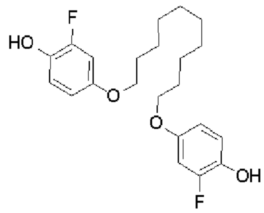
Phenol 7

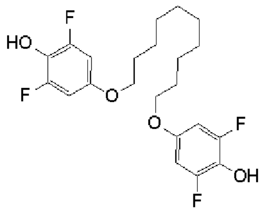
Phenol 8

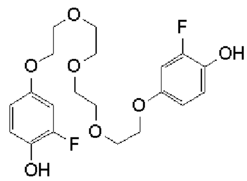
Phenol 9

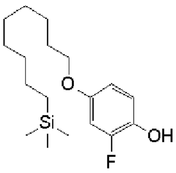
Phenol 10

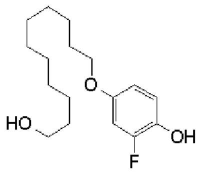
Phenol 11

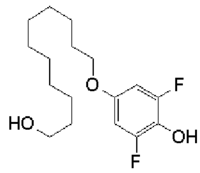
Phenol 12

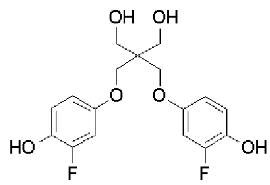
Phenol 13

According to the formulations shown in Table 3, a polymer, a conductive filler, a phenol compound, and a solvent (BCA) were stirred and mixed to prepare a conductive paste composition.

[TABLE 3]

| | Polymer [part by mass] | Conductive Filler [part by mass] | Solvent [part by mass] | Phenol Compound [part by mass] |
|---|---|---|---|---|
| CI-1 | PU1[11] | Silver Powder C[61] | BCA[28] | - |
| CI-2 | PU3[11] | Silver Powder A[61] | BCA[28] | - |
| CI-3 | PU5[11] | Silver Powder B[61] | BCA[28] | - |
| CI-4 | PU8[11] | Silver Powder D[61] | BCA[28] | - |
| CI-5 | PU13[11] | Silver Powder E[61] | BCA[28] | - |
| CI-6 | PU16[11] | Silver Powder C[61] | BCA[28] | - |
| CI-7 | PU1[11] | Silver Powder C[60] | BCA[27] | Phenol 1 [2] |

(continued)

| | Polymer [part by mass] | Conductive Filler [part by mass] | Solvent [part by mass] | Phenol Compound [part by mass] |
|---|---|---|---|---|
| CI-8 | PU1[11] | Silver Powder C[59] | BCA[27] | Phenol 3 [3] |
| CI-9 | PU1[11] | Silver Powder C[59] | BCA[27] | Phenol 7 [3] |
| CI-10 | PU1[11] | Silver Powder C[59] | BCA[27] | Phenol 10 [3] |
| CI-11 | PU1[11] | Silver Powder C[60] | BCA[27] | Phenol 11 [2] |
| CI-12 | PU2[11] | Silver Powder C[59] | BCA[27] | Phenol 2 [3] |
| CI-13 | PU3[11] | Silver Powder C[59] | BCA[27] | Phenol 4 [3] |
| CI-14 | PU4[11] | Silver Powder C[59] | BCA[27] | Phenol 5 [3] |
| CI-15 | PU6[11] | Silver Powder C[59] | BCA[27] | Phenol 6 [3] |
| CI-16 | PU7[11] | Silver Powder C[59] | BCA[27] | Phenol 7 [3] |
| CI-17 | PU8[11] | Silver Powder C[59] | BCA[27] | Phenol 8 [3] |
| CI-18 | PU9[11] | Silver Powder C[59] | BCA[27] | Phenol 8 [3] |
| CI-19 | PU10[11] | Silver Powder C[59] | BCA[27] | Phenol 9 [3] |
| CI-20 | PU11[11] | Silver Powder C[59] | BCA[27] | Phenol 11 [3] |
| CI-21 | PU12[11] | Silver Powder C[59] | BCA[27] | Phenol 11 [3] |
| CI-22 | PU14[11] | Silver Powder C[59] | BCA[27] | Phenol 12 [3] |
| CI-23 | PU15[11] | Silver Powder C[59] | BCA[27] | Phenol 13 [3] |
| CI-24 | PU16[11] | Silver Powder C[59] | BCA[27] | Phenol 13 [3] |
| CI-25 | PU17[11] | Silver Powder C[59] | BCA[27] | Phenol 7 [3] |
| CI-26 | PU1[11] | Copper Powder A[60] | BCA[27] | Phenol 11 [2] |
| CI-27 | Comparative PU1 [11] | Silver Powder C[59] | BCA[27] | - |
| CI-28 | Comparative PU2 [11] | Silver Powder D[59] | BCA[27] | - |
| CI-29 | Comparative PU3 [11] | Silver Powder E[59] | BCA[27] | - |

(continued)

| | Polymer [part by mass] | Conductive Filler [part by mass] | Solvent [part by mass] | Phenol Compound [part by mass] |
|---|---|---|---|---|
| CI-30 | Fluorine Rubber [12] | Silver Powder C[68] | BCA[20] | - |
| CI-31 | Polyester[12] | Silver Powder C[68] | BCA[20] | - |
| BCA: diethylene glycol monobutyl ether acetate | | | | |

Evaluation of Stretchable Conductive Wire

<Preparation of Evaluation Samples>

A conductive paste composition was applied onto a polyurethane film by using a screen printer MT-320TVC manufactured by Micro-tec Co., Ltd., followed by heating with a hot-air dryer to form a stretchable conductive wire with a line width of 10 mm, a length of 70 mm, and a film thickness of 10 $\mu$m.

<Measurement of Initial Electric Resistance in Non-Elongated State>

The electric resistance between two ends of the stretchable conductive wire formed on the polyurethane film was measured by 4-terminal resistance measurement method. The electric resistance was measured with PXIe-4136SMU resistance measurement unit manufactured by National Instruments Corp.

- Electric resistance ($\Omega$) R=V/I (V: voltage, I: current)

Table 4 shows the measurement results of the initial electric resistance (electric resistance in a non-elongated state).

<Measurement of Maximum Electric Resistance upon 20% Elongation>

The polyurethane film with the stretchable conductive wire formed thereon was elongated by 20% from the non-elongated state (0%) in which the polyurethane film was not loose. The polyurethane film in this state was immobilized to measure the electric resistance by the 4-terminal resistance measurement method.
The polyurethane film with the stretchable conductive wire formed thereon was elongated at a speed of 300 mm/min in a longitudinal direction of the stretchable conductive wire (rectangle) by using a precision universal testing machine AG-Xplus HS manufactured by Shimadzu Corporation.

- Change in electric resistance upon 20%

elongation=[electric resistance ($\Omega$) upon 20% elongation]÷[initial electric resistance ($\Omega$)]×100

Table 2 shows the change in electric resistance upon 20% elongation.

<Measurement of Maximum Electric Resistance upon 300% Elongation>

The polyurethane film with the stretchable conductive wire formed thereon was elongated by 300% from the non-elongated state of 0%, and was immobilized to measure the electric resistance.
The polyurethane film with the stretchable conductive wire formed thereon was elongated at a speed of 300 mm/min in a longitudinal direction of the stretchable conductive wire (rectangle) by using a precision universal testing machine AG-Xplus HS manufactured by Shimadzu Corporation.

- Change in electric resistance upon 300%

elongation=[electric resistance (Ω) upon 300% elongation]÷[initial electric resistance (Ω)]×100

Table 4 shows the change in electric resistance upon 300% elongation.

<Measurement of Maximum Resistance Value in Repetitive Elongations and Shrinkages at 0 to 20%>

The polyurethane film with the stretchable conductive wire formed thereon was reciprocately elongated and shrunk 1000 times between the non-elongated state (0%) in which the polyurethane film was not loose and 20%-elongated state, and the change in electric resistance of the conductive wire over time was measured.

In this repetitive stretching test, the polyurethane film was elongated and shrunk at a tensile speed of 300 mm/min in the longitudinal direction of the stretchable conductive wire (rectangle) by using a precision universal testing machine AG-Xplus HS manufactured by Shimadzu Corporation.

Moreover, electrodes were set inside a sample immobilization jig of the tensile tester (the precision universal testing machine AG-Xplus HS), and the electric resistance was measured by the 4-terminal resistance measurement method using PXIe-4136SMU resistance measurement unit manufactured by National Instruments Corp.

- Change in maximum electric resistance value by 1000-time repetitive elongations and shrinkages with an

elongation ratio of 0 to 20%=[maximum electric resistance (Ω) in repetitive stretching test]÷[initial electric resistance (Ω)]×100

Table 4 shows the change in a maximum electric resistance by 1000-time repetitive elongations and shrinkages with an elongation ratio of 20%.

[TABLE 4]

| | Conductive Paste Composition | Baking Temperature/ Time | Initial Electric Resistance (Ω) | Change in Electric Resistance (%) Upon 20% Elongation | Change in Electric Resistance (%) Upon 300% Elongation | Change in Maximum Electric Resistance (%) by 0-20% 1000-Time Repetitive Elongations and Shrinkages |
|---|---|---|---|---|---|---|
| Example 1 | CI-1 | 120°C/0.5hr | 4.6 | 390 | 44700 | 1740 |
| Example 2 | CI-2 | 120°C/0.5hr | 3.2 | 490 | - | 3260 |
| Example 3 | CI-3 | 120°C/0.5hr | 1.8 | 490 | - | 4540 |
| Example 4 | CI-4 | 120°C/0.5hr | 3.3 | 250 | - | 2510 |
| Example 5 | CI-5 | 120°C/0.5hr | 6.1 | 440 | - | 2170 |
| Example 6 | CI-6 | 120°C/0.5hr | 4.1 | 480 | - | 1890 |
| Example 7 | CI-7 | 120°C/0.5hr | 4.4 | 330 | 35800 | 1520 |
| Example 8 | CI-8 | 120°C/0.5hr | 5.5 | 490 | - | 1650 |
| Example 9 | CI-9 | 120°C/0.5hr | 5.2 | 190 | 21800 | 950 |
| Example 10 | CI-10 | 120°C/0.5hr | 3.8 | 350 | - | 1660 |
| Example 11 | CI-11 | 120°C/0.5hr | 3.9 | 400 | - | 1530 |
| Example 12 | CI-12 | 120°C/0.5hr | 6.5 | 440 | - | 1400 |
| Example 13 | CI-13 | 120°C/0.5hr | 4.4 | 390 | - | 1590 |
| Example 14 | CI-14 | 120°C/0.5hr | 6.5 | 330 | - | 1120 |

(continued)

| | Conductive Paste Composition | Baking Temperature/ Time | Initial Electric Resistance (Ω) | Change in Electric Resistance (%) Upon 20% Elongation | Change in Electric Resistance (%) Upon 300% Elongation | Change in Maximum Electric Resistance (%) by 0-20% 1000-Time Repetitive Elongations and Shrinkages |
|---|---|---|---|---|---|---|
| Example 15 | CI-15 | 120°C/0.5hr | 5.5 | 230 | - | 1080 |
| Example 16 | CI-16 | 120°C/0.5hr | 5.6 | 200 | - | 920 |
| Example 17 | CI-17 | 120°C/0.5hr | 3.8 | 380 | - | 1590 |
| Example 18 | CI-18 | 120°C/0.5hr | 5.2 | 220 | - | 1210 |
| Example 19 | CI-19 | 120°C/0.5hr | 4.7 | 340 | - | 1670 |
| Example 20 | CI-20 | 120°C/0.5hr | 3.3 | 400 | - | 1780 |
| Example 21 | CI-21 | 120°C/0.5hr | 3.3 | 390 | - | 1740 |
| Example 22 | CI-22 | 120°C/0.5hr | 3.5 | 340 | - | 1660 |
| Example 23 | CI-23 | 120°C/0.5hr | 4.4 | 330 | - | 1510 |
| Example 24 | CI-24 | 120°C/0.5hr | 3.9 | 350 | - | 1750 |
| Example 25 | CI-25 | 120°C/0.5hr | 4.2 | 290 | - | 1420 |
| Example 26 | CI-26 | 120°C/0.5hr | 10.2 | 220 | - | 3380 |
| Comparative Example 1 | CI-27 | 120°C/0.5hr | 5.7 | 510 | Cutoff | 3730 |
| Comparative Example 2 | CI-28 | 120°C/0.5hr | 8.2 | 490 | Cutoff | 6720 |
| Comparative Example 3 | CI-29 | 120°C/0.5hr | 8.6 | 550 | Cutoff | 5580 |
| Comparative Example 4 | CI-30 | 120°C/0.5hr | 5.2 | 650 | Cutoff | 6500 |
| Comparative Example 5 | CI-31 | 120°C/0.5hr | 9.9 | 1290 | Cutoff | 9850 |
| - : Not measured | | | | | | |

It can be understood that, as shown in Table 4, when the conductive paste composition blended with the polyurethane containing the phenolic hydroxyl group is used, increase in electric resistance due to wire elongation was small despite the baking performed at a low temperature of 120°C for short time, i.e. 30 minutes, and the resulting conductive wires were excellent in conduction stability against the repetitive elongations and shrinkages (Examples 1 to 26). Moreover, by adding the phenol compound as an additive, it is possible to suppress degradation due to wire elongations (Examples 7 to 26). In contrast, in Comparative Examples, the polyurethanes with no phenolic hydroxyl group (Comparative Examples 1 to 3) and the resins (Comparative Examples 4 to 5) showed considerable increase in electric resistance due to wire elongation and repetitive elongations and shrinkages.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A polyurethane comprising a phenolic hydroxyl group represented by the following general formula (1A),

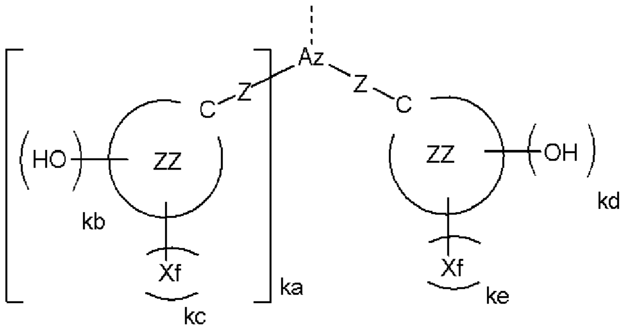

(1A)

wherein Az represents a linear, branched, or cyclic (ka+2)-valent hydrocarbon group or a fluorinated hydrocarbon group having 1 to 20 carbon atoms, and $-CH_2-$constituting the (ka+2)-valent hydrocarbon group is optionally substituted with -O-, $-NR^4-$, -C(=O)-, or - $Si(R^2R^3)-$; each of $R^2$ and $R^3$ is a linear, branched, or cyclic alkyl group having 1 to 6 carbon atoms, or a phenyl group; $R^4$ is a hydrogen atom, or a linear or branched alkyl group having 1 to 4 carbon atoms; Z represents a single bond or an oxygen atom; each Xf independently represents a hydrogen atom, a halogen atom, a linear, branched, or cyclic monovalent hydrocarbon group having 1 to 10 carbon atoms optionally substituted with a fluorine atom, an alkoxy group having 1 to 10 carbon atoms optionally substituted with a fluorine atom, or an electron-withdrawing group; each of ring ZZ independently represents an aromatic monocyclic or polycyclic ring having 5 to 20 carbon atoms; each carbon atom of the ring ZZ is optionally substituted with a nitrogen atom, an oxygen atom, or a sulfur atom; "ka" represents an integer of 0 to 2; "kb" and "kd" each represent 1 or 2; "kc" and "ke" each represent an integer of 0 to 2; and a dashed line represents a bonding arm.

2. The polyurethane according to claim 1, comprising a phenolic hydroxyl group represented by the following general formula (1B),

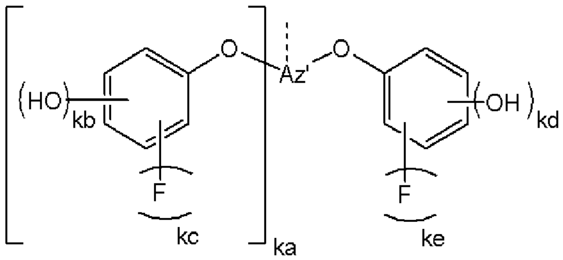

(1B)

wherein Az' represents a linear, branched, or cyclic (ka+2)-valent hydrocarbon group or a fluorinated hydrocarbon group having 1 to 19 carbon atoms, and $-CH_2-$constituting the (ka+2)-valent hydrocarbon group is optionally substituted with -O-, $-NR^4-$, -C(=O)-, or - $Si(R^2R^3)-$; "ka" represents an integer of 0 to 2; "kb", "kc", "kd", and "ke" each represent 1 or 2; $R^2$, $R^3$, and $R^4$ are as defined above; and a dashed line represents a bonding arm.

3. The polyurethane according to claim 1 or 2, further comprising one or more weakly acidic functional group represented by the following general formulae (1a) to (1c),

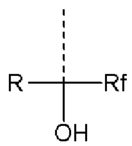

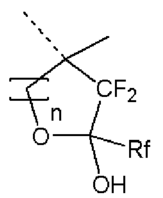

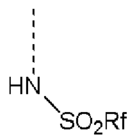

(1a) (1b) (1c)

wherein R represents a hydrogen atom, a fluorine atom, or a linear, branched, or cyclic hydrocarbon group having 1 to 10 carbon atoms optionally substituted with a fluorine atom; Rf represents a fluorine atom, or a linear, branched, or cyclic fluorinated hydrocarbon group having 1 to 10 carbon atoms; "n" is an integer of 1 or 2; and a dashed line represents a bonding arm.

**4.** The polyurethane according to any one of claims 1 to 3, further comprising one or more structures represented by the following general formulae (2a) to (2c),

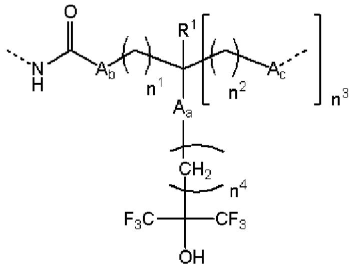
(2a)

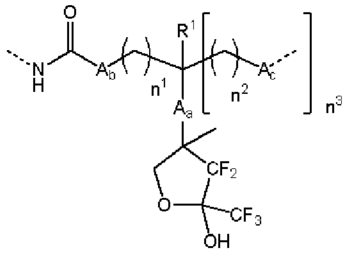
(2b)

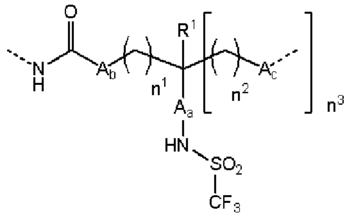
(2c)

wherein $R^1$ is a hydrogen atom or a monovalent hydrocarbon group having 1 to 3 carbon atoms; $A_a$ represents a single bond, or a linear, branched, or cyclic divalent hydrocarbon group having 1 to 20 carbon atoms, and $-CH_2-$ constituting $A_a$ is optionally substituted with -O-, -C(=O)-, -C(=O)O-, or $-C_6H_4-$, or is optionally $-NR^4-C(=O)-$; each of $A_b$ and $A_c$ independently represents any group selected from the group consisting of -O-, $-O-C(=O)-NR^4-$, $-NR^4-$, and - C(=O)O-; each of $n^1$, $n^2$, and $n^4$ is an integer of 0 to 10; $n^3$ is an integer of 0 or 1; $R^4$ is as defined above; and a dashed line represents a bonding arm.

**5.** A method for producing the polyurethane according to claim 2, comprising introducing the phenolic hydroxyl group to a polyurethane by using an alcohol or amine represented by the following general formula (1C) after a chain extension reaction,

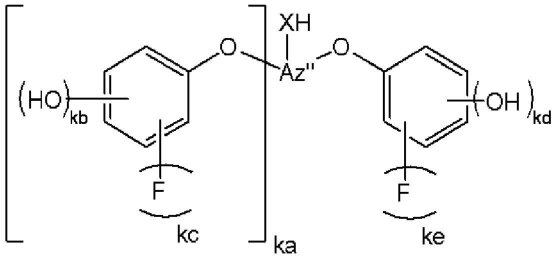
(1C)

wherein Az" represents a linear, branched, or cyclic (ka+2)-valent hydrocarbon group or a fluorinated hydrocarbon group having 1 to 19 carbon atoms, and $-CH_2-$constituting the (ka+2)-valent hydrocarbon group is optionally substituted with -O-, -C(=O)-, or $-Si(R^2R^3)-$; X represents an oxygen atom or $NR^4$; "ka" represents an integer of 0 to 2; "kb", "kc", "kd", and "ke" each represent 1 or 2; and $R^2$, $R^3$, and $R^4$ are as defined above.

**6.** The method for producing the polyurethane according to claim 5, comprising introducing a weakly acidic functional group to a polyurethane by using one or more alcohols represented by the following general formulae (3a) to (3c) as a chain extender,

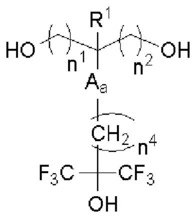
(3a)

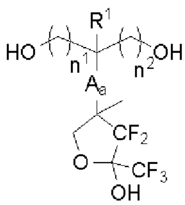
(3b)

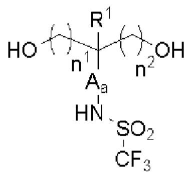
(3c)

wherein $R^1$ is a hydrogen atom or a monovalent hydrocarbon group having 1 to 3 carbon atoms; $A_a$ is a single bond, or a linear, branched, or cyclic divalent hydrocarbon group having 1 to 20 carbon atoms, and $-CH_2-$constituting $A_a$ is optionally substituted with -O-, - C(=O)-, -C(=O)O-, or $-C_6H_4-$, or is optionally $-NR^4-C(=O)-$; $R^4$ is a hydrogen atom, or a linear or branched alkyl group having 1 to 4 carbon atoms; and each of $n^1$, $n^2$, and $n^4$ is an integer of 0 to 10.

**7.** A conductive paste composition comprising (A) a conductive filler, (B) the polyurethane according to any one of claims 1 to 4, and (C) a solvent.

**8.** The conductive paste composition according to claim 7, further comprising (D) a phenol compound.

**9.** The conductive paste composition according to claim 8, wherein the phenol compound as the component (D) comprises a structure represented by the following general formula (2A),

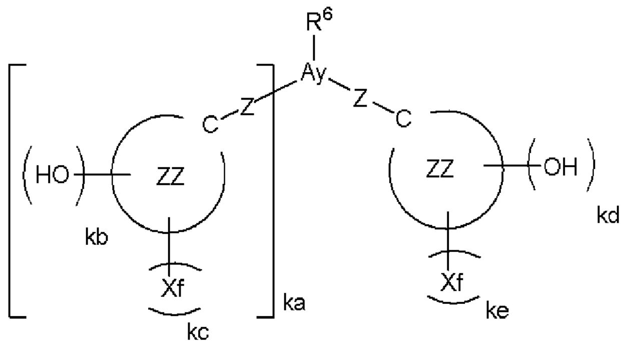

(2A)

wherein $R^6$ represents a hydrogen atom, a halogen atom, a cyano group, or a hydroxyl group; Ay represents a linear, branched, or cyclic (ka+2)-valent hydrocarbon group or a fluorinated hydrocarbon group having 1 to 20 carbon atoms, and $-CH_2-$ constituting the (ka+2)-valent hydrocarbon group is optionally substituted with -O-, - C(=O)-, or $-Si(R^2R^3)-$; "ka" represents an integer of 0 to 2; "kb" and "kd" each represent 1 or 2; "kc" and "ke" each represent an integer of 0 to 2; and Z, Xf, ZZ, $R^2$, and $R^3$ are as defined above.

**10.** The conductive paste composition according to claim 9, wherein the phenol compound as the component (D) comprises a structure represented by the following general formula (2B),

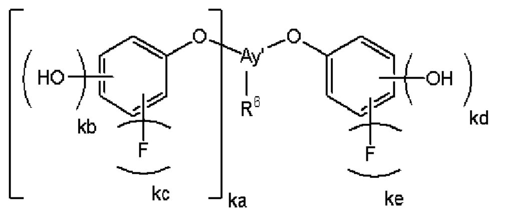

(2B)

wherein Ay' represents a linear, branched, or cyclic (ka+2)-valent hydrocarbon group or a fluorinated hydrocarbon group having 1 to 19 carbon atoms, and $-CH_2-$constituting the (ka+2)-valent hydrocarbon group is optionally substituted with -O-, -C(=0)-, or $-Si(R^2R^3)-$; "ka" represents an integer of 0 to 2; "kb", "kc", "kd", and "ke" each represent 1 or 2; and $R^2$, $R^3$, and $R^6$ are as defined above.

**11.** The conductive paste composition according to any one of claims 7 to 10, wherein the conductive filler as the component (A) is contained in a proportion exceeding 70 parts by mass relative to 100 parts by mass of a total of the components (A) and (B).

**12.** The conductive paste composition according to any one of claims 7 to 11, wherein the conductive filler as the component (A) is a powder selected from the group consisting of gold, silver, silver chloride, platinum, copper, tin, iron, magnesium, titanium, nickel, palladium, aluminum, tungsten, molybdenum, ruthenium, chromium, indium, solder, carbon, and composites thereof.

**13.** The conductive paste composition according to claim 12, wherein the conductive filler as the component (A) is a silver powder.

**14.** The conductive paste composition according to any one of claims 7 to 13, wherein the conductive filler as the component (A) has an average particle size of 5 nm to 10 μm.

**15.** A conductive wire formed on a substrate, the conductive wire comprising a baked product of the conductive paste composition according to any one of claims 7 to 14.

**16.** The conductive wire according to claim 15, wherein the substrate is stretchable.

**17.** The conductive wire according to claim 16, wherein the substrate is a thermoplastic polyurethane.

**18.** The conductive wire according to claim 16 or 17, wherein electric resistance upon 20% elongation is 500% or less of electric resistance before the elongation.

**19.** The conductive wire according to any one of claims 16 to 18, wherein a maximum electric resistance when the conductive wire is elongated and shrunk repeatedly 1000 times with an elongation ratio of 20% is 5000% or less of electric resistance before the elongations and shrinkages.

**20.** A method for producing a conductive wire by using the conductive paste composition according to any one of claims 7 to 14 to form a conductive wire on a substrate, wherein the conductive wire is formed with a baking temperature of 60 to 160°C.

**21.** A method for producing a conductive wire, comprising printing the conductive paste composition according to any one of claims 7 to 14 to form a conductive wire on a substrate.

## INTERNATIONAL SEARCH REPORT

International application No.
**PCT/JP2022/030244**

**A. CLASSIFICATION OF SUBJECT MATTER**

***C07C 43/23***(2006.01)i; ***C07C 255/54***(2006.01)i; ***C07C 311/09***(2006.01)i; ***C07C 49/82***(2006.01)i; ***H01B 1/22***(2006.01)i; ***C08G 18/32***(2006.01)i; ***H01B 13/00***(2006.01)i; ***H01B 7/06***(2006.01)i; ***C07D 307/20***(2006.01)i; ***C07C 69/675***(2006.01)i
FI: C08G18/32 003; C07C43/23 D; C07C49/82; C07C69/675; C07C255/54; C07C311/09; C07D307/20; C08G18/32 025; H01B1/22 A; H01B7/06; H01B13/00 503D

According to International Patent Classification (IPC) or to both national classification and IPC

**B. FIELDS SEARCHED**

Minimum documentation searched (classification system followed by classification symbols)

C08G18/32; C07C43/23; C07C49/82; C07C69/675; C07C255/54; C07C311/09; C07D307/20; H01B1/22; H01B7/06; H01B13/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2009-19081 A (TAIYO INK MFG. LTD.) 29 January 2009 (2009-01-29) claims 1, 3, paragraphs [0009], [0025], examples 1-4 | 1 |
| Y | | 7-8, 11-21 |
| A | | 2-6, 9-10 |
| Y | JP 2017-183207 A (TOYO INK SC HOLDINGS CO., LTD.) 05 October 2017 (2017-10-05) claims 1-7, paragraphs [0010], [0017], [0024], [0025], [0046], [0047], [0052], [0054]-[0082], example 17 | 7-8, 11-21 |
| A | JP 2006-257517 A (TOYO INK MFG. CO., LTD.) 28 September 2006 (2006-09-28) paragraph [0052] | 1-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 August 2022** | **13 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**
**Information on patent family members**

International application No.
**PCT/JP2022/030244**

| Patent document cited in search report | Publication date (day/month/year) | Patent family member(s) | Publication date (day/month/year) |
|---|---|---|---|
| JP 2009-19081 A | 29 January 2009 | CN 101343345 A<br>KR 10-2009-0006011 A<br>TW 200911867 A | |
| JP 2017-183207 A | 05 October 2017 | (Family: none) | |
| JP 2006-257517 A | 28 September 2006 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3923861 B
- JP 6657525 B
- JP 2019076214 A
- WO 2017217509 A1
- WO 2018110632 A1
- JP 2019110093 A
- WO 2018235734 A1